# EUROPEAN PATENT APPLICATION

(11) **EP 1 810 693 A2**
(43) Date of publication of application: **25.07.2007**
(21) Application number: 07009475.0
(22) Date of filing: 25.01.2002
(51) Int. Cl.: A61K 45/06, A61P 9/00, A61K 31/395

(54) **Combinations of sterol absorption inhibitor(s) with blood modifier(s) for treating vascular conditions**

(30) Priority: 26.01.2001 US 264396 P; 26.01.2001 US 264600 P; 26.01.2001 US 264275 P; 21.09.2001 US 324123 P
(62) Divisional of application: 02704233.2
(71) Applicant: SCHERING CORPORATION, Kenilworth, NJ 07033-0530 (US)
(72) Inventor: Kosoglou, Teddy, Bucks County PA 18929-1178 (US); Ress, Rudyard, Joseph, Flemington NJ 08822-5910 (US); Strony, John, Lebanon NJ 08833 (US); Veltri, Enrico, P., Princeton NJ 08540 (US)
(74) Representative: HOFFMANN EITLE

(57) **Abstract**

The present invention provides compositions, therapeutic combinations and methods including:
(a) at least one sterol absorption inhibitor, and
(b) at least one blood modifier, which can be useful for treating vascular conditions and lowering plasma levels of sterols.

## Description

### CROSS-REFERENCE TO RELATED APPLICATIONS

This application claims the benefit of priority from U.S. Provisional Patent Application Serial No. 60/324,123 filed September 21, 2001, U.S. Provisional Patent Application Serial No. 60/264,396 filed January 26,2001, U.S. Provisional Patent Application Serial No. 60/264,600 filed January 26, 2001, and U.S. Provisional Patent Application Serial No. 60/264,275 filed January 26, 2001, each incorporated herein by reference.

### FIELD OF THE INVENTION

The present invention relates to methods, compositions and therapeutic combinations for treating vascular conditions in mammals comprising blood modifier(s) and sterol absorption inhibitor(s).

### BACKGROUND OF THE INVENTION

Vascular disease is a term that broadly encompasses all disorders of blood vessels including small and large arteries and veins and blood flow. The most prevalent form of vascular disease is arteriosclerosis, a condition associated with the thickening and hardening of the arterial wall. Arteriosclerosis of the large vessels is referred to as atherosclerosis. Atherosclerosis is the predominant underlying factor in vascular disorders such as coronary artery disease, aortic aneurysm, arterial disease of the lower extremities and cerebrovascular disease.

One major risk factor for arteriosclerosis is high serum cholesterol. A total cholesterol level in excess of 225-250 mg/dl is associated with significant elevation of risk of vascular disease, particularly coronary heart disease.

Cholesteryl esters are a major component of atherosclerotic lesions and the major storage form of cholesterol in arterial wall cells. Formation of cholesteryl esters is also a step in the intestinal absorption of dietary cholesterol. Thus, inhibition of cholesteryl ester formation and reduction of serum cholesterol can inhibit the progression of atherosclerotic lesion formation, decrease the accumulation of cholesteryl esters in the arterial wall, and block the intestinal absorption of dietary cholesterol.

The regulation of whole-body cholesterol homeostasis in mammals and animals involves the regulation of dietary cholesterol and modulation of cholesterol biosynthesis, bile acid biosynthesis and the catabolism of the cholesterol-containing plasma lipoproteins. The liver is the major organ responsible for cholesterol biosynthesis and catabolism and, for this reason, it is a prime determinant of plasma cholesterol levels. The liver is the site of synthesis and secretion of very low density lipoproteins (VLDL) which are subsequently metabolized to low density lipoproteins (LDL) in the circulation. LDL are the predominant cholesterol-carrying lipoproteins in the plasma and an increase in their concentration is correlated with increased atherosclerosis. When intestinal cholesterol absorption is reduced, by whatever means, less cholesterol is delivered to the liver. The consequence of this action is decreased hepatic lipoprotein (VLDL) production and an increase in the hepatic clearance of plasma cholesterol, mostly as LDL. Thus, the net effect of inhibiting intestinal cholesterol absorption is a decrease in plasma cholesterol levels.

U.S. Patents Nos. 5,767,11-5, 5,624,920, 5,668,990, 5,656,624 and 5,688,787, respectively, disclose hydroxy-substituted azetidinone compounds and substituted β-lactam compounds useful for lowering cholesterol and/or in inhibiting the formation of cholesterol-containing lesions in mammalian arterial walls. U.S. Patents Nos. 5,846,966 and 5,661,145, respectively, disclose hydroxy-substituted azetidinone compounds or substituted β-lactam compounds in combination with HMG CoA reductase inhibitors for preventing or treating atherosclerosis and reducing plasma cholesterol levels.

PCT Patent Application No. WO 00/38725 discloses cardiovascular therapeutic combinations including an ileal bile acid transport inhibitor or cholesteryl ester transport protein inhibitor in combination with a fibric acid derivative, nicotinic acid derivative, microsomal triglyceride transfer protein inhibitor, cholesterol absorption antagonist, phytosterol, stanol, antihypertensive agent or bile acid sequestrant.

U.S. Patent No. 5,698,527 discloses ergostanone derivatives substituted with disaccharides as cholesterol absorption inhibitors, employed alone or in combination with certain other cholesterol lowering agents, which are useful in the treatment of hypercholesterolemia and related disorders.

Vascular conditions are often associated with thrombotic events sometimes resulting in myocardial infarction, stroke and ischemic attack. A thrombotic event is one associated with the formation or presence of a thrombus e.g.(blood clot), which results from an aggregation of blood factors, primarily platelets and fibrin with entrapment of cellular elements, frequently causing vascular obstruction at the point of its formation.

Blood coagulation is a process consisting of a complex interaction of various blood components, or factors, which eventually gives rise to a fibrin clot. Generally, the blood components which participate in what has been referred to as the coagulation "cascade" are proenzymes or zymogens, enzymatically inactive proteins, which are converted to proteolytic enzymes by the action of an activator, itself an activated clotting factor. Coagulation factors that have undergone such a conversion are generally referred to as "active factors", and are designated by the addition of the letter "a" to the name of the coagulation factor (e.g. fVIIa).

Activated factor X (fXa) is required to convert prothrombin to thrombin, which then converts fibrinogen to fibrin as a final stage in forming a fibrin clot. There are two systems or pathways that promote the activation of factor X. The "intrinsic pathway" refers to those reactions that lead to thrombin formation through utilization of factors present only in plasma. A series of protease-mediated activations ultimately generates factor IXa, which, in conjunction with factor VIIIa, cleaves factor X into Xa. An identical proteolysis is effected by fVIIa and its cofactor TF in the "extrinsic pathway" of blood coagulation. TF is a membrane bound protein and does not normally circulate in plasma. Upon vessel disruption, however, it is exposed and forms a complex with fVIIa to catalyze factor X activation or factor IX activation in the presence of Ca²⁺ and phospholipid. While the relative importance of the two coagulation pathways in hemostasis is unclear, in recent years fVIIa and TF have been found to play a pivotal role in the initiation and regulation of blood coagulation.

FVII is a trace plasma glycoprotein that circulates in blood as a single-chain zymogen. The zymogen is catalytically inactive. Single-chain fVII may be converted to two-chain fVIIa by factor Xa, factor Xlla, factor IXa, fVIIa or thrombin *in vitro.* Factor Xa is believed to be the major physiological activator of fVII. Like several other plasma proteins involved in hemostasis, fVII is dependent on vitamin K for its activity which is required for the gamma-carboxylation of multiple glutamic acid residues that are clustered in the amino terminus of the protein. These gamma-carboxylated glutamic acids are required for the metal-associated interactions of fVII with phospholipids.

The conversion of zymogen fVII into the activated two-chain molecule occurs by cleavage of an internal Arg 152-IIe 153 peptide bond. In the presence of TF, phospholipids and calcium ions, the two-chain fVIIa rapidly activates factor X or factor IX by limited proteolysis.

It is often desirable to selectively block or inhibit the coagulation cascade in a patient. Blood modifiers such as heparin, coumarin, derivatives of coumarin, indandione derivatives, thrombin inhibitors, factor Xa inhibitors, modified fVII or other agents may be used.

The use of drugs to modify blood is beneficial in a number of vascular disease states including atherosclerosis. Proliferation of smooth muscle cells (SMCs) in the vessel wall is an important event in the formation of vascular lesions in atherosclerosis, after vascular reconstruction or in response to other vascular injury. SMC proliferation typically occurs within the first few weeks and up to six months after injury.

In about 30% or more of patients treated by angioplasty or bypass grafts, thrombosis and or SMC proliferation causes re-occlusion of the vessel. This closure of the vessel subsequent to surgery is known as restenosis.

Modified FVIIa has been shown to effectively suppress the restenosis process possibly as a result of a decreased procoagulant activity and thrombin generation initially after treatment of the constricted vessel (see e.g. US Patent No. 5,639,739).

Other blood modifiers have been used to treat thrombotic events associated with vascular conditions (see for example, WO 01/21/21259). Despite recent improvements in the treatment of vascular disease, there remains a need in the art for improved compositions and treatments.

### SUMMARY OF THE INVENTION

In one embodiment of the present invention provides a composition comprising (a) at least one sterol absorption inhibitor or pharmaceutically acceptable salt or solvate thereof or prodrug of the at least one sterol absorption inhibitor or of the salt or solvate thereof; and (b) at least one blood modifier different from the component (a) above.

In another embodiment, the present invention provides a composition comprising (a) at least one substituted azetidinone compound or substituted β-lactam compound, or pharmaceutically acceptable salt or solvate thereof, or prodrug of the at least one substituted azetidinone compound or the at least one substituted β-lactam compound or of the salt or solvate thereof and (b) at least one blood modifier for treating vascular conditions which is different from component (a).

In another embodiment, the present invention provides a composition comprising:
(a) a compound represented by Formula (II) below: 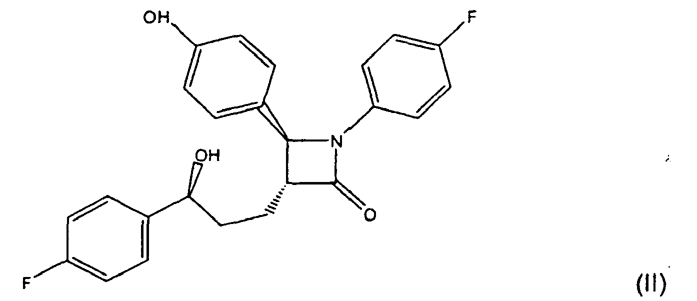 or pharmaceutically acceptable salt or solvate thereof, or prodrug of the compound of Formula (II) or of the salt or solvate thereof; and
(b) at least one blood modifier for treating vascular conditions which is different from component (a).

Pharmaceutical compositions for the treatment or prevention of vascular conditions or lowering a concentration of a sterol in plasma of a mammal, comprising a therapeutically effective amount of the above compositions or therapeutic combinations and a pharmaceutically acceptable carrier also are provided.

Methods of treating or preventing vascular conditions and/or lowering a concentration of a sterol in plasma of a mammal, comprising the step of administering to a mammal in need of such treatment an effective amount of the above compositions or therapeutic combinations also are provided.

Other than in the operating examples, or where otherwise indicated, all numbers expressing quantities of ingredients, reaction conditions, and so forth used in the specification and claims are to be understood as being modified in all instances by the term "about."

### DETAILED DESCRIPTION

In one embodiment, the present invention is directed to compositions, pharmaceutical compositions, therapeutic combinations, kits and methods of treatment using the same comprising at least one (one or more) blood modifier and at least one (one or more) sterol absorption inhibitor, such as but not limited to, substituted azetidinone sterol absorption inhibitors or substituted β-lactam sterol absorption inhibitors discussed in detail below.

The compositions and therapeutic combinations of the present invention can be administered to a mammal in need of such treatment in a therapeutically effective amount to treat "vascular conditions" such as atherosclerosis, hyperlipidaemia (including hypercholesterolaemia, hypertriglyceridaemia, sitosterolemia), vascular inflammation, hypertension, angina, cardiac arrhythmias, stroke, as well as conditions such diabetes, obesity, and/or to reduce the level of sterol(s) in the plasma. The compositions and treatments can be administered by any suitable means which produce contact of these compounds with the site of action in the body, for example in the plasma, liver or small intestine of a mammal or human.

"Blood modifiers" as used herein refer to those agents capable of altering the number of platelets per a given volume of blood, inhibiting platelet function, including but not limited to platelet adhesion, aggregation or factor release, or reducing platelet count in patients with abnormally high levels in certain hematological malignancies to levels approximating normal levels capable of impacting negatively upon the formation of blood clots, and decreasing blood viscosity. Blood modifiers useful in the present invention include but are not limited to anti-coagulants, antithrombotic agents, fibrinogen receptor antagonists, platelet inhibitors, platelet aggregation inhibitors, lipoprotein-associated coagulation inhibitor, hemorrheologic agents, Factor VIIa inhibitors, Factor Xa inhibitors, and combinations thereof and are meant to exclude HMG CoA reductase inhibitors and are chemically or structurally different from sterol absorption inhibitors as discussed below, for example, they contain one or more different atoms, have a different arrangement of atoms or a different number of one or more atoms than the sterol absorption inhibitor(s) discussed below.

"Anti-coagulant agents" are agents which inhibit the coagulation pathway by impacting negatively upon the production, deposition, cleavage and/or activation of factors essential in the formation of a blood clot. Useful anti-coagulant agents include but are not limited to argatroban (2-Piperidinecarboxylic acid, 1-[(2S)-5-[(aminoiminomethyl)amino]-1-oxo-2-[[(1,2,3,4-tetrahydro-3-methyl-8-quinolinyl)sulfonyl]amino]pentyl]-4-methyl-, CAS RN 74863-84-6); bivalirudin (L-Leucine, D-phenylalanyl-L-prolyl-L-arginyl-L-prolylglycylglycylglycylglycyl-L-asparaginylglycyl-L-.alpha.-aspartyl-L-phenylalanyl-L-.alpha.-glutamyl-L-.alpha.-glutamyl-L-isoleucyl-L-prolyl-L-.alpha.-glutamyl-L-.alpha.-glutamyl-L-tyrosyl- CAS RN 128270-60-0); dalteparin sodium (heparin) for example, FRAGMIN® Injection available from Pharmacia & Upjohn)); desirudin (Hirudin (Hirudo medicinalis isoform HV1), 63-desulfo CAS RN 120993-53-5)); dicumarol (2H-1-Benzopyran-2-one, 3,3'-methylenebis[4-hydroxy- CAS RN 66-76-2 for example, MEBARAL® Tablets available from Sanofi-Synthelabo); lyapolate sodium (Ethenesulfonic acid, homopolymer, sodium salt CAS RN 25053-27-4); nafamostate mesylate (Benzoic acid, 4-[(aminoiminomethyl)amino]-, 6-(aminoiminomethyl)-2-naphthalenyl ester, dimethanesulfonate CAS RN 82956-11-4);phenprocoumon (2H-1-Benzopyran-2-one, 4-hydroxy-8-methoxy-3-(1-phenylpropyl)- CAS RN 132605-68-6); tinzaparin sodium (Heparin, sodium salt, CAS RN 9041-08-1, for example INNOHEP® Injection® available from DuPont); warfarin sodium (3-((alpha)-acetonylbenzyl)-4-hydroxycoumarin, CAS RN 129-06-6, for example, Coumadin for Injection available from DuPont).

"Anti-thrombotic" agents are agents which prevent the formation of a blood thrombus. A thrombus is an aggregation of blood factors, primarily platelets and fibrin with entrapment of cellular elements, frequently causing vascular obstruction at the point of its formation. Suitable examples of anti-thrombotic agents include anagrelide hydrochloride (6,7-dichloro-1,5-dihydroimidazo[2,1-b]quinazolin-2(3H)-one monohydrochloride monohydrate) for example AGRYLIN® available from Shire US); Tinzaparin sodium as described above; cilostazol (6-[4-(1-cyclohexyl-1 *H-*tetrazol-5-yl)butoxy]-3,4-dihydro-2(1 *H*)-quinolinone, CAS-73963-72-1, for example PLETAL® Tablets (Pharmacia & Upjohn) ;Dalteparin sodium (as described above); danaparoid sodium, for example, ORGARAN® Injection available from Organon; Abciximab is the (Fab fragment of the chimeric human-murine monoclonal antibody 7E3. binds to the glycoprotein (GP) IIb/IIIa ((alpha) IIb (beta) 3) receptor of human platelets and inhibits platelet aggregation. Abciximab also binds to the vitronectin ((alpha) ᵥ(beta) ₃) receptor found on platelets and vessel wall endothelial and smooth muscle cells, for example Abciximab, REOPRO® available from Lily); ifetroban (Benzenepropanoic acid, 2-[[(1S,2R,3S,4R)-3-[4-[(pentylamino)carbonyl]-2-oxazolyl]-7 oxabicyclo[2.2.1]hept-2-yl]methyl]- CAS RN 143443-90-7); Bivalirudin as described above; Cilostazol as described above; efegatran sulfate (L-Prolinamide, N-methyl-D-phenylalanyl-N-[(1S)-4-[(aminoiminomethyl)amino]-1-formylbutyl]-, sulfate (1:1) CAS RN 126721-07-1); dazoxiben hydrochloride (Benzoic acid, 4-[2-(1 H-imidazol-1-yl)ethoxy]-, monohydrochloride CAS RN 74226-22-5); danaparoid sodium (a low molecular weight heparinoid, a mixture of the sodium salts of heparan sulfate (approximately 84%), dermatan sulfate (approximately 12%), and chondroitin sulfate (approximately 4%). It is derived from hog intestinal mucosa); lotrafiban hydrochloride (1 H-1,4-Benzodiazepine-2-acetic acid, 7-([4,4'-bipiperidin]-1-ylcarbonyl)-2,3,4,5-tetrahydro-4-methyl-3-oxo-, monohydrochloride, (2S)-)CAS RN 179599-82-7); ifetroban sodium(Benzenepropanoic acid, 2-[[(1 S,2R,3S,4R)-3-[4-[(pentylamino)carbonyl]-2-oxazolyl]-7-oxabicyclo[2.2.1]hept-2-yl]methyl]-, monosodium salt, CAS RN 156715-37-6); lamifiban(Acetic acid, [[1-[(2S)-2-[[4-(aminoiminomethyl)benzoyl)amino]-3-(4-hydroxyphenyl)-1-oxopropyl]-4-piperidinyl]oxy]- , CAS RN 144412-49-7); fluretofen (1,1'-Biphenyl, 4'-ethynyl-2-fluoro-CAS RN 56917-29-4); enoxaparin sodium (Heparin, sodium salt, CAS RN 9041-08-1); orbofiban acetate hydrate (beta.-Alanine, N-[[[(3S)-1-[4-(aminoiminomethyl)phenyl]-2-oxo-3-pyrrolidinyl]amino]carbonyl]-, ethyl ester, acetate, hydrate (4:4:1),CAS RN 165800-05-5));napsagatran (Glycine, N-[[(3S)-1-(aminoiminomethyl)-3-piperidinyl]methyl]-N2-(2-naphthalenylsulfonyl)-L-asparaginyl-N-cyclopropyl-, CAS RN 154397-77-0); roxifiban acetate(L-Alanine, 3-[[[(5R)-3-[4-(aminoiminomethyl)phenyl]-4,5-dihydro-5-isoxazolyl]acetyl]amino]-N-(butoxycarbonyl)-,methyl ester, monoacetate, CAS RN 176022-59-6); sibrafiban(Acetic acid, [[1-[(2S)-2-[[4-[(Z)-amino(hydroxyimino)methyl]benzoyl]amino]-1-oxopropyl]-4-piperidinyl]oxy]-, ethyl ester, CAS RN 172927-65-0);zolimomab aritox, (Immunoglobulin G1, anti-(human CD5 (antigen) heavy chain) (mouse monoclonal H65-RTA .gamma. 1-chain), disulfide with mouse monoclonal H65-RTA light chain, dimer, disulfide with ricin (castor bean A-chain), CAS RN 141483-72-9); trifenagrel (Ethanamine, 2-[2-(4,5-diphenyl-1H-imidazol-2-yl)phenoxy]-N,N-dimethyl- ,CAS RN 84203-09-8).

"Fibrinogen receptor antagonists" are those agents which inhibit the common pathway of platelet aggregation. Suitable fibrinogen receptor antagonists include but are not limited toroxifiban acetate as described above; lotrafiban hydrochloride as described above, sibrafiban as described above, monoclonal antibody 7E3 (Fab fragment of the chimeric human-murine monoclonal antibody 7E3. binds to the glycoprotein (GP) IIb/IIIa ((alpha) _{IIb} (beta) ₃) receptor of human platelets and inhibits platelet aggregation); orbofiban, (beta.-Alanine, N-[[[(3S)-1-[4-(aminoiminomethyl)phenyl]-2-oxo-3-pyrrolidinyl]amino]carbonyl]-, ethyl ester, CAS RN 163250-90-6); xemilofiban (4-Pentynoic acid, 3-[[4-[[4-(aminoiminomethyl)phenyl]amino]-1,4-dioxobutyl]amino]-, ethyl ester, (3S)-,CAS RN 149820-74-6); fradafiban, (3-Pyrrolidineacetic acid, 5-[[[4'-(aminoiminomethyl)[1,1'-biphenyl]-4-yl]oxy]methyl]-2-oxo-, (3S,5S)-,CAS RN 148396-36-5); tirofiban (L-Tyrosine, N-(butylsulfonyl)-O-[4-(4-piperidinyl)butyl]-, CAS RN 144494-65-5, for example AGGRASTATt® Injection Premixed available from Merck.

"Platelet inhibitors" are those agents that impair the ability of mature platelets to perform their normal physiological roles (i.e., their normal function). Platelets are normally involved in a number of physiological processes such as adhesion, for example, to cellular and non-cellular entities, aggregation, for example, for the purpose of forming a blood clot, and release of factors such as growth factors (e.g. platelet-derived growth factor (PDGF)) and platelet granular components. Suitable platelet inhibitors include, but are not limited to clopidogrel bisulfate, (Thieno[3,2-c]pyridine-5(4H)-acetic acid, alpha.-(2-chlorophenyl)-6,7-dihydro-, methyl ester, (alpha. S)-, sulfate (1:1), for example, PLAVIX® Tablets such as those available from Sanofi-Synthelabo); indomethacin, such as INDOCIN® I.V. (Indomethacin Sodium Trihydrate) available from Merck);mefenamate, (for example, Ponstel® Kapseals (mefenamic acid) 2-{(2,3-dimethylphenyl) amino-N-2,3-xylylanthranilic acid available from First Horizan); Ticlopidine hydrochloride, (Thieno[3,2-c]pyridine, 5-[(2-chlorophenyl)methyl]-4,5,6,7-tetrahydro-, hydrochloride, for example TICLID Tablets® , available from Roche Laboratories); epoprostenol sodium, (Prosta-5,13-dien-1-oic acid, 6,9-epoxy-11,15-dihydroxy-, monosodium salt, (5Z,9.alpha.,11.alpha., 13E, 15S)-CAS RN 61849-14-7, for example, FLOLAN® for Injection available from Glaxo Wellcome); aspirin, Benzoic acid, 2-(acetyloxy)-CAS RN 50-78-2); epoprostenol, (Prosta-5,13-dien-1-oic acid, 6,9-epoxy-11,15-dihydroxy-, (5Z,9.alpha.,11.alpha.,13E,15S)-, CAS RN 35121-78-9); naproxen (2-Naphthaleneacetic acid, 6-methoxy-.alpha.-methyl-, (.alpha.S)- CAS RN 22204-53-1, for example, EC-NAPROSYN® Delayed-Release Tablets available from Roche Laboratories); buprofen, (Benzeneacetic acid, .alpha.-methyl-4-(2-methylpropyl)-, CAS RN 15687-27-1); droxicam, (2H,5H-1,3-Oxazino[5,6-c][1,2]benzothiazine-2,4(3H)-dione, 5-methyl-3-(2-pyridinyl)-, 6,6-dioxide, CAS RN 90101-16-9); diclofenac, (Benzeneacetic acid, 2-[(2,6-dichlorophenyl)amino]- CAS RN 15307-86-5 for example, Arthrotec® Tablets available from Searle); sulfinpyrazone, (3,5-Pyrazolidinedione, 1,2-diphenyl-4-[2-(phenylsulfinyl)ethyl]- CAS Registry Number 57-96-5, for example, Sectral® Capsules available from Wyeth-Ayerst); piroxicam, (2H-1,2-Benzothiazine-3-carboxamide, 4-hydroxy-2-methyl-N-2-pyridinyl-, 1,1-dioxide, CAS Registry Number 36322-90-4, for example FELDENECapsules® available from Pfizer); dipyridamole, (Ethanol, 2,2',2",2"'-[(4,8-di-1-piperidinylpyrimido[5,4-d]pyrimidine-2,6-diyl)dinitrilo]tetrakis-CAS Registry Number 58-32-2, for example Aggrenox® Capsules available from Boehringer Ingelheim); lexipafant,(L-Leucine, N-methyl-N-[[4-[(2-methyl-1 H-imidazo[4,5-c]pyridin-1-yl)methyl]phenyl]sulfonyl]-, ethyl ester, CAS Registry Number 139133-26-9); apafant Morpholine, 4-[3-[4-(2-chlorophenyl)-9-methyl-6H-thieno[3,2-f][1,2,4]triazolo[4,3-a][1,4]diazepin-2-yl]-1-oxopropyl]-, CAS Registry Number 105219-56-5).

"Platelet aggregation inhibitors" as used herein refer to those compounds which reduce or halt the ability of platelets to associate physically with themselves or with other cellular and non-cellular components, thereby precluding the ability of a platelet to form a thrombus. Suitable platelet aggregation inhibitors include but are not limited beraprost, (1 H-Cyclopenta[b]benzofuran-5-butanoic acid, 2,3,3a,8b-tetrahydro-2-hydroxy-1-(3-hydroxy-4-methyl-1-octen-6-ynyl)-, CAS RN 88430-50-6); acadesine, (1H-Imidazole-4-carboxamide, 5-amino-1-.beta.-D-ribofuranosyl- , CAS RN 2627-69-2);beraprost sodium, (1 H-Cyclopenta[b]benzofuran-5-butanoic acid, 2,3,3a,8b-tetrahydro-2-hydroxy-1-(3-hydroxy-4-methyl-1-octen-6-ynyl)-, monosodium salt, CAS RN 88475-69-8); ciprostene calcium, (Pentanoic acid, 5-[(3aS,5R,6R,6aR)-hexahydro-5-hydroxy-6-[(1E,3S)-3-hydroxy-1-octenyl]-3a-methyl-2(1H)-pentalenylidene]-, calcium salt (2:1), (5Z)- CAS Registry Number 81703-55-1),itazigrel, (Thiazole, 4,5-bis(4-methoxyphenyl)-2-(trifluoromethyl)-CAS Registry Number 70529-35-0); lifarizine(Piperazine, 1-(diphenylmethyl)-4-[[5-methyl-2-(4-methylphenyl)-1H-imidazol-4-yl]methyl]-),CAS Registry Number 119514-66-8);oxagrelate, (6-Phthalazinecarboxylic acid, 3,4-dihydro-1-(hydroxymethyl)-5,7-dimethyl-4-oxo-, ethyl ester, CAS Registry Number 56611-65-5).

"Hemorrheologic agent" as used herein describes those compounds which improve the flow properties of blood by decreasing its viscosity. A suitable hemorrheologic agent of the present invention is pentoxifylline (1 H-Purine-2,6-dione, 3,7-dihydro-3,7-dimethyl-1-(5-oxohexyl)- (9Cl) (CA INDEX NAME) Theobromine, 1-(5-oxohexyl)- ,CAS Registry Number 6493-05-6 for example, TRENTALI® Tablets available from Aventis).

Pentoxifylline and its metabolites (which can be useful in the present invention) improve the flow properties of blood by decreasing its viscosity. In patients with chronic peripheral arterial disease, this increases blood flow to the affected microcirculation and enhances tissue oxygenation. The precise mode of action of pentoxifylline and the sequence of events leading to clinical improvement are still to be defined. Pentoxifylline administration has been shown to produce dose-related hemorrheologic effects, lowering blood viscosity, and improving erythrocyte flexibility. Leukocyte properties of hemorrheologic importance have been modified in animal and in vitro human studies. Pentoxifylline has been shown to increase leukocyte deformability and to inhibit neutrophil adhesion and activation. Tissue oxygen levels have been shown to be significantly increased by therapeutic doses of pentoxifylline in patients with peripheral arterial disease.

Lipoprotein-associated coagulation inhibitor (LACI) is a serum glycoprotein with a molecular weight of 38,000 Kd useful as a blood modifier of the present invention It is also known as tissue factor inhibitor because it is a natural inhibitor of thromboplastin (tissue factor) induced coagulation (US Patent Nos., 5,110,730 and 5,106,833 described tissue factor and are hereby incorporated by reference their entireties). LACI is a protease inhibitor and has 3 Kunitz domains, two of which are known to interact with factors VII and Xa respectively, while the function of the third domain is unknown. Many of the structural features of LACI can be deduced because of its homology with other well studies proteases. LACI is not an enzyme, so it probably inhibits its protease target in a stoichiometric manner; namely, one of the domains of LACI inhibits one protease molecule (see US Patent No. 6,063,74 herein incorporated by reference).

"Factor VIIa Inhibitors" as used herein are those agents which inhibit activated Factor VIIa from acting to contribute to the formation of a fibrin clot. Suitable Factor VIIa Inhibitors include but are not limited to, 4H-31-benzoxazin-4-ones, 4H-3,1-benzoxazin-4-thiones, quinazolin-4-thiones, benzothiazin-4-ones described in US Patent 6,180, 625, imidazolyl-boronic acid-derived peptide analogues as described in US Patent No. 5,639,739, TFPI-derived peptides described in US Patent No. 6,180,625.

Additional suitable Factor VIIa Inhibitors include but are not limited to Naphthalene-2-sulfonic acid {1-[3-(aminoiminomethyl)-benzyl]-2-oxo-pyrrolidin-3-(S)-yl} amide trifluoroacetate, dibenzofuran-2-sulfoic acid {1-[3-(aminomethyl)-benzyl]-5-oxo-pyrrolidin-3-yl}-amide, tolulene-4-sulfonic acid {1-[3-(aminoiminomethyl)-benzyl]-2-oxo-pyrrolidin-3-(S)-yl}-amide tribluoroacetate, 3,4-dihydro-1 H-isoquinoline-2-sulfonic acid {1-[3-(aminoiminomethyl)-benzyl]-2-oxo-pyrrolin-3-(S)-yl}-amide tribluoroacetate or combinations thereof.

"Factor Xa inhibitors" as used herein are those agents which inhibit activated Factor X from acting to contribute to the formation of a fibrin clot. Suitable agents for use in the present invention as Factor Xa inhibitors include but are not limited to disubstituted pyrazolines, disubstituted triazolines as described in US Patent No. 6,191,159, lipoprotein-associated coagulation inhibitor (LACI) (as described above), low molecular weight heparins described as below, heparinoids described as below, benzimidazolines, benzoxazolinones, bensopiperazinones, indanones, as described in US Patent No. 6,207,697, dibasic (amidinoaryl)propanoic acid derivatives as described in J. Med. Chem. 37:1200-1207 (1994), bis-arlysulfonylaminobenzamide derivatives as described in US Patent No. 5,612,378, amidinophenyl-pyrrolidines, amidinophenyl-pyrrolines, amidinophenyl-isoxazolidines as described in US Patent No. 6,057,342, amidinoindoles, amidinoazoles as described in US Patent No. 6,043,257.peptidic Factor Xa inhibitors as described below, substituted n-[(aminoiminomethyl)phenyl]propylamides, substituted n-[(aminomethyl)phenyl]propylamides as described in US Patent No. 6,080,767 or combinations thereof.

Peptidic factor Xa inhibitors such as the leech-derived, 119-amino acid protein antistasin and the soft tick derived protein TAP (tick anticoagulant peptide) accelerate clot lysis and prevented reocclusion when given as adjuncts to thrombolysis (Mellott et al., Circulafion Research 70:1152-1160 (1992); Sitko et al., Circulation 85:805-815 (1992)). US Patent No. 5,385,885 issued Jan. 31, 1995 discloses smooth muscle cell proliferation inhibitory activity of both tick anticoagulant peptide and antistasin. The peptide ecotin is another selective, reversible, tight-binding inhibitor of factor Xa that exhibits protein anticoagulant activity (Seymour et al., Biochemistry 33:3949-3959 (1994); PCT Published Application WO 94/20535, 09/14/1994). Ixodidae, argasin and ancylostomatin are other representative peptidic factor Xa inhibitors isolated from animals that feed on blood (Markwardt, Thrombosis and Hemostasis 72: 477-479 (1994).

These non-limiting examples of peptidic Factor Xa inhibitors which may be used in the present invention are listed below with their CAS registry Number. These include Proteinase inhibitor, antistasin, CAS Registry Number 110119-38-5; tick anticoagulant peptide, (Proteinase inhibitor, TAP) CAS Registry Number 129737-17-3; ecotin, (Proteinase inhibitor, ecotin) CAS Registry Number 87928-05; argasin , CAS Registry Number 53092-89-0 ;ancylostomatin , CAS Registry Number 11011-09-9; Ixodidae (as described in Markwardt, 1994).

"Low molecular weight heparins" as used herein refer to agents derived from heparins which reduces the incidence of bleeding when compared with standard heparin. Heparins are glycosaminoglycans. MW range from 2000-10000. They may be produced from porcine intestinal mucosa and except for nadroparan, are all sodium salts. A suitable heparinoid of the present invention includes but is not limited to enoxaparin, nardroparin, dalteparin, certroparin, parnaparin, reviparin, tinzaparin and combinations thereof.

"Heparinoid" as used herein refers to a modified form of heparin which reduces the incidence of bleeding when compared with standard heparin. A suitable heparinoid of the present invention includes but is not limited to Danaparoid CAS Registry Number 308068-55-5, (for example, Orgaran Injection Organon)

Generally, a total dosage of the above-described blood modifier agents or medications can range from 1 to 3,000 mg/day, desirably from about 1 to 1,000 mg/day and more desirably from about 1 to 200 mg/day in single or 2-4 divided doses.

Treatments can be administered in a therapeutically effective amount of blood modifier to treat the specified condition, for example in a daily dose preferably ranging from about 1 to about 1000 mg per day, and more preferably about 5 to about 200 mg per day, given in a single dose or 2-4 divided doses. The exact dose, however, is determined by the attending clinician and is dependent on such factors as the potency of the compound administered, the age, weight, condition and response of the patient.

The term "therapeutically effective amount" means that amount of a therapeutic agent of the composition, such as the blood modifiers, sterol absorption inhibitor(s) and other pharmacological or therapeutic agents described below, that will elicit a biological or medical response of a tissue, system, animal or mammal that is being sought by the administrator (such as a researcher, doctor or veterinarian) which includes alleviation of the symptoms of the condition or disease being treated and the prevention, slowing or halting of progression of the condition (for example a vascular condition as discussed above).

As used herein, "combination therapy" or "therapeutic combination" means the administration of two or more therapeutic agents, such as blood modifiers and sterol absorption inhibitor(s), to prevent or treat vascular conditions. Such administration includes coadministration of these therapeutic agents in a substantially simultaneous manner, such as in a single tablet or capsule having a fixed ratio of active ingredients or in multiple, separate capsules for each therapeutic agent. Also, such administration includes use of each type of therapeutic agent in a sequential manner. In either case, the treatment using the combination therapy will provide beneficial effects in treating vascular and other conditions as discussed above. A potential advantage of the combination therapy disclosed herein may be a reduction in the required amount of an individual therapeutic compound or the overall total amount of therapeutic compounds that are effective in treating blood modifiers. By using a combination of therapeutic agents, the side effects of the individual compounds can be reduced as compared to a monotherapy, which can improve patient compliance. Also, therapeutic agents can be selected to provide a broader range of complimentary effects or complimentary modes of action.

As discussed above, the compositions, pharmaceutical compositions and therapeutic combinations of the present invention comprise one or more sterol absorption inhibitor(s), such as the substituted azetidinone sterol absorption inhibitors or substituted β-lactam sterol absorption inhibitors discussed in detail below. As used herein, "sterol absorption inhibitor" means a compound capable of inhibiting the absorption of one or more sterols, including but not limited to cholesterol, phytosterols (such as sitosterol, campesterol, stigmasterol and avenosterol), 5α-stanols (such as cholestanol, 5α-campestanol, 5α-sitostanol), and mixtures thereof, when administered in a therapeutically effective (sterol absorption inhibiting) amount to a mammal or human.

In a preferred embodiment, sterol absorption inhibitors useful in the compositions, therapeutic combinations and methods of the present invention are represented by Formula (I) below: 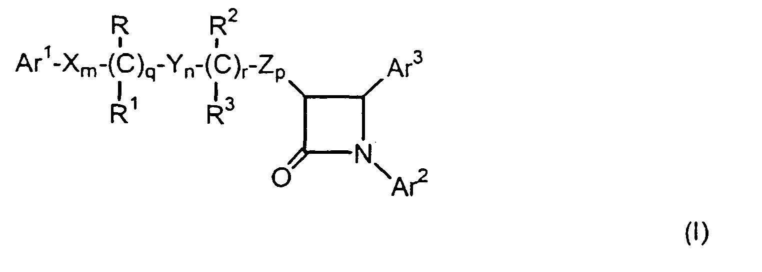 or isomers of the compounds of Formula (I), or pharmaceutically acceptable salts or solvates of the compounds of Formula (1) or of the isomers of the compounds of Formula (I), or prodrugs of the compounds of Formula (I) or of the isomers, salts or solvates of the compounds of Formula (I), wherein, in Formula (I) above:
Ar¹ and Ar² are independently selected from the group consisting of aryl and R⁴-substituted aryl;
Ar³ is aryl or R⁵-substituted aryl;
X; Y and Z are independently selected from the group consisting of -CH₂-, -CH(lower alkyl)- and -C(dilower alkyl)-;
R and R² are independently selected from the group consisting of -OR⁶, -O(CO)R⁶, -O(CO)OR⁹ and -O(CO)NR⁶R⁷;
R¹ and R³ are independently selected from the group consisting of hydrogen, lower alkyl and aryl;
q is 0 or 1; r is 0 or 1; m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
R⁴ is 1-5 substituents independently selected from the group consisting of lower alkyl, -OR⁶, -O(CO)R⁶, -O(CO)OR⁹, ,-O(CH₂)₁₋₅OR⁶, O(CO)NR⁶R⁷, -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶, -SO₂NR⁶R⁷, S(O)₀₋₂R⁹, -O(CH₂)₁₋₁₀-COOR⁶, -O(CH₂)₁₋₁₀CONR⁶R⁷, -(lower alkylene)COOR⁶, ,-CH=CH-COOR⁶, -CF₃, -CN, -NO₂ and halogen;
R⁵ is 1-5 substituents independently selected from the group consisting of -OR⁶, -O(CO)R⁶, -O(CO)OR⁹, -O(CH₂)₁₋₅OR⁶, -O(CO)NR⁶R⁷, -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶,-SO₂NR⁶R⁷, S(O)₀₋₂R⁹, -O(CH2)₁₋₁₀-COOR⁶, O(CH₂)₁₋₁₀CONR⁶R⁷, -(lower alkylene)COOR⁶ and
   -CH=CH-COOR⁶;
R⁶, R⁷ and R⁸ are independently selected from the group consisting of hydrogen, lower alkyl, aryl and aryl-substituted lower alkyl; and
R⁹ is lower alkyl, aryl or aryl-substituted lower alkyl.
Preferably, R⁴ is 1-3 independently selected substituents, and R⁵ is preferably 1-3 independently selected substituents.

As used herein, the term "alkyl" or "lower alkyl" means straight or branched alkyl chains having from 1 to 6 carbon atoms and "alkoxy" means alkoxy groups having 1 to 6 carbon atoms. Non-limiting examples of lower alkyl groups include, for example methyl, ethyl, propyl, and butyl groups.

"Alkenyl" means straight or branched carbon chains having one or more double bonds in the chain, conjugated or unconjugated. Similarly, "alkynyl" means straight or branched carbon chains having one or more triple bonds in the chain. Where an alkyl, alkenyl or alkynyl chain joins two other variables and is therefore bivalent, the terms alkylene, alkenylene and alkynylene are used.

"Cycloalkyl" means a saturated carbon ring of 3 to 6 carbon atoms, while "cycloalkylene" refers to a corresponding bivalent ring, wherein the points of attachment to other groups include all positional isomers.

"Halogeno" refers to fluorine, chlorine, bromine or iodine radicals.

"Aryl" means phenyl, naphthyl, indenyl, tetrahydronaphthyl or indanyl.

"Phenylene" means a bivalent phenyl group, including ortho, meta and parasubstitution.

The statements wherein, for example, R, R¹, R² and R³, are said to be independently selected from a group of substituents, mean that R, R¹, R² and R³ are independently selected, but also that where an R, R¹, R² and R³ variable occurs more than once in a molecule, each occurrence is independently selected (e.g., if R is -OR⁶, wherein R⁶ is hydrogen, R² can be -OR⁶ wherein R⁶ is lower alkyl). Those skilled in the art will recognize that the size and nature of the substituent(s) will affect the number of substituents that can be present.

Compounds of the invention have at least one asymmetrical carbon atom and therefore all isomers, including enantiomers, stereoisomers, rotamers, tautomers and racemates of the compounds of Formulae (I-XI) (where they exist) are contemplated as being part of this invention. The invention includes d and I isomers in both pure form and in admixture, including racemic mixtures. Isomers can be prepared using conventional techniques, either by reacting optically pure or optically enriched starting materials or by separating isomers of a compound of the Formulae I-XI. Isomers may also include geometric isomers, e.g., when a double bond is present.

Those skilled in the art will appreciate that for some of the compounds of the Formulas I-XI, one isomer will show greater pharmacological activity than other isomers.

Compounds of the invention with an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

As used herein, "solvate" means a molecular or ionic complex of molecules or ions of solvent with those of solute (for example, one or more compounds of Formulae I-XI, isomers of the compounds of Formulae I-XI, or prodrugs of the compounds of Formulae I-XI). Non-limiting examples of useful solvents include polar, protic solvents such as water and/or alcohols (for example methanol).

As used herein, "prodrug" means compounds that are drug precursors which, following administration to a patient, release the drug *in vivo* via some chemical or physiological process (e.g., a prodrug on being brought to the physiological pH or through enzyme action is converted to the desired drug form).

Preferred compounds of Formula (I) are those in which Ar¹ is phenyl or R⁴-substituted phenyl, more preferably (4-R⁴)-substituted phenyl. Ar² is preferably phenyl or R⁴-substituted phenyl, more preferably (4-R⁴)-substituted phenyl. Ar³ is preferably R⁵-substituted phenyl, more preferably (4-R⁵)-substituted phenyl. When Ar¹ is (4-R⁴)-'substituted phenyl, R⁴ is preferably a halogen. When Ar² and Ar³ are R⁴- and R⁵-substituted phenyl, respectively, R⁴ is preferably halogen or -OR⁶ and R⁵ is preferably -OR⁶, wherein R⁶ is lower alkyl or hydrogen. Especially preferred are compounds wherein each of Ar¹ and Ar² is 4-fluorophenyl and Ar³ is 4-hydroxyphenyl or 4-methoxyphenyl.

X, Y and Z are each preferably -CH₂-. R¹ and R³ are each preferably hydrogen. R and R² are preferably -OR⁶ wherein R⁶ is hydrogen, or a group readily metabolizable to a hydroxyl (such as -O(CO)R⁶, -O(CO)OR⁹ and -O(CO)NR⁶R⁷, defined above).

The sum of m, n, p, q and r is preferably 2, 3 or 4, more preferably 3. Preferred are compounds wherein m, n and r are each zero, q is 1 and p is 2.

Also preferred are compounds of Formula (I) in which p, q and n are each zero, r is 1 and m is 2 or 3. More preferred are compounds wherein m, n and r are each zero, q is 1, p is 2, Z is -CH₂ and R is -OR⁶, especially when R⁶ is hydrogen.

Also more preferred are compounds of Formula (I) wherein p, q and n are each zero, r is 1, m is 2, X is -CH₂ and R² is -OR⁶, especially when R⁶ is hydrogen.

Another group of preferred compounds of Formula (I) is that in which Ar¹ is phenyl or R⁴-substituted phenyl, Ar² is phenyl or R⁴-substituted phenyl and Ar³ is R⁵-substituted phenyl. Also preferred are compounds in which Ar¹ is phenyl or R⁴-substituted phenyl, Ar² is phenyl or R⁴-substituted phenyl, Ar³ is R⁵-substituted phenyl, and the sum of m, n, p, q and r is 2, 3 or 4, more preferably 3. More preferred are compounds wherein Ar¹ is phenyl or R⁴-substituted phenyl, Ar² is phenyl or R⁴-substituted phenyl, Ar³ is R⁵-substituted phenyl, and wherein m, n and r are each zero, q is 1 and p is 2, or wherein p, q and n are each zero, r is 1 and m is 2 or 3.

In a preferred embodiment, a sterol inhibitor of Formula (I) useful in the compositions, therapeutic combinations and methods of the present invention is represented by Formula (II) (ezetimibe) below: 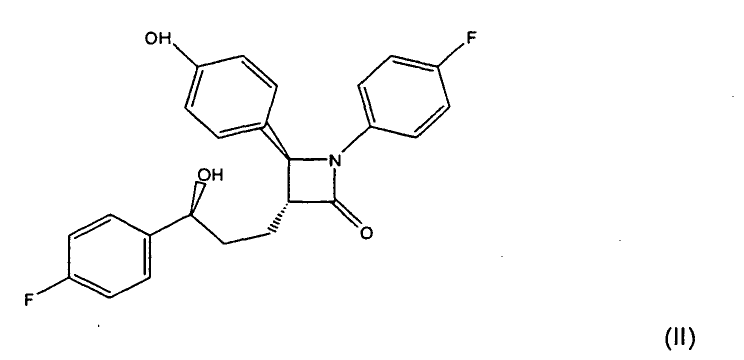 or pharmaceutically acceptable salts or solvates of the compound of Formula (II), or prodrugs of the compound of Formula (II) or of the salts or solvates of the compound of Formula (II).

Compounds of Formula I can be prepared by a variety of methods well known to those skilled in the art, for example such as are disclosed in -U.S. Patents Nos. 5,631,365, 5,767,115, 5,846,966, 6,207,822, U.S. Provisional Patent Application No. 60/279,288 filed March 28, 2001 and PCT Patent Application WO 93/02048, each of which is incorporated herein by reference, and in the Example below. For example, suitable compounds of Formula I can be prepared by a method comprising the steps of:
(a) treating with a strong base a lactone of the Formula A or B: 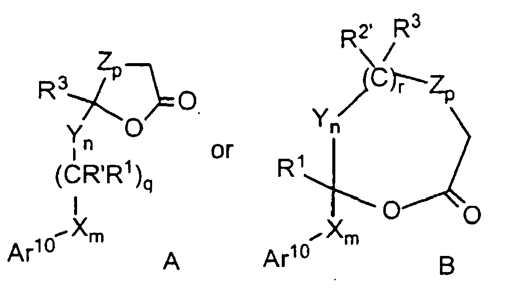 wherein R' and R2' are R and R², respectively, or are suitably protected hydroxy groups; Ar¹⁰ is Ar¹, a suitably protected hydroxy-substituted aryl or a suitably protected amino-substituted aryl; and the remaining variables are as defined above for Formula I, provided that in lactone of formula B, when n and r are each zero, p is 1-4;
(b) reacting the product of step (a) with an imine of the formula 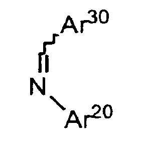 wherein Ar²⁰ is Ar², a suitably protected hydroxy-substituted aryl or a suitably protected amino-substituted aryl; and Ar³⁰ is Ar³, a suitably protected hydroxy-substituted aryl or a suitably protected amino-substituted aryl;
(c) quenching the reaction with an acid;
(d) optionally removing the protecting groups from R', R^{2'}, Ar¹⁰, Ar²⁰ and Ar³⁰, when present; and
(e) optionally functionalizing hydroxy or amino substituents at R, R², Ar¹, Ar² and Ar³.

Using the lactones shown above, compounds of Formula IA and IB are obtained as follows: 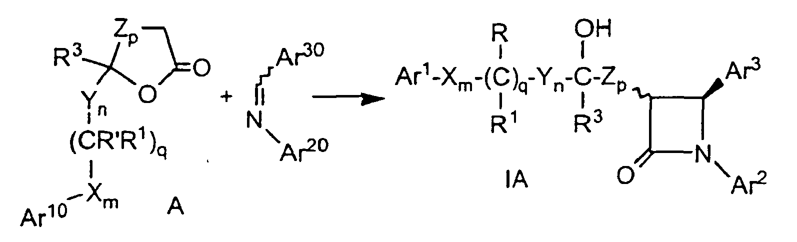 wherein the variables are as defined above; and 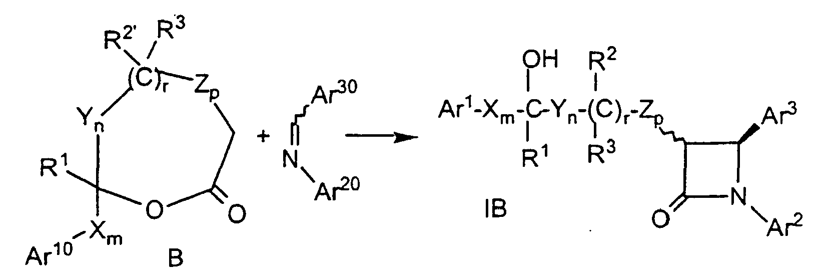 wherein the variables are as defined above.

Alternative sterol absorption inhibitors useful in the compositions, therapeutic combinations and methods of the present invention are represented by Formula (III) below: 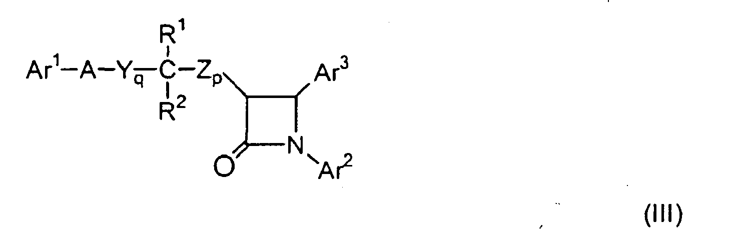 or isomers of the compounds of Formula (III), or pharmaceutically acceptable salts or solvates of the compounds of Formula (III) or of the isomers of the compounds of Formula (III), or prodrugs of the compounds of Formula (III) or of the isomers, salts or solvates of the compounds of Formula (III), wherein, in Formula (III) above:
Ar¹ is R³-substituted aryl;
Ar ² is R⁴-substituted aryl;
Ar³ is R⁵-substituted aryl;
Y and Z are independently selected from the group consisting of -CH₂-, -CH(lower alkyl)- and -C(dilower alkyl)-;
A is selected from -O-, -S-, -S(O)- or -S(O)₂-;
R¹ is selected from the group consisting of -OR⁶, -O(CO)R⁶, -O(CO)OR⁹ and -O(CO)NR⁶R⁷; R² is selected from the group consisting of hydrogen, lower alkyl and aryl; or R¹ and R² together are =O;
q is 1, 2 or 3;
p is 0, 1, 2, 3 or 4;
R⁵ is 1-3 substituents independently selected from the group consisting of -OR⁶, -O(CO)R⁶, -O(CO)OR⁹, -O(CH₂)₁₋₅OR⁹, -O(CO)NR⁶R⁷, -NR⁶R⁷, -NR⁶(CO)R⁷, -NR⁶(CO)OR⁹, -NR⁶(CO)NR⁷R⁸, -NR⁶SO₂-lower alkyl, -NR⁶SO₂-aryl, -CONR⁶R⁷, -COR⁶, -SO₂NR⁶R⁷, S(O)₀₋₂-alkyl, S(O)₀₋₂-aryl, -O(CH₂)₁₋₁₀-COOR⁶, -O(CH₂)₁₋₁₀CONR⁶R⁷, o-halogeno, m-halogeno, o-lower alkyl, m-lower alkyl, -(lower alkylene)-COOR⁶, and -CH=CH-COOR⁶;
R³ and R⁴ are independently 1-3 substituents independently selected from the group consisting of R⁵, hydrogen, p-lower alkyl, aryl, -NO₂, -CF₃ and p-halogeno;
R⁶, R⁷ and R⁸ are independently selected from the group consisting of hydrogen, lower alkyl, aryl and aryl-substituted lower alkyl; and R⁹ is lower alkyl, aryl or aryl-substituted lower alkyl.

Preferred compounds of Formula I include those in which Ar¹ is R³-substituted phenyl, especially (4-R³)-substituted phenyl. Ar² is preferably R⁴-substituted phenyl, especially (4-R⁴)-substituted phenyl. Ar³ is preferably R⁵-substituted phenyl, especially (4-R⁵)-substituted phenyl. Mono-substitution of each of Ar¹, Ar² and Ar³ is preferred.

Y and Z are each preferably -CH₂-. R² is preferably hydrogen. R¹ is preferably -OR⁶ wherein R⁶ is hydrogen, or a group readily metabolizable to a hydroxyl (such as -O(CO)R⁶, -O(CO)OR⁹ and -O(CO)NR⁶R⁷, defined above). Also preferred are compounds wherein R¹ and R² together are =O.

The sum of q and p is preferably 1 or 2, more preferably 1. Preferred are compounds wherein p is zero and q is 1. More preferred are compounds wherein p is zero, q is 1, Y is -CH₂- and R¹ is -OR⁶, especially when R⁶ is hydrogen.

Another group of preferred compounds is that in which Ar¹ is R³-substituted phenyl, Ar² is R⁴-substituted phenyl and Ar³ is R⁵-substituted phenyl

Also preferred are compounds wherein Ar¹ is R³-substituted phenyl, Ar² is R⁴-substituted phenyl, Ar³ is R⁵-substituted phenyl, and the sum of p and q is 1 or 2, especially 1. More preferred are compounds wherein Ar¹ is R³-substituted phenyl, Ar² is R⁴-substituted phenyl, Ar³ is R⁵-substituted phenyl, p is zero and q is 1.

A is preferably -O-.

R³ is preferably -COOR⁶, -CONR⁶R⁷, -COR⁶, -SO₂NR⁶R⁷, S(O)₀₋₂-alkyl, S(O)₀₋₂-aryl, NO₂ or halogeno. A more preferred definition for R³ is halogeno, especially fluoro or chloro.

R⁴ is preferably hydrogen, lower alkyl, -OR⁶, -O(CO)R⁶, -O(CO)OR⁹, -O(CO)NR⁶R⁷, -NR⁶R⁷, COR⁶ or halogeno, wherein R⁶ and R⁷ are preferably independently hydrogen or lower alkyl, and R⁹ is preferably lower alkyl. A more preferred definition for R⁴ is hydrogen or halogeno, especially fluoro or chloro.

R⁵ is preferably -OR⁶, -O(CO)R⁶, -O(CO)OR⁹, -O(CO)NR⁶R⁷, -NR⁶R⁷, -(lower alkylene)-COOR⁶ or -CH=CH-COOR⁶, wherein R⁶ and R⁷ are preferably independently hydrogen or lower alkyl, and R⁹ is preferably lower alkyl. A more preferred definition for R⁵ is -OR⁶, -(lower alkylene)-COOR⁶ or -CH=CH-COOR⁶, wherein R⁶ is preferably hydrogen or lower alkyl.

Methods for making compounds of Formula III are well known to those skilled in the art. Non-limiting examples of suitable methods are disclosed in U.S. Patent No. 5,688,990, which is incorporated herein by reference.

In another embodiment, sterol absorption inhibitors useful in the compositions, therapeutic combinations and methods of the present invention are represented by Formula (IV): 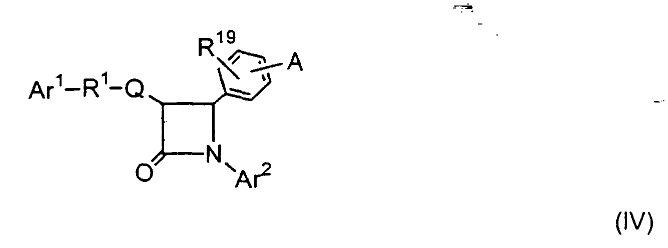 or isomers of the compounds of Formula (IV), or pharmaceutically acceptable salts or solvates of the compounds of Formula (IV) or of the isomers of the compounds of Formula (IV), or prodrugs of the compounds of Formula (IV) or of the isomers, salts or solvates of the compounds of Formula (IV), wherein, in Formula (IV) above:
A is selected from the group consisting of R²-substituted heterocycloalkyl, R²-substituted heteroaryl, R²-substituted benzofused heterocycloalkyl, and R²-substituted benzofused heteroaryl;
Ar¹ is aryl or R³-substituted aryl;
Ar² is aryl or R⁴-substituted aryl;
Q is a bond or, with the 3-position ring carbon of the azetidinone, forms the spiro group 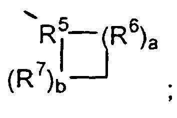 and
R¹ is selected from the group consisting of:
   - (CH₂)_{q}-, wherein q is 2-6, provided that when Q forms a spiro ring, q can also be zero or 1;
   - (CH₂)ₑ-G-(CH₂)ᵣ-, wherein G is -O-, -C(O)-, phenylene, -NR⁸- or
   - S(O)₀₋₂-, e is 0-5 and r is 0-5, provided that the sum of e and r is 1-6;
   - (C₂-C₆ alkenylene)-; and
   - (CH₂)_{f}-V-(CH₂)_{g}-, wherein V is C₃-C₆ cycloalkylene, f is 1-5 and g is 0-5, provided that the sum of f and g is 1-6;
R⁵ is selected from: 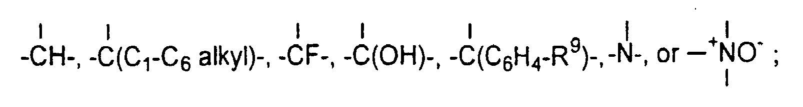
R⁶ and R⁷ are independently selected from the group consisting of -CH₂-, -CH(C₁-C₆ alkyl)-, -C(di-(C₁-C₆) alkyl), -CH=CH- and -C(C₁-C₆ alkyl)=CH-; or R⁵ together with an adjacent R⁶, or R⁵ together with an adjacent R⁷, form a -CH=CH- or a -CH=C(C₁-C₆ alkyl)- group;
a and b are independently 0, 1, 2 or 3, provided both are not zero; provided that when R⁶ is -CH=CH- or -C(C₁-C₆ alkyl)=CH-, a is 1; provided that when R⁷ is -CH=CH- or -C(C₁-C₆ alkyl)=CH-, b is 1; provided that when a is 2 or 3, the R⁶'s can be the same or different; and provided that when b is 2 or 3, the R⁷'s can be the same or different;
and when Q is a bond, R¹ also can be selected from: 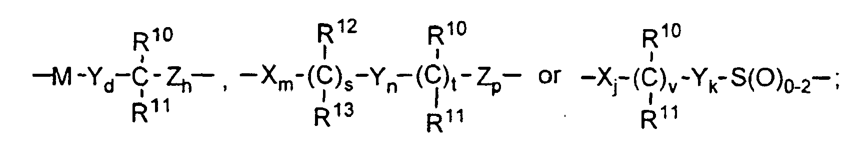 where M is -O-, -S-, -S(O)- or -S(O)₂-;
X, Y and Z are independently selected from the group consisting of -CH₂-, -CH(C₁-C₆ alkyl)- and -C(di-(C₁-C₆) alkyl);
R¹⁰ and R¹² are independently selected from the group consisting of -OR¹⁴, -O(CO)¹⁴, -O(CO)OR¹⁶ and -O(CO)NR ¹⁴R¹⁵;
R¹¹ and R¹³ are independently selected from the group consisting of hydrogen, (C₁-C₆)alkyl and aryl; or R¹⁰ and R¹¹ together are =O, or R¹² and R¹³ together are =O;
d is 1, 2 or 3;
h is 0, 1, 2, 3 or 4;
s is 0 or 1: t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
R² is 1-3 substituents on the ring carbon atoms selected from the group consisting of hydrogen, (C₁-C₁₀)alkyl, (C₂-C₁₀)alkenyl, (C₂-C₁₀)alkynyl, (C₃-C₆)cycloalkyl, (C₃-C₆)cycloalkenyl, R¹⁷-substituted aryl, R¹⁷-substituted benzyl, R¹⁷-substituted benzyloxy, R¹⁷-substituted aryloxy, halogeno, -NR¹⁴R¹⁵, NR¹⁴R¹⁵(C₁-C₆ alkylene)-, NR¹⁴R¹⁵C(O)(C₁-C₆ alkylene)-,-NHC(O)R¹⁶, OH, C₁-C₆ alkoxy, - OC(O)R¹⁶,
-COR¹⁴, hydroxy(C₁-C₆)alkyl, (C₁-C₆)alkoxy(C₁-C₆)alkyl, NO₂, -S(O)₀₋₂R¹⁶, - SO₂NR¹⁴-R¹⁵ and -(C₁-C₆ alkylene)COOR¹⁴; when R² is a substituent on a heterocycloalkyl ring, R² is as defined, or is =O or 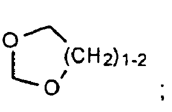 and, where R is a substituent on a substitutable ring nitrogen, it is hydrogen, (C₁-C₆)alkyl, aryl, (C₁-C₆)alkoxy, aryloxy, (C₁-C₆)alkylcarbonyl, arylcarbonyl, hydroxy, -(CH₂)₁₋₆CONR¹⁸R¹⁸, 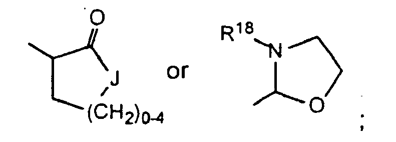 wherein J is -O-, -NH-, -NR¹⁸- or -CH₂- ;
R³ and R⁴ are independently selected from the group consisting of 1-3 substituents independently selected from the group consisting of (C₁-C₆)alkyl, -OR¹⁴, -O(CO)R¹⁴, -O(CO)OR¹⁶, -O(CH₂)₁₋₅OR¹⁴, -O(CO)NR¹⁴R¹⁵, NR¹⁴R¹⁵, -NR¹⁴(CO)R¹⁵ , -NR¹⁴(CO)OR¹⁶ , -NR¹⁴(CO)NR¹⁵R¹⁹ , -NR¹⁴SO₂R¹⁶, -COOR¹⁴ , -CONR¹⁴R¹⁵, -COR¹⁴, -SO₂NR¹⁴R¹⁵, S(O)₀₋₂R¹⁶, -O(CH₂)₁₋₁₀-COOR¹⁴, -O(CH₂)₁₋₁₀CONR¹⁴R¹⁵, -(C₁-C₆ alkylene)-COOR¹⁴, -CH=CH-COOR¹⁴, -CF₃, -CN, - NO₂ and halogen;
R⁸ is hydrogen, (C₁-C₆)alkyl, aryl (C₁-C₆)alkyl, -C(O)R¹⁴ or -COOR¹⁴;
R⁹ and R¹⁷ are independently 1-3 groups independently selected from the group consisting of hydrogen, (C₁-C₆)alkyl, (C₁-C₆)alkoxy, -COOH, NO₂, -NR¹⁴R¹⁵, OH and halogeno;
R¹⁴ and R¹⁵ are independently selected from the group consisting of hydrogen, (C₁-C₆)alkyl, aryl and aryl-substituted (C₁-C₆)alkyl;
R¹⁶ is (C₁-C₆)alkyl, aryl or R¹⁷-substituted aryl;
R¹⁸ is hydrogen or (C₁-C₆)alkyl; and
R¹⁹ is hydrogen, hydroxy or (C₁-C₆)alkoxy.

As used in Formula (IV) above, "A" is preferably an R²-substituted, 6-membered heterocycloalkyl ring containing 1 or 2 nitrogen atoms. Preferred heterocycloalkyl rings are piperidinyl, piperazinyl and morpholinyl groups. The ring "A" is preferably joined to the phenyl ring through a ring nitrogen. Preferred R² substituents are hydrogen and lower alkyl. R¹⁹ is preferably hydrogen.

Ar² is preferably phenyl or R⁴-phenyl, especially (4-R⁴)-substituted phenyl. Preferred definitions of R⁴ are lower alkoxy, especially methoxy, and halogeno, especially fluoro.

Ar¹ is preferably phenyl or R³-substituted phenyl, especially (4-R³)-substituted phenyl.

There are several preferred definitions for the -R¹-Q- combination of variables:
Q is a bond and R¹ is lower alkylene, preferably propylene;
Q is a spiro group as defined above, wherein preferably R⁶ and R⁷ are each ethylene and R⁵ is 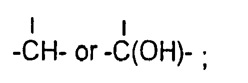
Q is a bond and R¹ is 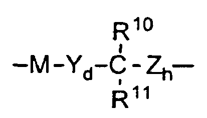 wherein the variables are chosen such that R¹ is -O-CH₂-CH(OH)-;
Q is a bond and R¹ is 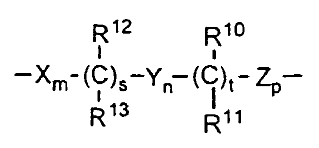 wherein the variables are chosen such that R¹is -CH(OH)-(CH₂)₂-; and
Q is a bond and R¹ is 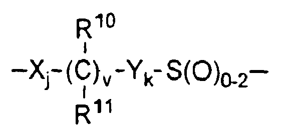 wherein the variables are chosen such that R¹ is -CH(OH)-CH₂-S(O)₀₋₂-,

Methods for making compounds of Formula IV are well known to those skilled in the art. Non-limiting examples of suitable methods are disclosed in U.S. Patent No. 5,656,624, which is incorporated herein by reference.

In another embodiment, sterol absorption inhibitors useful in the compositions, therapeutic combinations and methods of the present invention are represented by Formula (V): 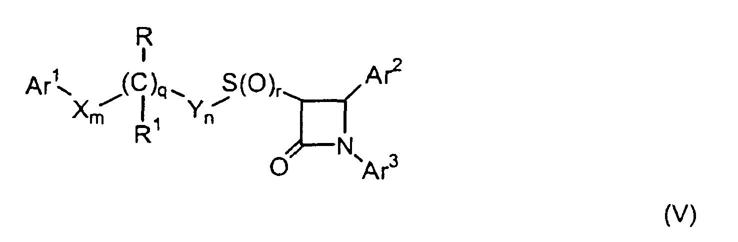 or isomers of the compounds of Formula (V), or pharmaceutically acceptable salts or solvates of the compounds of Formula (V) or of the isomers of the compounds of Formula (V), or prodrugs of the compounds of Formula (V) or of the isomers, salts or solvates of the compounds of Formula (V), wherein, in Formula (V) above:
Ar¹ is aryl, R¹⁰-substituted aryl or heteroaryl;
Ar² is aryl or R⁴-substituted aryl;
Ar³ is aryl or R⁵-substituted aryl;
X and Y are independently selected from the group consisting of -CH₂-, -CH(lower alkyl)- and -C(dilower alkyl)-;
R is -OR⁶, -O(CO)R⁶, -O(CO)OR⁹ or -O(CO)NR⁶R⁷; R¹ is hydrogen, lower alkyl or aryl; or R and R¹ together are =O;
q is 0 or 1 ;
r is 0, 1 or 2;
m and n are independently 0, 1, 2, 3, 4 or 5; provided that the sum of m, n and q is 1, 2, 3, 4 or 5;
R⁴ is 1-5 substituents independently selected from the group consisting of lower alkyl, -OR⁶, -O(CO)R⁶, -O(CO)OR⁹, -O(CH₂)₁₋₅OR⁶, -O(CO)NR⁶R⁷, -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶, -SO₂NR⁶R⁷, S(O)₀₋₂R⁹, O(CH₂)₁₋₁₀-COOR⁶, -O(CH₂)₁₋₁₀CONR⁶R⁷, -(lower alkylene)COOR⁶ and -CH=CH-COOR⁶;
R⁵ is 1-5 substituents independently selected from the group consisting of -OR⁶, -O(CO)R⁶, -O(CO)OR⁹, -O(CH₂)₁₋₅OR⁶, -O(CO)NR⁶R⁷, -NR⁶R⁷, NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶,-SO₂NR⁶R⁷, S(O)₀₋₂R⁹, -O(CH₂)₁₋₁₀-COOR⁶,-O(CH₂)₁₋₁₀CONR R , -CF₃, -CN, -NO₂, halogen,
   -(lower alkylene)COOR⁶ and -CH=CH-COOR⁶;
R⁶, R⁷ and R⁸ are independently selected from the group consisting of hydrogen, lower alkyl, aryl and aryl-substituted lower alkyl;
R⁹ is lower alkyl, aryl or aryl-substituted lower alkyl; and
R¹⁰ is 1-5 substituents independently selected from the group consisting of lower alkyl, -OR⁶, -O(CO)R⁶, -O(CO)OR⁹, -O(CH₂)₁₋₅OR⁶, -O(CO)NR⁶R⁷, -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶, -SO₂NR⁶R⁷, -S(O)₀₋₂R⁹, -O(CH₂)₁₋₁₀-COOR⁶, -O(CH₂)₁₋₁₀CONR⁶R⁷,
   -CF₃, -CN, -NO₂ and halogen.

Within the scope of Formula V, there are included two preferred structures. In Formula VA, q is zero and the remaining variables are as defined above, and in Formula VB, q is 1 and the remaining variables are as defined above:

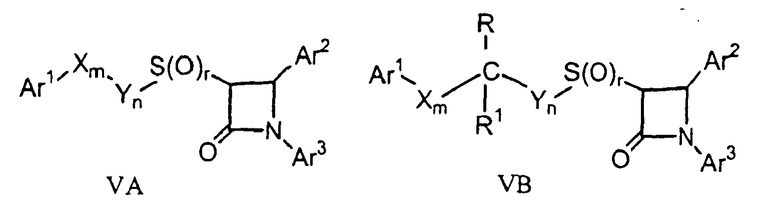

R⁴, R⁵ and R¹⁰ are each preferably 1-3 independently selected substituents as set forth above. Preferred are compounds of Formula (V) wherein Ar¹ is phenyl, R¹⁰-substituted phenyl or thienyl, especially (4-R¹⁰)-substituted phenyl or thienyl. Ar² is preferably R⁴-substituted phenyl, especially (4-R⁴)-substituted phenyl. Ar³ is preferably phenyl or R⁵-substituted phenyl, especially (4-R⁵)-substituted phenyl. When Ar¹ is R¹⁰-substituted phenyl, R¹⁰ is preferably halogeno, especially fluoro. When Ar² is R⁴-substituted phenyl, R⁴ is preferably -OR⁶, especially wherein R⁶ is hydrogen or lower alkyl. When Ar³ is R⁵-substituted phenyl, R⁵ is preferably halogeno, especially fluoro. Especially preferred are compounds of Formula (V) wherein Ar¹ is phenyl, 4-fluorophenyl or thienyl, Ar² is 4-(alkoxy or hydroxy)phenyl, and Ar³ is phenyl or 4-fluorophenyl.

X and Y are each preferably -CH₂-. The sum of m, n and q is preferably 2, 3 or 4, more preferably 2. When q is 1, n is preferably 1 to 5.

Preferences for X, Y, Ar¹, Ar² and Ar³ are the same in each of Formulae (VA) and (VB).

In compounds of Formula (VA), the sum of m and n is preferably 2, 3 or 4, more preferably 2. Also preferred are compounds wherein the sum of m and n is 2, and r is 0 or 1.

In compounds of Formula (VB), the sum of m and n is preferably 1, 2 or 3, more preferably 1. Especially preferred are compounds wherein m is zero and n is 1. R¹ is preferably hydrogen and R is preferably -OR⁶ wherein R⁶ is hydrogen, or a group readily metabolizable to a hydroxyl (such as -O(CO)R⁶, -O(CO)OR⁹ and -O(CO)NR⁶R⁷, defined above), or R and R¹ together form a =O group.

Methods for making compounds of Formula V are well known to those skilled in the art. Non-limiting examples of suitable methods are disclosed in U.S. Patent No. 5,624,920, which is incorporated herein by reference.

In another embodiment, sterol absorption inhibitors useful in the compositions, therapeutic combinations and methods of the present invention are represented by Formula (VI): 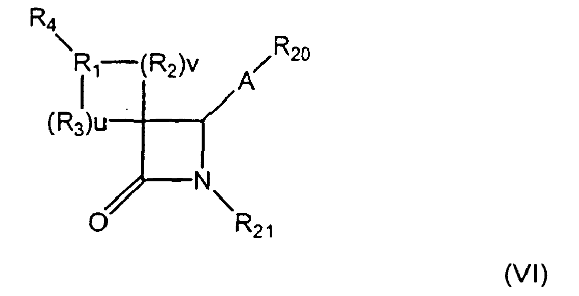 or isomers of the compounds of Formula (VI), or pharmaceutically acceptable salts or solvates of the compounds of Formula (VI) or of the isomers of the compounds of Formula (VI), or prodrugs of the compounds of Formula (VI) or of the isomers, salts or solvates of the compounds of Formula (VI), wherein:
R₁ is 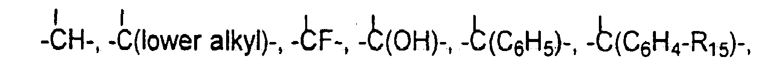 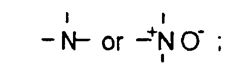
R₂ and R₃ are independently selected from the group consisting of: -CH₂-, -CH(lower alkyl)-, -C(di-lower alkyl)-, -CH=CH- and -C(lower alkyl)=CH-; or R₁ together with an adjacent R₂, or R₁ together with an adjacent R₃, form a -CH=CH- or a -CH=C(lower alkyl)- group;
u and v are independently 0, 1, 2 or 3, provided both are not zero; provided that when R₂ is -CH=CH- or -C(lower alkyl)=CH-, v is 1; provided that when R₃ is - CH=CH- or -C(lower alkyl)=CH-, u is 1; provided that when v is 2 or 3, the R₂'s can be the same or different; and provided that when u is 2 or 3, the R₃'s can be the same or different;
R₄ is selected from B-(CH₂)ₘC(O)-, wherein m is 0, 1, 2, 3, 4 or 5; B-(CH₂)_{q}-, wherein q is 0, 1, 2, 3, 4, 5 or 6; B-(CH₂)ₑ-Z-(CH₂)ᵣ-, wherein Z is -O-, -C(O)-, phenylene, -N(R₈)- or -S(O)₀₋₂-, e is 0, 1, 2, 3, 4 or 5 and r is 0, 1, 2, 3, 4 or 5, provided that the sum of e and r is 0, 1, 2, 3, 4, 5 or 6; B-(C₂-C₆ alkenylene)-; B-(C₄-C₆ alkadienylene)-; B-(CH₂)ₜ-Z-(C₂-C₆ alkenylene)-, wherein Z is as defined above, and wherein t is 0, 1, 2 or 3, provided that the sum of t and the number of carbon atoms in the alkenylene chain is 2, 3, 4, 5 or 6; B-(CH₂)_{f}-V-(CH₂)_{g}-, wherein V is C₃-C₆ cycloalkylene, f is 1, 2, 3, 4 or 5 and g is 0, 1, 2, 3, 4 or 5, provided that the sum of f and g is 1, 2, 3, 4, 5 or 6; B-(CH₂)ₜ-V-(C₂-C₆ alkenylene)- or B-(C₂-C₆ alkenylene)-V-(CH₂)ₜ-, wherein V and t are as defined above, provided that the sum of t and the number df carbon atoms in the alkenylene chain is 2, 3, 4, 5 or 6; B-(CH₂)ₐ-Z-(CH₂)_{b}-V-(CH₂)_{d}-, wherein Z and V are as defined above and a, b and d are independently 0, 1, 2, 3, 4, 5 or 6, provided that the sum of a, b and d is 0, 1, 2, 3, 4, 5 or 6; or T-(CH₂)ₛ-, wherein T is cycloalkyl of 3-6 carbon atoms and s is 0, 1, 2, 3, 4, 5 or 6; or
R₁ and R₄ together form the group 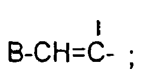
B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or 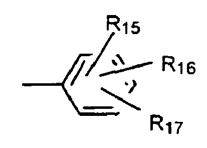
W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, -CF₃, -OCF₃, benzyl, R₇-benzyl, benzyloxy, R₇-benzyloxy, phenoxy, R₇-phenoxy, dioxolanyl, NO₂, -N(R₈)(R₉), N(R₈)(R₉)-lower alkylene-, N(R₈)(R₉)-lower alkylenyloxy-, OH, halogeno, -CN, -N₃, -NHC(O)OR₁₀, -NHC(O)R¹⁰, R¹¹O₂SNH-, (R₁₁O₂S)₂N-, -S(O)₂NH₂, -S(O)₀₋₂R₈, tert-butyldimethyl-silyloxymethyl, -C(O)R₁₂, -COOR₁₉, -CON(R₈)(R₉), -CH=CHC(O)R₁₂, -lower alkylene-C(O)R₁₂, R₁₀C(O)(tower alkylenyloxy)-, N(R₈)(R₉)C(O)(lower alkylenyloxy)- and 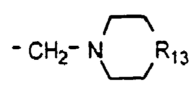 for substitution on ring carbon atoms, and the substituents on the substituted heteroaryl ring nitrogen atoms, when present, are selected from the group consisting of lower alkyl, lower alkoxy, -C(O)OR₁₀, -C(O)R₁₀, OH, N(R₈)(R₉)-lower alkylene-, N(R₈)(R₉)-lower alkylenyloxy-, -S(O)₂NH₂ and 2-(trimethylsilyl)-ethoxymethyl;
R₇ is 1-3 groups independently selected from the group consisting of lower alkyl, lower alkoxy, -COOH, NO₂, -N(R₈)(R₉), OH, and halogeno;
R₈ and R₉ are independently selected from H or lower alkyl;
R₁₀ is selected from lower alkyl, phenyl, R₇-phenyl, benzyl or R₇-benzyl;
R₁₁ is selected from OH, lower alkyl, phenyl, benzyl, R₇-phenyl or R₇-benzyl;
R₁₂ is selected from H, OH, alkoxy, phenoxy, benzyloxy, 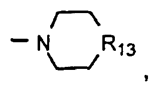 -N(R₈)(R₉), lower alkyl, phenyl or R₇-phenyl;
R₁₃ is selected from -O-, -CH₂-, -NH-, -N(lower alkyl)- or -NC(O)R₁₉;
R₁₅. R₁₆ and R₁₇ are independently selected from the group consisting of H and the groups defined for W; or R₁₅ is hydrogen and R₁₆ and R₁₇, together with adjacent carbon atoms to which they are attached, form a dioxolanyl ring;
R₁₉ is H, lower alkyl, phenyl or phenyl lower alkyl; and
R₂₀ and R₂₁ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above.

One group of preferred compounds of Formula VI is that in which R₂₁ is selected from phenyl, W-substituted phenyl, indanyl, benzofuranyl, benzodioxolyl, tetrahydronaphthyl, pyridyl, pyrazinyl, pyrimidinyl, quinolyl or cyclopropyl,
wherein W is lower alkyl, lower alkoxy, OH, halogeno, -N(R₈)(R₉), -NHC(O)OR₁₀, -NHC(O)R₁₀, NO₂, -CN, -N₃, -SH, -S(O)₀₋₂-(lower alkyl), -COOR₁₉, -CON(R₈)(R₉), -COR₁₂, phenoxy, benzyloxy, -OCF₃, -CH=C(O)R₁₂ or tert-butyldimethylsilyloxy, wherein R₈, R₉, R₁₀, R₁₂ and R₁₉ are as defined for Formula IV. When W is 2 or 3 substituents, the substituents can be the same or different.

Another group of preferred compounds of Formula VI is that in which R₂₀ is phenyl or W-substituted phenyl, wherein preferred meanings of W are as defined above for preferred definitions of R₂₁.

More preferred are compounds of Formula VI wherein R₂₀ is phenyl or W-substituted phenyl and R₂₁ is phenyl, W-substituted phenyl, indanyl, benzofuranyl, benzodioxolyl, tetrahydronaphthyl, pyridyl, pyrazinyl, pyrimidinyl, quinolyl or cyclopropyl; W is lower alkyl, lower alkoxy, OH, halogeno, -N(R₈)(R₉), -NHC(O)OR₁₀, -NHC(O)R₁₀, NO₂, -CN, -N₃, -SH, -S(O)₀₋₂-(lower alkyl), -COOR₁₉, -CON(R₈)(R₉), -COR₁₂, phenoxy, benzyloxy, -CH=CHC(O)R₁₂, -OCF₃ or tert-butyldimethyl-silyloxy, wherein when W is 2 or 3 substituents, the substituents can be the same or different, and wherein R₈, R₉, R₁₀, R₁₂ and R₁₉ are as defined in Formula VI.

Also preferred are compounds of Formula VI wherein R₁ is

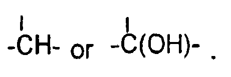

Another group of preferred compounds of Formula VI is in which R₂ and R₃ are each -CH₂- and the sum of u and v is 2, 3 or 4, with u=v=2 being more preferred.

R₄ is preferably B-(CH₂)_{q}- or B-(CH₂)ₑ-Z-(CH₂)ᵣ-, wherein B, Z, q, e and r are as defined above. B is preferably 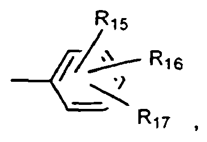 wherein R₁₆ and R₁₇ are each hydrogen and wherein R₁₅ is preferably H, OH, lower alkoxy, especially methoxy, or halogeno, especially chloro.

Preferably Z is -O-, e is 0, and r is 0.

Preferably q is 0-2.

R₂₀ is preferably phenyl or W-substituted phenyl.

Preferred W substituents for R₂₀ are lower alkoxy, especially methoxy and ethoxy, OH, and -C(O)R₁₂, wherein R₁₂ is preferably lower alkoxy.

Preferably R₂₁ is selected from phenyl, lower alkoxy-substituted phenyl and F-phenyl.

Especially preferred are compounds of Formula VI wherein R₁ is 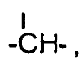 or

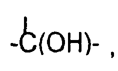

R₂ and R₃ are each -CH₂-, u=v=2, R₄ is B-(CH₂)q-, wherein B is phenyl or phenyl substituted by lower alkoxy or chloro, q is 0-2, R₂₀ is phenyl, OH-phenyl, lower alkoxy-substituted phenyl or lower alkoxycarbonyl-substituted phenyl, and R₂₁ is phenyl, lower alkoxy-substituted phenyl or
F-phenyl.

Methods for making compounds of Formula VI are well known to those skilled in the art. Non-limiting examples of suitable methods are disclosed in U.S. Patent No. 5,698,548, which is incorporated herein by reference.

In another embodiment, sterol inhibitors useful in the compositions, therapeutic combinations and methods of the present invention are represented by Formulas (VIIA) and (VIIB): 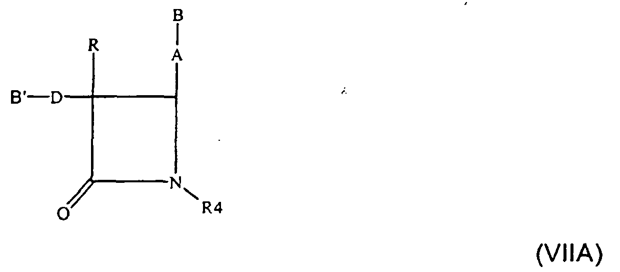 and 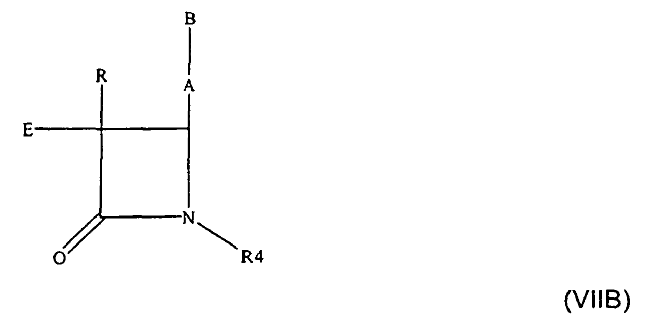 or isomers of the compounds of Formulae (VIIA) or (VIIB), or pharmaceutically acceptable salts or solvates of the compounds of Formulae (VIIA) or (VIIB) or of the isomers of the compounds of Formulae (VIIA) or (VIIB), or prodrugs of the compounds of Formulae (VIIA) or (VIIB) or of the isomers, salts or solvates of the compounds of Formulae (VIIA) or (VIIB), wherein, in Formulae (VIIA) or (VIIB) above:
A is -CH=CH-, -C≡C- or -(CH₂)ₚ- wherein p is 0, 1 or 2;
B is 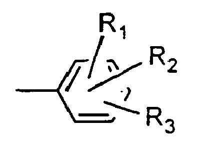
B' is 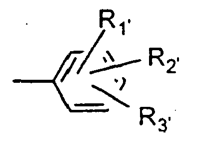
D is -(CH₂)ₘC(O)- or -(CH₂)_{q}- wherein m is 1, 2, 3 or 4 and q is 2, 3 or 4;
E is C₁₀ to C₂₀ alkyl or -C(O)-(C₉ to C₁₉)-alkyl, wherein the alkyl is straight or branched, saturated or containing one or more double bonds;
R is hydrogen, C₁-C₁₅ alkyl, straight or branched, saturated or containing one or more double bonds, or B-(CH₂)ᵣ -, wherein r is 0, 1, 2, or 3;
R₁, R₂, R₃, R_{1'}, R_{2'}, and R_{3'} are independently selected from the group consisting of hydrogen, lower alkyl, lower alkoxy, carboxy, NO₂, NH₂, OH, halogeno, lower alkylamino, dilower alkylamino, -NHC(O)OR₅, R₆O₂SNH- and -S(O)₂NH₂;
R₄ is 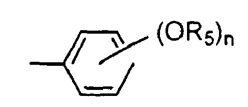 wherein n is 0, 1, 2 or 3;
R₅ is lower alkyl; and
R₆ is OH, lower alkyl, phenyl, benzyl or substituted phenyl wherein the substituents are 1-3 groups independently selected from the group consisting of lower-alkyl, lower alkoxy, carboxy, NO₂, NH₂, OH, halogeno, lower alkylamino and dilower alkylamino.

Preferred are compounds of Formula (VIIA) wherein R is hydrogen, saturated or mono-unsaturated C₁ -C₁₀ alkyl or phenyl. Another group of preferred compounds of Formula (VIIA) is that in which D is propyl (i.e., -(CH₂)_{q}- and q is 3). A third group of preferred compounds of Formula (VIIA) is that wherein R₄ is p-methoxyphenyl or 2,4,6-trimethoxyphenyl. Still another group of preferred compounds of Formula (VIIA) is that wherein A is ethylene or a bond (i.e., -(CH₂ )p- wherein p is zero). R_{1'}, R_{2'}, and R_{3'} are preferably each hydrogen, and preferably R₁ is hydrogen, hydroxy, nitro, lower alkoxy, amino or t-butoxycarbonyl-amino and R₂ and R₃ are each hydrogen.

More preferred are compounds of Formula (VIIA) wherein R_{1'}, R_{2'}, and R_{3'} are each hydrogen; R₁ is hydrogen, hydroxy, nitro, lower alkoxy, amino or t-butoxycarbonyl-amino and R₂ and R₃ are each hydrogen; R is hydrogen, ethyl or phenyl; D is propyl; R₄ is p-methoxyphenyl or 2,4,6-trimethoxyphenyl; and A is ethylene or a bond.

Preferred compounds of Formula (VIIA), wherein B' is phenyl, are shown in the following table:

| D | R | A | B | R₄ |
|---|---|---|---|---|
| -(CH₂)₃- | H | --- | p-MeO-phenyl | p-MeO-phenyl |
| -CH₂C(O)- | phenyl | --- | phenyl | p-MeO-phenyl |
| -(CH₂)₃- | H | --- | phenyl | p-MeO-phenyl |
| -(CH₂)₃- | H | --- | p-OH-phenyl | p-MeO-phenyl |
| -(CH₂)₃- | H | ethylene | p-MeO-phenyl | p-MeO-phenyl |
| -(CH₂)₃- | H | --- | 3-MeO-phenyl | p-MeO-phenyl |
| -(CH₂)₃- | ethyl | --- | phenyl | p-MeO-phenyl |
| -(CH₂)₃- | phenyl | --- | phenyl | p-MeO-phenyl |
| -(CH₂)₃- | ethyl | --- | phenyl | 2,4,6-tri-MeO-phenyl |
| -(CH₂)₃- | methyl | --- | phenyl | p-MeO-phenyl |
| -(CH₂)₃- | H | --- | p-NH₂- phenyl | p-MeO-phenyl |

The first-listed compound in the above table having the (3R,4S) absolute stereochemistry is more preferred.

Preferred compounds of Formula (VIIB) are those wherein R is hydrogen, methyl, ethyl, phenyl or phenylpropyl. Another group of preferred compounds of Formula (VIIB) is that wherein R₄ is p-methoxyphenyl or 2,4,6-trimethoxyphenyl. Still another group of preferred compounds of Formula (VIIB) is that wherein A is ethylene or a bond. Yet another group of preferred compounds of Formula (VIIB) is that wherein E is decyl, oleoyl or 7-Z-hexadecenyl. Preferably R₁, R₂ and R₃ are each hydrogen.

More preferred compounds of Formula (VIIB) are those wherein R is hydrogen, methyl, ethyl, phenyl or phenylpropyl; R₄ is p-methoxyphenyl or 2,4,6-trimethoxyphenyl; A is ethylene or a bond; E is decyl, oleoyl or 7-Z-hexadecenyl; and R₁, R₂ and R₃ are each hydrogen.

A preferred compound of Formula (VIIB) is that wherein E is decyl, R is hydrogen, B-A is phenyl and R₄ is p-methoxyphenyl.

In another embodiment, sterol inhibitors useful in the compositions, therapeutic combinations and methods of the present invention are represented by Formula (VIII): 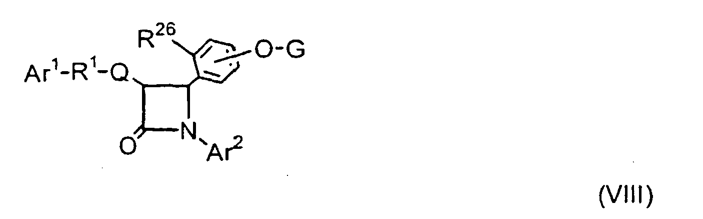
or isomers of the compounds of Formula (VIII), or pharmaceutically acceptable salts or solvates of the compounds of Formula (VIII) or of the isomers of the compounds of Formula (VIII), or prodrugs of the compounds of Formula (VIII) or of the isomers, salts or solvates of the compounds of Formula (VIII), wherein, in Formula (VIII) above,
R²⁶ is H or OG¹;
G and G¹ are independently selected from the group consisting of 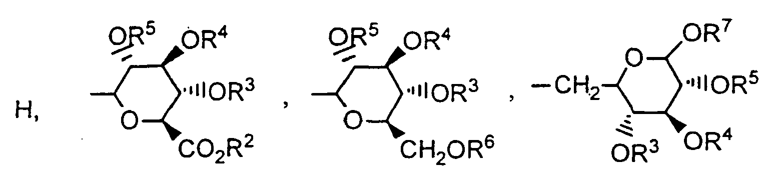 and 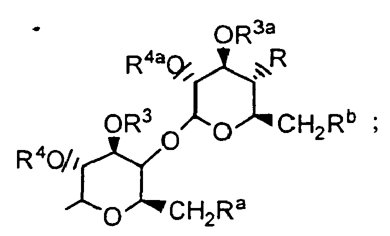 provided that when R²⁶ is H or OH, G is not H;
R, R^{a} and R^{b} are independently selected from the group consisting of H, -OH, halogeno, -NH₂, azido, (C₁-C₆)alkoxy(C₁-C₆)-alkoxy or -W-R³⁰;
W is independently selected from the group consisting of -NH-C(O)-, -O-C(O)-, -O-C(O)-N(R³¹)-, -NH-C(O)-N(R³¹)- and -O-C(S)-N(R³¹)-;
R2 and R⁶ are independently selected from the group consisting of H, (C₁-C₆)alkyl, aryl and aryl(C₁-C₆)alkyl;
R³, R⁴, R⁵, R⁷, R^{3a} and R^{4a} are independently selected from the group consisting of H, (C₁-C₆)alkyl, aryl(C₁-C₆)alkyl, -C(O)(C₁-C₆)alkyl and -C(O)aryl;
R³⁰ is selected from the group consisting of R³²-substituted T, R³²-substituted-T-(C₁-C₆)alkyl, R³²-substituted-(C₂-C₄)alkenyl, R³²-substituted-(C₁-C₆)alkyl, R³²-substituted-(C₃-C₇)cycloalkyl and R³²-substituted-(C₃-C₇)cycloalkyl(C₁-C₆)alkyl;
R³¹ is selected from the group consisting of H and (C₁-C₄)alkyl;
T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;
R³² is independently selected from 1-3 substituents independently selected from the group consisting of halogeno, (C₁-C₄)alkyl, -OH, phenoxy, -CF₃, -NO₂, (C₁-C₄)alkoxy, methylenedioxy, oxo, (C₁-C₄)alkylsulfanyl, (C₁-C₄)alkylsulfinyl, (C₁-C₄)alkylsulfonyl, -N(CH₃)₂, -C(O)-NH(C₁-C₄)alkyl, -C(O)-N((C₁-C₄)alkyl)₂, -C(O)-(C₁-C₄)alkyl, -C(O)-(C₁-C₄)alkoxy and pyrrolidiriylcarbonyl; or R³² is a covalent bond and R³¹, the nitrogen to which it is attached and R³² form a pyrrolidinyl, piperidinyl, N-methyl-piperazinyl, indolinyl or morpholinyl group, or a (C₁-C₄)alkoxycarbonyl-substituted pyrrolidinyl, piperidinyl, N-methylpiperazinyl, indolinyl or morpholinyl group;
Ar¹ is aryl or R¹⁰-substituted aryl;
Ar² is aryl or R¹¹-substituted aryl;
Q is a bond or, with the 3-position ring carbon of the azetidinone, forms the spiro group 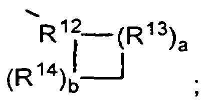 and
R¹ is selected from the group consisting of
   -(CH₂)_{q}-, wherein q is 2-6, provided that when Q forms a spiro ring, q can also be zero or 1;
   -(CH₂)ₑ-E-(CH₂)ᵣ, wherein E is -O-, -C(O)-, phenylene, -NR²²- or -S(O)₀₋₂-, e is 0-5 and r is 0-5, provided that the sum of e and r is 1-6;
   -(C₂-C₆)alkenylene-; and
   -(CH₂)_{f}-V-(CH₂)g-, wherein V is C₃-C₆ cycloalkylene, f is 1-5 and g is 0-5, provided that the sum of f and g is 1-6;
R¹² is 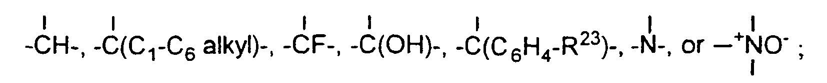
R¹³ and R¹⁴ are independently selected from the group consisting of
   -CH₂-, -CH(C₁-C₆ alkyl)-, -C(di-(C₁-C₆) alkyl), -CH=CH- and -C(C₁-C₆ alkyl)=CH-; or R¹² together with an adjacent R¹³, or R¹² together with an-adjacent R¹⁴, form a -CH=CH- or a -CH=C(C₁-C₆ alkyl)- group;
a and b are independently 0, 1, 2 or 3, provided both are not zero;
provided that when R¹³ is -CH=CH- or -C(C₁-C₆ alkyl)=CH-, a is 1;
provided that when R¹⁴ is -CH=CH- or -C(C₁-C₆ alkyl)=CH-, b is 1;
provided that when a is 2 or 3, the R¹³'s can be the same or different; and
provided that when b is 2 or 3, the R¹⁴'s can be the same or different; and when Q is a bond, R¹ also can be: 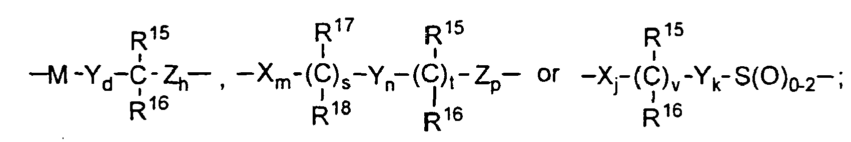
M is -O-, -S-, -S(O)- or -S(O)₂-;
X, Y and Z are independently selected from the group consisting of -CH₂-, -CH(C₁-C₆)alkyl- and -C(di-(C₁-C₆)alkyl);
R¹⁰ and R¹¹ are independently selected from the group consisting of 1-3 substituents independently selected from the group consisting of (C₁-C₆)alkyl, -OR¹⁹, -O(CO)R¹⁹, -O(CO)OR²¹, -O(CH₂)₁₋₅OR¹⁹, -O(C_{O})NR¹⁹R²⁰, -NR¹⁹R²⁰, -NR¹⁹(CO)R²⁰, -NR¹⁹(CO)OR²¹, -NR¹⁹(CO)NR²⁰R²⁵, -NR¹⁹SO₂R²¹, -COOR¹⁹, -CONR¹⁹R²⁰, -COR¹⁹, -SO₂NR¹⁹R²⁰, S(O)₀₋₂R²¹, -O(CH₂)₁₋₁₀-COOR¹⁹, -O(CH₂)₁₋₁₀CONR¹⁹R²⁰, -(C₁-C₆ alkylene)-COOR¹⁹, -CH=CH-COOR¹⁹, -CF₃, -CN, -NO₂ and halogen;
R¹⁵ and R¹⁷ are independently selected from the group consisting of -OR¹⁹, -O(CO)R¹⁹, -O(CO)OR21 and -O(CO)NR¹⁹R²⁰;
R¹⁶ and R¹⁸ are independently selected from the group consisting of H, (C₁-C₆)alkyl and aryl; or R¹⁵ and R¹⁶ together are =O, or R¹⁷ and R¹⁸ together are =O;
d is 1, 2 or 3;
h is 0, 1, 2, 3 or 4;
s is 0 or 1; t is 0 or 1; m, n and p are independently 0-4;
provided that at least one of s and t is 1, and the sum of m, n, p, s and t is 1-6;
provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
and when Q is a bond and R¹ is 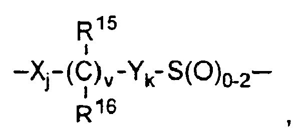 Ar¹ can also be pyridyl, isoxazolyl, furanyl, pyrrolyl, thienyl, imidazolyl, pyrazolyl, thiazolyl, pyrazinyl, pyrimidinyl or pyridazinyl;
R¹⁹ and R²⁰ are independently selected from the group consisting of H, (C₁-C₆)alkyl, aryl and aryl-substituted (C₁-C₆)alkyl;
R²¹ is (C₁-C₆)alkyl, aryl or R²⁴-substituted aryl;
R²² is H, (C₁-C₆)alkyl, aryl (C₁-C₆)alkyl, -C(O)R¹⁹ or -COOR¹⁹;
R²³ and R²⁴ are independently 1-3 groups independently selected from the group consisting of H, (C₁-C₆)alkyl, (C₁-C₆)alkoxy, -COOH, NO₂,
   -NR¹⁹R²⁰, -OH and halogeno; and
R²⁵ is H, -OH or (C₁-C₆)alkoxy.

Ar² is preferably phenyl or R¹¹ -phenyl, especially (4-R¹¹) -substituted phenyl. Preferred definitions of R¹¹ are lower alkoxy, especially methoxy, and halogeno, especially fluoro.

Ar¹ is preferably phenyl or R¹⁰-substituted phenyl, especially (4-R¹⁰)-substituted phenyl. Preferably R¹⁰ is halogeno, and more preferably fluoro.

There are several preferred definitions for the -R¹-Q- combination of variables:
Q is a bond and R¹ is lower alkylene, preferably propylene;
Q is a spiro group as defined above, wherein preferably R¹³ and R¹⁴ are each ethylene and R¹² is 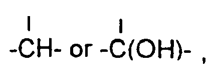 and R¹ is -(CH₂)_{q} wherein q is 0-6;
Q is a bond and R¹ is 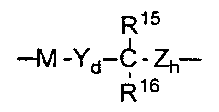 wherein the variables are chosen such that R¹ is -O-CH₂-CH(OH)-;
Q is a bond and R¹ is 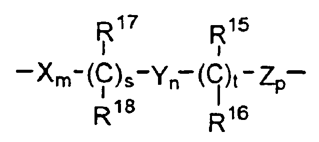 wherein the variables are chosen such that R¹ is -CH(OH)-(CH₂)₂-; and
Q is a bond and R¹ is 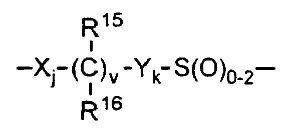 wherein the variables are chosen such that R¹ is -CH(OH)-CH₂-S(O)₀₋₂-.

A preferred compound of Formula (VIII) therefore, is one wherein G and G¹ are as defined above and in which the remaining variables have the following definitions:
Ar¹ is phenyl or R¹⁰-substituted phenyl, wherein R¹⁰ is halogeno;
Ar² is phenyl or R¹¹-phenyl, wherein R¹¹ is 1 to 3 substituents independently selected from the group consisting of C₁-C₆ alkoxy and halogeno;
Q is a bond and R¹ is lower alkylene; Q, with the 3-position ring carbon of the azetidinone, forms the group 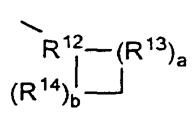
wherein preferably R¹³ and R¹⁴ are each ethylene and a and b are each 1, and wherein R¹² is 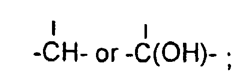 Q is a bond and R¹ is -O-CH₂-CH(OH)-: Q is a bond and R¹ is -CH(OH)-(CH₂)₂-; or Q is a bond and R¹ is -CH(OH)-CH₂-S(O)₀₋₂-.

Preferred variables for G and G¹ groups of the formulae 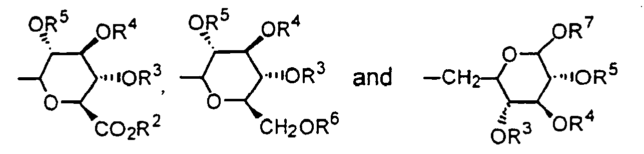 are as follows:
R², R³, R⁴, R⁵, R⁶ and R⁷ are independently selected from the group consisting of H, (C₁-C₆)alkyl, benzyl and acetyl.

Preferred variables for group G or G¹ of the formula: 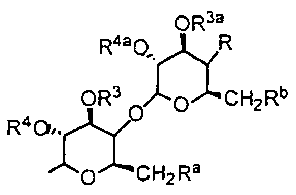 are as follows:
R³, R^{3a}, R⁴ and R^{4a} are selected from the group consisting of H, (C₁-C₆)alkyl, benzyl and acetyl;
R, R^{a} and R^{b} are independently selected from the group consisting of H, -OH, halogeno, -NH₂, azido, (C₁-C₆)alkoxy(C₁-C₆)alkoxy and -W-R³⁰,
   wherein W is -O-C(O)- or -O-C(O)-NR³¹-, R³¹ is H and
   R³⁰ is (C₁-C₆)alkyl, -C(O)-(C₁-C₄)alkoxy-(C₁-C₆)alkyl, T , T-(C₁-C₆)alkyl, or T or T-(C₁-C₆)alkyl wherein T is substituted by one or two halogeno or (C₁-C₆)alkyl groups.

Preferred R³⁰ substituents are selected from the group consisting of: 2-fluorophenyl, 2,4-difluoro-phenyl, 2,6-dichlorophenyl, 2-methylphenyl, 2-thienylmethyl, 2-methoxy-carbonylethyl, thiazol-2-yl-methyl, 2-furyl, 2-methoxycarbonylbutyl and phenyl.

Preferred combinations of R, R^{a} and R^{b} are as follows:
1) R, R^{a} and R^{b} are independently -OH or -O-C(O)-NH-R³⁰, especially wherein R^{a} is -OH and R and R^{b} are -O-C(O)-NH-R³⁰ and R³⁰ is selected from the preferred substituents identified above, or wherein R and R^{a} are each -OH and R^{b} is-O-C(O)-NH-R³⁰ wherein R³⁰ is 2-fluorophenyl, 2,4-difluoro-phenyl, 2,6-dichlorophenyl;
2) R^{a} is -OH, halogeno, azido or (C₁-C₆)-alkoxy(C₁-C₆)alkoxy, R^{b} is H, halogeno, azido or (C₁-C₆)alkoxy(C₁-C₆)-alkoxy, and R is -O-C(O)-NH-R³⁰, especially compounds wherein R^{a} is -OH, R^{b} is H and R³⁰ is 2-fluorophenyl;
3) R, R^{a} and R^{b} are independently -OH or -O-C(O)-R³⁰ and R³⁰ is (C₁-C₆)alkyl, T , or T substituted by one or two halogeno or (C₁-C₆)alkyl groups, especially compounds wherein R is -OH and R^{a} and R^{b} are -O-C(O)-R³⁰ wherein R³⁰ is 2-furyl; and
4) R, R^{a} and R^{b} are independently -OH or halogeno. Three additional classes of preferred compounds are those wherein the C¹' anomeric oxy is beta, wherein the C²' anomeric oxy is beta, and wherein the R group is alpha. G and G¹ are preferably selected from: 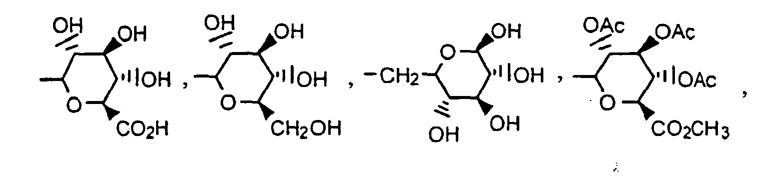 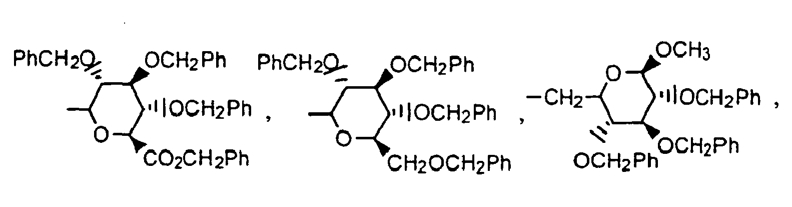 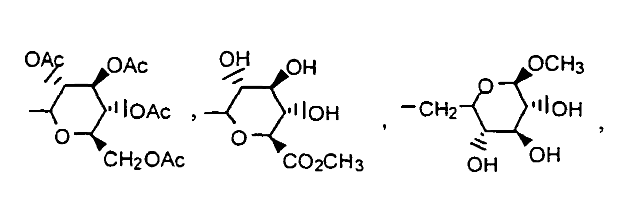 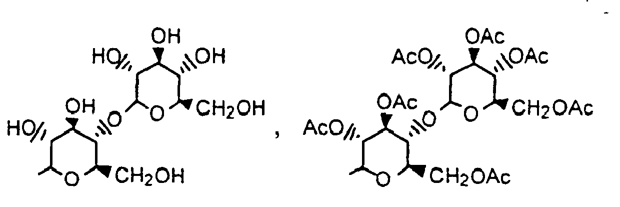 and 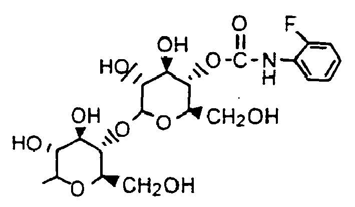
wherein Ac is acetyl and Ph is phenyl.

Preferably, R²⁶ is H or OH, more preferably H. The -O-G substituent is preferably in the 4-position of the phenyl ring to which it is attached.

In another embodiment, sterol inhibitors useful in the compositions, therapeutic combinations and methods of the present invention are represented by Formula (IX) below: 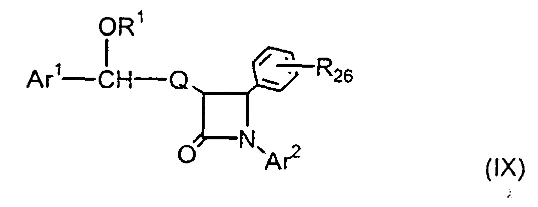 or isomers of the compounds of Formula (IX), or pharmaceutically acceptable salts or solvates of the compounds of Formula (IX) or of the isomers of the compounds of Formula (IX), or prodrugs of the compounds of Formula (IX) or of the isomers, salts or solvates of the compounds of Formula (IX), wherein in Formula (lX) above:
R²⁶ is selected from the group consisting of:
   a) OH;
   b) OCH₃;
   c) fluorine and
   d) chlorine.
R¹ is selected from the group consisting of 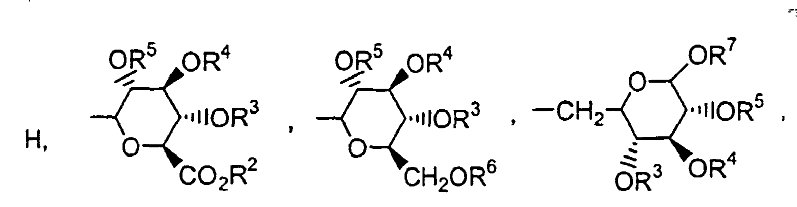 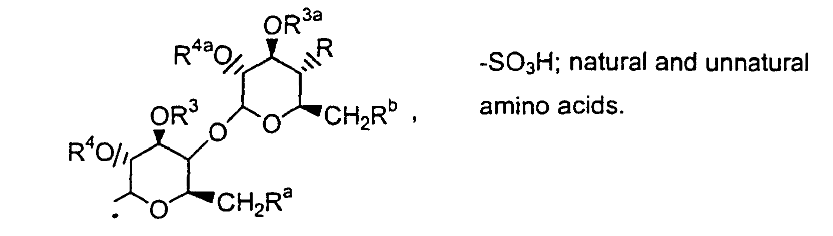
R, R^{a} and R^{b} are independently selected from the group consisting of H, -OH, halogeno, -NH₂, azido, (C₁-C₆)alkoxy(C₁-C₆)-alkoxy and -W-R³⁰;
W is independently selected from the group consisting of -NH-C(O)-, -O-C(O)-, -O-C(O)-N(R³¹)-, -NH-C(O)-N(R³¹)- and -O-C(S)-N(R³¹)-;
R2 and R⁶ are independently selected from the group consisting of H, (C₁-C₆)alkyl, aryl and aryl(C₁-C₆)alkyl;
R³, R⁴, R⁵, R⁷, R^{3a} and R^{4a} are independently selected from the group consisting of H, (C₁-C₆)alkyl, aryl(C₁-C₆)alkyl, -C(O)(C₁-C₆)alkyl and -C(O)aryl;
R³⁰ is independently selected form the group consisting of R32-substituted T, R³²-substituted-T-(C₁-C₆)alkyl, R³²-substituted-(C₂-C₄)alkenyl, R³²-substituted-(C₁-C₆)alkyl, R³²-substituted-(C₃-C7)cycloalkyl and R³²-substituted-(C₃-C7)cycloalkyl(C₁-C₆)alkyl;
R³¹ is independently selected from the group consisting of H and (C₁-C₄)alkyl;
T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;
R³² is independently selected from 1-3 substituents independently selected from the group consisting of H, halogeno, (C₁-C₄)alkyl, -OH, phenoxy, -CF₃, -NO₂, (C₁-C₄)alkoxy, methylenedioxy, oxo, (C₁-C₄)alkylsulfanyl, (C₁-C₄)alkylsulfinyl, (C₁-C₄)alkylsulfonyl, -N(CH₃)₂, -C(O)-NH(C₁-C₄)alkyl, -C(O)-N((C₁-C₄)alkyl)₂, -C(O)-(C₁-C₄)alkyl, -C(O)-(C₁-C₄)alkoxy and pyrrolidinylcarbonyl; or R³² is a covalent bond and R³¹, the nitrogen to which it is attached and R³² form a pyrrolidinyl, piperidinyl, N-methyl-piperazinyl, indolinyl or morpholinyl group, or a (C₁-C₄)alkoxycarbonyl-substituted pyrrolidinyl, piperidinyl, N-methylpiperazinyl, indolinyl or morpholinyl group;
Ar¹ is aryl or R¹⁰-substituted aryl;
Ar² is aryl or R¹¹-substituted aryl;
Q is -(CH₂)_{q}-, wherein q is 2-6, or, with the 3-position ring carbon of the azetidinone, forms the spiro group 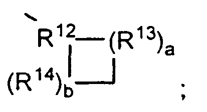
R¹² is 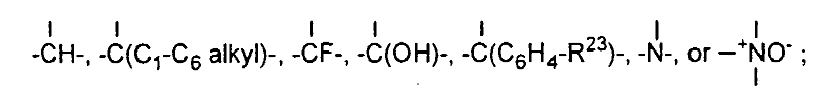
R¹³ and R¹⁴ are independently selected from the group consisting of -CH₂-, - CH(C₁-C₆ alkyl)-, -C(di-(C₁-C₆) alkyl), -CH=CH- and -C(C₁-C₆ alkyl)=CH-; or R¹² together with an adjacent R¹³, or R¹² together with an adjacent R¹⁴, form a -CH=CH-or a -CH=C(C₁-C₆ alkyl)- group;
a and b are independently 0, 1, 2 or 3, provided both are not zero; provided that when R¹³ is -CH=CH- or -C(C₁-C₆ alkyl)=CH-, a is 1; provided that when R¹⁴ is -CH=CH- or -C(C₁-C₆ alkyl)=CH-, b is 1; provided that when a is 2 or 3, the R¹³'s can be the same or different; and provided that when b is 2 or 3, the R¹⁴'s can be the same or different;
R¹⁰ and R¹¹ are independently selected from the group consisting of 1-3 substituents independently selected from the group consisting of (C₁-C₆)alkyl, -OR¹⁹, -O(CO)R¹⁹, -O(CO)OR²¹, -O(CH₂)₁₋₅₀R¹⁹, -O(CO)NR¹⁹R²⁰, -NR¹⁹R²⁰, -NR¹⁹(CO)R²⁰, -NR¹⁹(CO)OR²¹, -NR¹⁹(CO)NR²⁰R²⁵, -NR¹⁹SO₂R²¹, -COOR¹⁹, -CONR¹⁹R²⁰, -COR¹⁹, -SO₂NR¹⁹R²⁰, S(O)₀₋₂R²¹, -O(CH₂)₁₋₁₀-COOR¹⁹, -O(CH₂)₁₋₁₀CONR¹⁹R²⁰, -(C₁-C₆ alkylene)-COOR¹⁹, -CH=CH-COOR¹⁹,-CF₃,-CN,
   -NO₂ and halogen;
Ar¹ can also be pyridyl, isoxazolyl, furanyl, pyrrolyl, thienyl, imidazolyl, pyrazolyl, thiazolyl, pyrazinyl, pyrimidinyl or pyridazinyl;
R¹⁹ and R²⁰ are independently selected from the group consisting of H, (C₁-C₆)alkyl, aryl and aryl-substituted (C₁-C₆)alkyl;
R21 is (C₁-C₆)alkyl, aryl or R²⁴-substituted aryl;
R²² is H, (C₁-C₆)alkyl, aryl (C₁-C₆)alkyl, -C(O)R¹⁹ or -COOR¹⁹;
R23 and R²⁴ are independently 1-3 groups independently selected from the group consisting of H, (C₁-C₆)alkyl, (C₁-C₆)alkoxy, -COOH, NO₂, -NR¹⁹R²⁰, -OH and halogeno; and
R25 is H, -OH or (C₁-C₆)alkoxy.

Ar² is preferably phenyl or R¹¹-phenyl, especially (4-R¹¹) -substituted phenyl. Preferred definitions of R¹¹ are lower alkoxy,
especially methoxy, and halogeno, especially fluoro.

Ar¹ is preferably phenyl or R¹⁰-substituted phenyl, especially (4-R¹⁰)-substituted phenyl. A preferred definition of R¹⁰ is halogeno, especially fluoro.

Preferably Q is a lower alkyl or a spiro group as defined above, wherein preferably R¹³ and R¹⁴ are each ethylene and R¹² is -CH- or -C(OH)-.

A preferred compound of formula IX, therefore, is one wherein R¹ is as defined above and in which the remaining variables have the following definitions:
Ar¹ is phenyl or R¹⁰-substituted phenyl, wherein R¹⁰ is halogeno;
Ar² is phenyl or R¹¹-phenyl, wherein R¹¹ is 1 to 3 substituents independently selected from the group consisting of C₁-C₆ alkoxy and halogeno;
Q is a lower alkyl (i.e. C-1 to C-2) with Q = C-2 being preferred, or Q, with the 3-position ring carbon of the azetidinone, forms the group 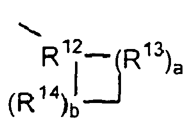 wherein preferably R¹³ and R¹⁴ are each ethylene and a and b are each 1, and
   wherein R¹² is

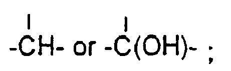

Preferred variables for R¹ groups of the formula 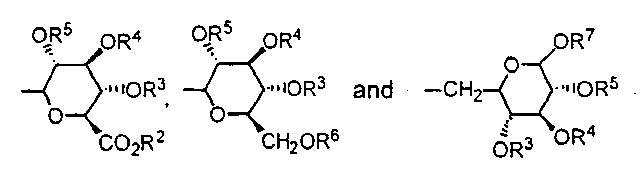 are as follows:
R², R³, R⁴, R⁵, R⁶ and R⁷ are independently selected from the group consisting of H, (C₁-C₆)alkyl, benzyl and acetyl.

Preferred variables for group R¹ of the formula 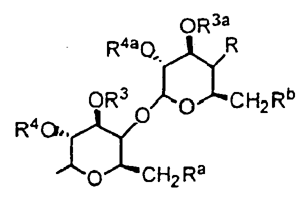 are as follows:
R³, R^{3a}, R⁴ and R^{4a} are selected from the group consisting of H, (C₁-C₆)alkyl, benzyl and acetyl;
R, R^{a} and R^{b} are independently selected from the group consisting of H, -OH, halogeno, -NH₂, azido, (C₁-C₆)alkoxy(C₁-C₆)alkoxy and -W-R³⁰, wherein W is -O-C(O)- or -O-C(O)-NR³¹-, R³¹ is H and R³⁰ is (C₁-C₆)alkyl, -C(O)-(C₁-C₄)alkoxy-(C₁-C₆)alkyl, T , T-(C₁-C₆)alkyl, or T or T-(C₁-C₆)alkyl wherein T is substituted by one or two halogeno or (C₁-C₆)alkyl groups.

Preferred R³⁰ substituents are 2-fluorophenyl, 2,4-difluoro-phenyl, 2,6-dichlorophenyl, 2-methylphenyl, 2-thienylmethyl, 2-methoxy-carbonylethyl, thiazol-2-yl-methyl, 2-furyl, 2-methoxycarbonylbutyl and phenyl. Preferred combinations of R, R^{a} and R^{b} are as follows: 1) R, R^{a} and R^{b} are independently -OH or -O-C(O)-NH-R³⁰, especially wherein R^{a} is -OH and R and R^{b} are -O-C(O)-NH-R³⁰ and R³⁰ is selected from the preferred substituents identified above, or wherein R and R^{a} are OH and R^{b} is -O-C(O)NH-R³⁰ wherein R³⁰ is 2-fluorophenyl, 2,4-difluoro-phenyl, 2,6-dichlorophenyl; 2) R^{a} is -OH, halogeno, azido or (C₁-C₆)-alkoxy(C₁-C₆)alkoxy, R^{b} is H, halogeno, azido or (C₁-C₆)alkoxy(C₁-C₆)-alkoxy, and R is -O-C(O)NH-R³⁰, especially compounds wherein R^{a} is -OH, R^{b} is H and R³⁰ is 2-fluorophenyl; 3) R, R^{a} and R^{b} are independently -OH or -O-C(O)-R³⁰ and R³⁰ is (C₁-C₆)alkyl, T , or T substituted by one or two halogeno or (C₁-C₆)alkyl groups, especially compounds wherein R is -OH and R^{a} and R^{b} are -O-C(O)R³⁰ wherein R³⁰ is 2-furyl; and 4) R, R^{a} and R^{b} are independently -OH or halogeno. Three additional classes of preferred are compounds are those wherein the C^{1'} anomeric oxy is beta, wherein the C^{2'} anomeric oxy is beta, and wherein the R group is alpha.

R¹ is preferably selected from: 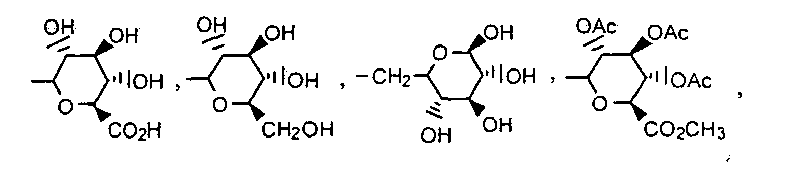 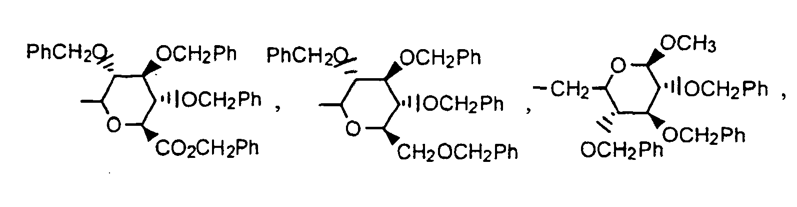 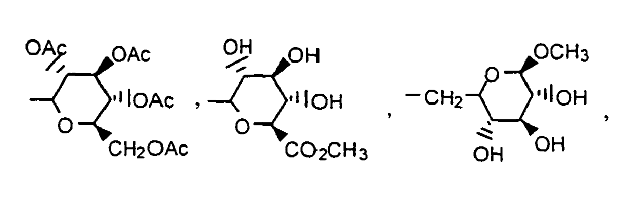 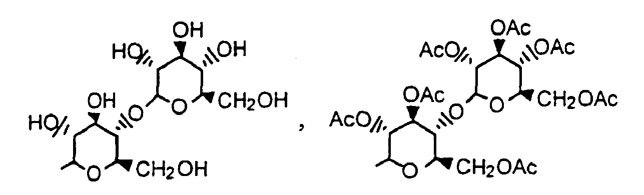 and 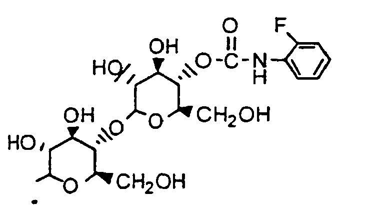 wherein Ac is acetyl and Ph is phenyl.

An example of a useful compound of this invention is one represented by the formula X: 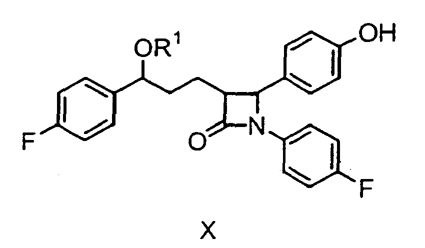 or pharmaceutically acceptable salts or solvates of the compound of Formula (X), or prodrugs of the compound of Formula (X) or of the pharmaceutically acceptable salts or solvates of the compound of Formula (X), wherein R¹ is defined as above.

A more preferred compound is one represented by formula XI: 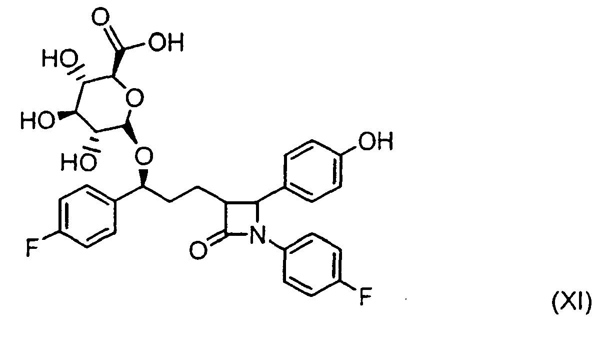 or pharmaceutically acceptable salts or solvates of the compound of Formula (X), or prodrugs of the compound of Formula (XI) or of the pharmaceutically acceptable salts or solvates of the compound of Formula (XI).

In another embodiment, compositions, pharmaceutical compositions, therapeutic combinations, kits and methods of treatment as described above are provided which comprise: (a) a first amount of at least one blood modifier; and (b) a second amount of at least one substituted azetidinone compound or at least one substituted β-lactam compound or isomers of the at least one substituted azetidinone compound or the at least one substituted β-lactam compound, or pharmaceutically acceptable salts or solvates of the at least one substituted azetidinone compound or the at least one substituted β-lactam compound or of the isomers of the at least one substituted azetidinone compound or the at least one substituted β-lactam compound, or prodrugs of the at least one substituted azetidinone compound or the at least one substituted β-lactam compound, or isomers, salts or solvates of the at least one substituted azetidinone compound or the at least one substituted β-lactam compound, wherein the first amount and the second amount together in their totality (whether administered concurrently or consecutively) comprise a therapeutically effective amount for the treatment or prevention of a vascular condition, diabetes, obesity or lowering a concentration of a sterol in plasma of a mammal. Suitable substituted azetidinone compounds or substituted β-lactam compounds can be selected from any of the compounds discussed above in Formulae I-XI. Other useful substituted azetidinone compounds include N-sulfonyl-2-azetidinones such as are disclosed in U.S. Patent No. 4,983,597 and ethyl 4-(2-oxoazetidin-4-yl)phenoxy-alkanoates such as are disclosed in Ram et al., Indian J. Chem. Sect. B. 29B, 12 (1990), p. 1134-7, which are incorporated by reference herein.

The compounds of Formulae I-XI can be prepared by known methods, including the methods discussed above and, for example, WO 93/02048 describes the preparation of compounds wherein -R¹-Q- is alkylene, alkenylene or alkylene interrupted by a hetero atom, phenylene or cycloalkylene; WO 94/17038 describes the preparation of compounds wherein Q is a spirocyclic group; WO 95/08532 describes the preparation of compounds wherein -R¹-Q- is a hydroxy-substituted alkylene group; PCT/US95/03196 describes compounds wherein -R¹-Q- is a hydroxy-substituted alkylene attached to the Ar¹ moiety through an -O- or S(O)₀₋₂-group; and U.S. Serial No. 08/463,619, filed June 5, 1995, describes the preparation of compounds wherein -R¹-Q- is a hydroxy-substituted alkylene group attached the azetidinone ring by a -S(O)₀₋₂- group.

The daily dose of the sterol absorption inhibitor(s) can range from about 0.1 to about 1000 mg per day, preferably about 0.25 to about 50 mg/day, and more preferably about 10 mg per day, given in a single dose or 2-4 divided doses. The exact dose, however, is determined by the attending clinician and is dependent on the potency of the compound administered, the age, weight, condition and response of the patient.

For administration of pharmaceutically acceptable salts of the above compounds, the weights indicated above refer to the weight of the acid equivalent or the base equivalent of the therapeutic compound derived from the salt.

In one embodiment of the present invention, the compositions or therapeutic combinations can further comprise one or more pharmacological or therapeutic agents or drugs such as cholesterol biosynthesis inhibitors and/or lipid-lowering agents discussed below.

In another embodiment, the composition or treatment can further comprise one or more cholesterol biosynthesis inhibitors coadministered with or in combination with the blood modifier(s) and sterol absorption inhibitor(s) discussed above.

Non-limiting examples of cholesterol biosynthesis inhibitors for use in the compositions, therapeutic combinations and methods of the present invention include competitive inhibitors of HMG CoA reductase, the rate-limiting step in cholesterol biosynthesis, squalene synthase inhibitors, squalene epoxidase inhibitors and mixtures thereof. Non-limiting examples of suitable HMG CoA reductase inhibitors include statins such as lovastatin (for example MEVACOR® which is available from Merck & Co.), pravastatin (for example PRAVACHOL® which is available from Bristol Meyers Squibb), fluvastatin, simvastatin (for example ZOCOR® which is available from Merck & Co.), atorvastatin, cerivastatin, rosuvastatin, rivastatin (sodium 7-(4-fluorophenyl)-2,6-diisopropyl-5-methoxymethylpyridin-3-yl)-3,5-dihydroxy-6-heptanoate Cl-981 and pitavastatin (such as NK-104 of Negma Kowa of Japan); HMG CoA synthetase inhibitors, for example L-659,699 ((E,E)-11-[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyl-N-(6,6-dimethyl-2-hepten-4-ynyl)-3-[(3,3'-bithiophen-5-yl)methoxy]benzene-methanamine hydrochloride) and other sterol biosynthesis inhibitors such as DMP-565. Preferred HMG CoA reductase inhibitors include lovastatin, pravastatin and simvastatin. The most preferred HMG CoA reductase inhibitor is simvastatin.

Generally, a total daily dosage of cholesterol biosynthesis inhibitor(s) can range from about 0.1 to about 160 mg per day, and preferably about 0.2 to about 80 mg/day in single or 2-3 divided doses.

In another preferred embodiment, the composition or treatment comprises the compound of Formula (II) in combination with one or more blood modifiers and one or more cholesterol biosynthesis inhibitors. Preferably the cholesterol biosynthesis inhibitor comprises one or more HMG CoA reductase inhibitors, such as, for example, lovastatin, pravastatin and/or simvastatin.

In another alternative embodiment, the compositions or treatments of the present invention can further comprise nicotinic acid (niacin) and/or derivatives thereof coadministered with or in combination with the blood modifier(s); and sterol absorption inhibitor(s) discussed above.

As used herein, "nicotinic acid derivative" means a compound comprising a pyridine-3-carboxylate structure or a pyrazine-2-carboxylate structure, including acid forms, salts, esters, zwitterions and tautomers, where available. Examples of nicotinic acid derivatives include niceritrol, nicofuranose and acipimox (5-methyl pyrazine-2-carboxylic acid 4-oxide). Nicotinic acid and its derivatives inhibit hepatic production of VLDL and its metabolite LDL and increases HDL and apo A-1 levels. An example of a suitable nicotinic acid product is NIASPAN® (niacin extended-release tablets) which are available from Kos.

Generally, a total daily dosage of nicotinic acid or a derivative thereof can range from about 500 to about 10,000 mg/day, preferably about 1000 to about 8000 mg/day, and more preferably about 3000 to about 6000 mg/day in single or divided doses.

In another alternative embodiment, the compositions or treatments of the present invention can further comprise one or more AcylCoA:Cholesterol O-acyltransferase ("ACAT") Inhibitors, which can reduce LDL and HDL levels, coadministered with or in combination with the blood modifier(s) and sterol absorption inhibitor(s) discussed above. ACAT is an enzyme responsible for esterifying excess intracellular cholesterol and may reduce the synthesis of VLDL, which is a product of cholesterol esterification, and overproduction of apo B-100-containing lipoproteins.

Non-limiting examples of useful ACAT inhibitors include avasimibe ([[2,4,6-tris(1-methylethyl)phenyl]acetyl]sulfamic acid, 2,6-bis(1-methylethyl)phenyl ester, formerly known as Cl-1011), HL-004, lecimibide (DuP-128) and CL-277082 (N-(2,4-difluorophenyl)-*N*-[[4-(2,2-dimethylpropyl)phenyl]methyl]-*N*-heptylurea). See P. Chang et al., "Current, New and Future Treatments in Dyslipidaemia and Atherosclerosis", Drugs 2000 Jul;60(1); 55-93, which is incorporated by reference herein.

In another alternative embodiment, the compositions or treatments of the present invention can further comprise probucol or derivatives thereof (such as AGI-1067 and other derivatives disclosed in U.S. Patents Nos. 6,121,319 and 6,147,250), which can reduce LDL and HDL levels, coadministered with or in combination with the blood modifier(s) and sterol absorption inhibitor(s) discussed above.

Generally, a total daily dosage of probucol or derivatives thereof can range from about 10 to about 2000 mg/day, and preferably about 500 to about 1500 mg/day in single or 2-4 divided doses.

In another alternative embodiment, the compositions or treatments of the present invention can further comprise low-density lipoprotein (LDL) receptor activators, coadministered with or in combination with the blood modifier(s) and sterol absorption inhibitor(s) discussed above. Non-limiting examples of suitable LDL-receptor activators include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler. Thromb. 1993; 13:1005-12.

Generally, a total daily dosage of LDL receptor activator(s) can range from about 1 to about 1000 mg/day in single or 2-4 divided doses.

In another alternative embodiment, the compositions or treatments of the present invention can further comprise fish oil, which contains Omega 3 fatty acids (3-PUFA), which can reduce VLDL and triglyceride levels, coadministered with or in combination with the blood modifier(s) and sterol absorption inhibitor(s) discussed above. Generally, a total daily dosage of fish oil or Omega 3 fatty acids can range from about 1 to about 30 grams per day in single or 2-4 divided doses.

In another alternative embodiment, the compositions or treatments of the present invention can further comprise natural water soluble fibers, such as psyllium, guar, oat and pectin, which can reduce cholesterol levels, coadministered with or in combination with the blood modifier(s) and sterol absorption inhibitor(s) discussed above. Generally, a total daily dosage of natural water soluble fibers can range from about 0.1 to about 10 grams per day in single or 2-4 divided doses.

In another alternative embodiment, the compositions or treatments of the present invention can further comprise plant sterols, plant stanols and/or fatty acid esters of plant stanols, such as sitostanol ester used in BENECOL® margarine, which can reduce cholesterol levels, coadministered with or in combination with the blood modifier(s) and sterol absorption inhibitor(s) discussed above. Generally, a total daily dosage of plant sterols, plant stanols and/or fatty acid esters of plant stanols can range from about 0.5 to about 20 grams per day in single or 2-4 divided doses.

In another alternative embodiment, the compositions or treatments of the present invention can further comprise antioxidants, such as probucol, tocopherol, ascorbic acid, β-carotene and selenium, or vitamins such as vitamin B₆ or vitamin B₁₂, coadministered with or in combination with the blood modifier(s) and sterol absorption inhibitor(s) discussed above. Generally, a total daily dosage of antioxidants or vitamins can range from about 0.05 to about 10 grams per day in single or 2-4 divided doses.

In another alternative embodiment, the compositions, therapeutic combinations or methods of the present invention can further comprise one or more bile acid sequestrants (insoluble anion exchange resins), coadministered with or in combination with the blood modifier(s) and sterol absorption inhibitor(s) discussed above.

Bile acid sequestrants bind bile acids in the intestine, interrupting the enterohepatic circulation of bile acids and causing an increase in the faecal excretion of steroids. Use of bile acid sequestrants is desirable because of their non-systemic mode of action. Bile acid sequestrants can lower intrahepatic cholesterol and promote the synthesis of apo B/E (LDL) receptors which bind LDL from plasma to further reduce cholesterol levels in the blood.

Non-limiting examples of suitable bile acid sequestrants include cholestyramine (a styrene-divinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo), water soluble derivatives such as 3,3-ioene, N-(cycloalkyl) alkylamines and poliglusam, insoluble quatemized polystyrenes, saponins and mixtures thereof. Other useful bile acid sequestrants are disclosed in PCT Patent Applications Nos. WO 97/11345 and WO 98/57652, and U.S. Patents Nos. 3,692,895 and 5,703,188 which are incorporated herein by reference. Suitable inorganic cholesterol sequestrants include bismuth salicylate plus montmorillonite clay, aluminum hydroxide and calcium carbonate antacids.

Generally, a total daily dosage of bile acid sequestrant(s) can range from about 1 to about 50 grams per day, and preferably about 2 to about 16 grams per day in single or 2-4 divided doses.

Also useful with the present invention are compositions or therapeutic combinations that can further comprise at least one (one or more) activators for peroxisome proliferator-activated receptors (PPAR). The activators act as agonists for the peroxisome proliferator-activated receptors. Three subtypes of PPAR have been identified, and these are designated as peroxisome proliferator-activated receptor alpha (PPARα), peroxisome proliferator-activated receptor gamma (PPARγ) and peroxisome proliferator-activated receptor delta (PPARδ). It should be noted that PPARδ is also referred to in the literature as PPARβ and as NUC1, and each of these names refers to the same receptor.

PPARα regulates the metabolism of lipids. PPARα is activated by fibrates and a number of medium and long-chain fatty acids, and it is involved in stimulating β-oxidation of fatty acids. The PPARγ receptor subtypes are involved in activating the program of adipocyte differentiation and are not involved in stimulating peroxisome proliferation in the liver. PPARδ has been identified as being useful in increasing high density lipoprotein (HDL) levels in humans. See, e.g., WO 97/28149.

PPARα activator compounds are useful for, among other things, lowering triglycerides, moderately lowering LDL levels and increasing HDL levels. Useful examples of PPARα activators include fibrates.

Non-limiting examples of suitable fibric acid derivatives ("fibrates") include clofibrate (such as ethyl 2-(p-chlorophenoxy)-2-methyl-propionate, for example ATROMID-S® Capsules which are commercially available from Wyeth-Ayerst); gemfibrozil (such as 5-(2,5-dimethylphenoxy)-2,2-dimethylpentanoic acid, for example LOPID® tablets which are commercially available from Parke Davis); ciprofibrate (C.A.S. Registry No. 52214-84-3, see U.S. Patent No. 3,948,973 which is incorporated herein by reference); bezafibrate (C.A.S. Registry No. 41859-67-0, see U.S. Patent No. 3,781,328 which is incorporated herein by reference); clinofibrate (C.A.S. Registry No. 30299-08-2, see U.S. Patent No. 3,716,583 which is incorporated herein by reference); binifibrate (C.A.S. Registry No. 69047-39-8, see BE 884722 which is incorporated herein by reference); lifibrol (C.A.S. Registry No. 96609-16-4); fenofibrate (such as TRICOR® micronized fenofibrate (2-[4-(4-chlorobenzoyl) phenoxy]-2-methyl-propanoic acid, 1-methylethyl ester) which is commercially available from Abbott Laboratories or LIPANTHYL® micronized fenofibrate which is commercially available from Labortoire Founier, France) and mixtures thereof. These compounds can be used in a variety of forms, including but not limited to acid form, salt form, racemates, enantiomers, zwitterions and tautomers.

Other examples of PPARα activators useful with the practice of the present invention include suitable fluorophenyl compounds as disclosed in U.S. No. 6,028,109 which is incorporated herein by reference; certain substituted phenylpropionic compounds as disclosed in WO 00/75103 which is incorporated herein by reference; and PPARα activator compounds as disclosed in WO 98/43081 which is incorporated herein by reference.

Non-limiting examples of suitable PPARγ activators include derivatives of glitazones or thiazolidinediones, such as, troglitazone (such as REZULIN® troglitazone (-5-[[4-[3,4-dihydro-6-hydroxy-2,5,7,8-tetramethyl-2H-1-benzopyran-2-yl)methoxy]phenyl] methyl]-2,4-thiazolidinedione) commercially available from Parke-Davis); rosiglitazone (such as AVANDIA® rosiglitazone maleate (-5-[[4-[2-(methyl-2-pyridinylamino)ethoxy] phenyl] methyl]-2,4-thiazolidinedione, (Z) -2-butenedioate) (1:1) commercially available from SmithKline Beecham) and pioglitazone (such as ACTOS^{™} pioglitazone hydrochloride (5-[[4-[2-(5-ethyl-2-pyridinyl)ethoxy]phenyl]methyl]-2,4-] thiazolidinedione monohydrochloride) commercially available from Takeda Pharmaceuticals). Other useful thiazolidinediones include ciglitazone, englitazone, darglitazone and BRL 49653 as disclosed in WO 98/05331 which is incorporated herein by reference; PPARγ activator compounds disclosed in WO 00/76488 which is incorporated herein by reference; and PPARy activator compounds disclosed in U.S. Patent No. 5,994,554 which is incorporated herein by reference.

Other useful PPARγ activator compounds include certain acetylphenols as disclosed in U.S. Patent No. 5,859,051 which is incorporated herein by reference; certain quinoline phenyl compounds as disclosed in WO 99/20275 which is incorporated herein by reference; aryl compounds as disclosed by WO 99/38845 which is incorporated herein by reference; certain 1,4-disubstituted phenyl compounds as disclosed in WO 00/63161; certain aryl compounds as disclosed in WO 01/00579 which is incorporated herein by reference; benzoic acid compounds as disclosed in WO 01/12612 and WO 01/12187 which are incorporated herein by reference; and substituted 4-hydroxy-phenylalconic acid compounds as disclosed in WO 97/31907 which is incorporated herein by reference.

PPARδ compounds are useful for, among other things, lowering triglyceride levels or raising HDL levels. Non-limiting examples of PPARδ activators include suitable thiazole and oxazole derivates, such as C.A.S. Registry No. 317318-32-4, as disclosed in WO 01/00603 which is incorporated herein by reference); certain fluoro, chloro or thio phenoxy phenylacetic acids as disclosed in WO 97/28149 which is incorporated herein by reference; suitable non-β-oxidizable fatty acid analogues as disclosed in U.S. Patent No. 5,093,365 which is incorporated herein by reference; and PPARδ compounds as disclosed in WO 99/04815 which is incorporated herein by reference.

Moreover, compounds that have multiple functionality for activating various combinations of PPARα, PPARγ and PPARδ are also useful with the practice of the present invention. Non-limiting examples include certain substituted aryl compounds as disclosed in U.S. Patent No. 6,248,781; WO 00/23416; WO 00123415; WO 00/23425; WO 00/23445; WO 00/23451; and WO 00/63153, all of which are incorporated herein by reference, are described as being useful PPARα and/or PPARγ activator compounds. Other non-limiting examples of useful PPARα and/or PPARγ activator compounds include activator compounds as disclosed in WO 97/25042 which is incorporated herein by reference; activator compounds as disclosed in WO 00/63190 which is incorporated herein by reference; activator compounds as disclosed in WO 01/21181 which is incorporated herein by reference; biaryl-oxa(thia)zole compounds as disclosed in WO 01/16120 which is incorporated herein by reference; compounds as disclosed in WO 00/63196 and WO 00/63209 which are incorporated herein by reference; substituted 5-aryl-2,4-thiazolidinediones compounds as disclosed in U.S. Patent No. 6,008,237 which is incorporated herein by reference; arylthiazolidinedione and aryloxazolidinedione compounds as disclosed in WO 00/78312 and WO 00/78313G which are incorporated herein by reference; GW2331 or (2-(4-[difluorophenyl]-1 heptylureido)ethyl]phenoxy)-2-methylbutyric compounds as disclosed in WO 98/05331 which is incorporated herein by reference; aryl compounds as disclosed in U.S. Patent No. 6,166,049 which is incorporated herein by reference; oxazole compounds as disclosed in WO 01/17994 which is incorporated herein by reference; and dithiolane compounds as disclosed in WO 01/25225 and WO 01/25226 which are incorporated herein by reference.

Other useful PPAR activator compounds include substituted benzylthiazolidine-2,4-dione compounds as disclosed in WO 01/14349, WO 01/14350 and WO/01/04351 which are incorporated herein by reference; mercaptocarboxylic compounds as disclosed in WO 00/50392 which is incorporated herein by reference; ascofuranone compounds as disclosed in WO 00/53563 which is incorporated herein by reference; carboxylic compounds as disclosed in WO 99/46232 which is incorporated herein by reference; compounds as disclosed in WO 99/12534 which is incorporated herein by reference; benzene compounds as disclosed in WO 99/15520 which is incorporated herein by reference; o-anisamide compounds as disclosed in WO 01/21578 which is incorporated herein by reference; and PPAR activator compounds as disclosed in WO 01/40192 which is incorporated herein by reference.

The peroxisome proliferator-activated receptor(s) activator(s) are administered in a therapeutically effective amount to treat the specified condition, for example in a daily dose preferably ranging from about 50 to about 3000 mg per day, and more preferably about 50 to about 2000 mg per day, given in a single dose or 2-4 divided doses. The exact dose, however, is determined by the attending clinician and is dependent on such factors as the potency of the compound administered, the age, weight, condition and response of the patient.

In an alternative embodiment, the compositions or treatments of the present invention can further comprise one or more ileal bile acid transport ("IBAT") inhibitors (or apical sodium co-dependent bile acid transport ("ASBT") inhibitors) coadministered with or in combination with the blood modifier(s) and sterol absorption inhibitor(s) discussed above. The IBAT inhibitors can inhibit bile acid transport to reduce LDL cholesterol levels. Non-limiting examples of suitable IBAT inhibitors include benzothiepines such as therapeutic compounds comprising a 2,3,4,5-tetrahydro-1-benzothiepine 1,1-dioxide structure such as are disclosed in PCT Patent Application WO 00/38727 which is incorporated herein by reference.

Generally, a total daily dosage of IBAT inhibitor(s) can range from about 0.01 to about 1000 mg/day, and preferably about 0.1 to about 50 mg/day in single or 2-4 divided doses.

In another alternative embodiment, the compositions or treatments of the present invention can further comprise one or more Cholesteryl Ester Transfer Protein ("CETP") Inhibitors coadministered with or in combination with the blood modifier(s) and sterol absorption inhibitor(s) discussed above. CETP is responsible for the exchange or transfer of cholesteryl ester carrying HDL and triglycerides in VLDL.

Non-limiting examples of suitable CETP inhibitors are disclosed in PCT Patent Application No. WO 00/38721 and U.S. Patent No. 6,147,090, which are incorporated herein by reference. Pancreatic cholesteryl ester hydrolase (pCEH) inhibitors such as WAY-121898 also can be coadministered with or in combination with the peroxisome proliferator-activated receptor(s) activator and sterol absorption inhibitor(s) discussed above.

Generally, a total daily dosage of CETP inhibitor(s) can range from about 0.01 to about 1000 mg/day, and preferably about 0.5 to about 20 mg/kg body weight/day in single or divided doses.

Also useful with the present invention are compositions or therapeutic combinations that further comprise hormone replacement agents and compositions. Useful hormone agents and compositions for hormone replacement therapy of the present invention include androgens, estrogens, progestins, their pharmaceutically acceptable salts and derivatives. Combinations of these agents and compositions are also useful.

The dosage of androgen and estrogen combinations vary, desirably from about 1 mg to about 4 mg androgen and from about 1 mg to about 3 mg estrogen. Examples include, but are not limited to, androgen and estrogen combinations such as the combination of esterified estrogens (sodium estrone sulfate and sodium equilin sulfate) and methyltestosterone (17-hydroxy-17-methyl-, (17B)- androst-4-en-3-one) available from Solvay Pharmaceuticals, Inc., Marietta, GA, under the tradename Estratest.

Estrogens and estrogen combinations may vary in dosage from about 0.01 mg up to 8 mg, desirably from about 0.3 mg to about 3.0 mg. Examples of useful estrogens and estrogen combinations include:
(a) the blend of nine (9) synthetic estrogenic substances including sodium estrone sulfate, sodium equilin sulfate, sodium 17 α -dihydroequilin sulfate, sodium 17 α -estradiol sulfate, sodium 17 β -dihydroequilin sulfate, sodium 17 α -dihydroequilenin sulfate, sodium 17 β -dihydroequilenin sulfate, sodium equilenin sulfate and sodium 17 β -estradiol sulfate; available from Duramed Pharmaceuticals, Inc., Cincinnati, OH, under the tradename Cenestin;
(b) ethinyl estradiol (19-nor-17 α -pregna-1,3,5(10)-trien-20-yne-3,17-diol; available by Schering Plough Corporation, Kenilworth, NJ, under the tradename Estinyl;
(c) esterified estrogen combinations such as sodium estrone sulfate and sodium equilin sulfate; available from Solvay under the tradename Estratab and from Monarch Pharmaceuticals, Bristol, TN, under the tradename Menest;
(d) estropipate (piperazine estra-1,3,5(10)-trien-17-one, 3-(sulfooxy)-estrone sulfate); available from Pharmacia & Upjohn, Peapack, NJ, under the tradename Ogen and from Women First Health Care, Inc., San Diego, CA, under the tradename Ortho-Est; and
(e) conjugated estrogens (17 α-dihydroequilin, 17 α-estradiol, and 17 β-dihydroequilin); available from Wyeth-Ayerst Pharmaceuticals, Philadelphia, PA, under the tradename Premarin.

Progestins and estrogens may also be administered with a variety of dosages, generally from about .05 to about 2.0 mg progestin and about .001 mg to about 2 mg estrogen, desirably from about .1 mg to about 1 mg progestin and about 0.01 mg to about .5 mg estrogen. Examples of progestin and estrogen combinations that may vary in dosage and regimen include:
(a) the combination of estradiol (estra-1, 3, 5 (10)-triene-3, 17 β-diol hemihydrate) and norethindrone (17 β-acetoxy-19-nor-17 α-pregn-4-en-20-yn-3-one); which is available from Pharmacia & Upjohn, Peapack, NJ, under the tradename Activella;
(b) the combination of levonorgestrel (d(-)-13 β-ethyl-17 α-ethinyl-17 β-hydroxygon- 4-en-3-one) and ethinyl estradial; available from Wyeth-Ayerst under the tradename Alesse, from Watson Laboratories, Inc., Corona, CA, under the tradenames Levora and Trivora, Monarch Pharmaceuticals, under the tradename Nordette, and from Wyeth-Ayerst under the tradename Triphasil;
(c) the combination of ethynodiol diacetate (19-nor-17 α-pregn-4-en-20-yne-3β, 17-diol diacetate) and ethinyl estradiol; available from G.D. Searle & Co., Chicago, IL, under the tradename Demulen and from Watson under the tradename Zovia;
(d) the combination of desogestrel (13-ethyl-11- methylene-18,19-dinor-17 α-pregn- 4-en- 20-yn-17-ol) and ethinyl estradiol; available from Organon under the tradenames Desogen and Mircette, and from Ortho-McNeil Pharmaceutical, Raritan, NJ, under the tradename Ortho-Cept;
(e) the combination of norethindrone and ethinyl estradiol; available from Parke-Davis, Morris Plains, NJ, under the tradenames Estrostep and femhrt, from Watson under the tradenames Microgestin, Necon, and Tri-Norinyl, from Ortho-McNeil under the tradenames Modicon and Ortho-Novum, and from Warner Chilcott Laboratories, Rockaway, NJ, under the tradename Ovcon;
(f) the combination of norgestrel ( (±)-13-ethyl-17-hydroxy-18, 19-dinor-17 α-preg-4-en-20-yn-3-one) and ethinyl estradiol; available from Wyeth-Ayerst under the tradenames Ovral and Lo/Ovral, and from Watson under the tradenames Ogestrel and Low-Ogestrel;
(g) the combination of norethindrone, ethinyl estradiol, and mestranol (3-methoxy-19-nor-17 a-pregna-1,3,5(10)-trien-20-yn-17-ol); available from Watson under the tradenames Brevicon and Norinyl;
(h) the combination of 17 β-estradiol (estra-1,3,5(10)-triene-3,17 β-diol) and micronized norgestimate (17 α-17-(Acetyloxyl)-13-ethyl-18,19-dinorpregn-4-en-20-yn-3-one3-oxime); available from Ortho-McNeil under the tradename Ortho-Prefest;
(i) the combination of norgestimate (18,19-dinor-17-pregn-4-en-20-yn-3-one, 17-(acetyloxy)-13-etnyl-,oxime, (17(α)-(+)-) and ethinyl estradiol; available from Ortho-McNeil under the tradenames Ortho Cyclen and Ortho Tri-Cyclen; and
(j) the combination of conjugated estrogens (sodium estrone sulfate and sodium equilin sulfate) and medroxyprogesterone acetate (20-dione, 17-(acetyloxy)-6-methyl-, (6(α))- pregn-4-ene-3); available from Wyeth-Ayerst under the tradenames Premphase and Prempro.
   In general, a dosage of progestins may vary from about .05 mg to about 10 mg or up to about 200 mg if microsized progesterone is administered. Examples of progestins include norethindrone; available from ESI Lederie, Inc., Philadelphia, PA, under the tradename Aygestin, from Ortho-McNeil under the tradename Micronor, and from Watson under the tradename Nor-QD; norgestrel; available from Wyeth-Ayerst under the tradename Ovrette; micronized progesterone (pregn-4-ene-3, 20-dione); available from Solvay under the tradename Prometrium; and medroxyprogesterone acetate; available from Pharmacia & Upjohn under the tradename Provera.

The compositions, therapeutic combinations or methods of the present invention can further comprise one or more obesity control medications. Useful obesity control medications include, but are not limited to, drugs that reduce energy intake or suppress appetite, drugs that increase energy expenditure and nutrient-partitioning agents. Suitable obesity control medications include, but are not limited noradrenergic agents (such as diethylpropion, mazindol, phenylpropanolamine, phentermine, phendimetrazine, phendamine tartrate, methamphetamine, phendimetrazine and tartrate); serotonergic agents (such as sibutramine, fenfluramine, dexfenfluramine, fluoxetine, fluvoxamine and paroxtine); thermogenic agents (such as ephedrine, caffeine, theophylline, and selective β3-adrenergic agonists); an alpha-blocking agent; a kainite or AMPA receptor antagonist; a leptin-lipolysis stimulated receptor; a phosphodiesterase enzyme inhibitor; a compound having nucleotide sequences of the mahogany gene; a fibroblast growth factor-10 polypeptide; a monoamine oxidase inhibitor (such as befloxatone, moclobemide, brofaromine, phenoxathine, esuprone, befol, toloxatone, pirlindol, amiflamine, sercloremine, bazinaprine, lazabemide, milacemide and caroxazone); a compound for increasing lipid metabolism (such as evodiamine compounds); and a lipase inhibitor (such as orlistat). Generally, a total dosage of the above-described obesity control medications can range from 1 to 3,000 mg/day, desirably from about 1 to 1,000 mg/day and more desirably from about 1 to 200 mg/day in single or 2-4 divided doses.

The compositions, therapeutic combinations or methods of the present invention can further comprise one or more cardiovascular agents which are chemically or structurally different from the substituted azetidinone or substituted β-lactam compounds (such as compounds (I-XI) above) and the blood modifiers discussed above, for example, they contain one or more different atoms, have a different arrangement of atoms or a different number of one or more atoms than the sterol absorption inhibitor(s) discussed below. Useful cardiovascular agents include but are not limited to calcium channel blockers (clentiazem maleate, amlodipine besylate, isradipine, nimodipine, felodipine, nilvadipine, nifedipine, teludipine hydrochloride, diltiazem hydrochloride, belfosdil, verapamil hydrochloride, fostedil); adrenergic blockers (fenspiride hydrochloride, labetalol hydrochloride, proroxan, alfuzosin hydrochloride, acebutolol, acebutolol hydrochloride, alprenolol hydrochloride, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol sulfate, labetalol hydrochloride, levobetaxolol hydrochloride, levobunolol hydrochloride, metalol hydrochloride, metoprolol, metoprolol tartrate, nadolol, pamatolol sulfate, penbutolol sulfate, practolol, propranolol hydrochloride, sotalol hydrochloride, timolol, timolol maleate, tiprenolol hydrochloride, tolamolol, bisoprolol, bisoprolol fumarate, nebivolol); adrenergic stimulants; angiotensin converting enzyme (ACE) inhibitors (benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride, spiraprilat, teprotide, enalapril maleate, lisinopril, zofenopril calcium, perindopril erbumine); antihypertensive agents (althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amlodipine besylate, amlodipine maleate, bevantolol hydrochloride); angiotensin II receptor antagonists (candesartan, irbesartan, losartan potassium, candesartan cilexetil, telmisartan); anti-anginal agents (amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride); coronary vasodilators (fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate, terodiline hydrochloride, tolamolol, verapamil); diuretics (the combination product of hydrochlorothiazide and spironolactone and the combination product of hydrochlorothiazide and triamterene).

The compositions, therapeutic combinations or methods of the present invention can further comprise one or more antidiabetic medications for reducing blood glucose levels in a human. Useful antidiabetic medications include, but are not limited to, drugs that reduce energy intake or suppress appetite, drugs that increase energy expenditure and nutrient-partitioning agents. Suitable antidiabetic medications include, but are not limited to, sulfonylurea (such as acetohexamide, chlorpropamide, gliamilide, gliclazide, glimepiride, glipizide, glyburide, glibenclamide, tolazamide, and tolbutamide), meglitinide (such as repaglinide and nateglinide), biguanide (such as metformin and buformin), thiazolidinedione (such as troglitazone, rosiglitazone, pioglitazone, ciglitazone, englitazone, and darglitazone), alpha-glucosidase inhibitor (such as acarbose, miglitol, camiglibose, and voglibose), certain peptides (such as amlintide, pramlintide, exendin, and GLP-1 agonistic peptides), and orally administrable insulin or insulin composition for intestinal delivery thereof. Generally, a total dosage of the above-described antidiabetic medications can range from 0.1 to 1,000 mg/day in single or 2-4 divided doses.

Compositions and therapeutic combinations of the present invention having the above-described sterol absorption inhibitors are also useful for treating or preventing vascular inflammation or for reducing c-reactive protein levels in a mammal in need of such treatment. Vascular inflammation refers to arterial damage and bodily responses thereto. For example, cholesteryl esters are a major component of atherosclerotic lesions which results in vascular inflammation and an increase in plasma c-reactive protein levels. The inhibition of cholesteryl ester formation and reduction of serum cholesterol can inhibit the progression of atherosclerotic lesion formation, thereby treating or preventing vascular inflammation. Moreover, these sterol absorption inhibitors are useful lowering or controlling c-reactive protein blood levels in a mammal to less than about 3.4 mg/dL, desirably to less than 1.0 mg/dL, and more desirably to less than 0.4 mg/dL.

Mixtures of any of the pharmacological or therapeutic agents described above can be used in the compositions and therapeutic combinations of these other embodiments of the present invention.

The compositions and therapeutic combinations of the present invention can be administered to a mammal in need of such treatment in a therapeutically effective amount to treat vascular conditions such as vascular conditions. The compositions and treatments can be administered by any suitable means that produce contact of these compounds with the site of action in the body, for example in the plasma, liver or small intestine of a mammal.

The daily dosage for the various compositions and therapeutic combinations described above can be administered to a patient in a single dose or in multiple subdoses, as desired. Subdoses can be administered 2 to 6 times per day, for example. Sustained release dosages can be used. Where the blood modifiers and sterol absorption inhibitor(s) are administered in separate dosages, the number of doses of each component given per day may not necessarily be the same, e.g., one component may have a greater duration of activity and will therefore need to be administered less frequently.

The pharmaceutical treatment compositions and therapeutic combinations of the present invention can further comprise one or more pharmaceutically acceptable carriers, one or more excipients and/or one or more additives. Non-limiting examples of pharmaceutically acceptable carriers include solids and/or liquids such as ethanol, glycerol, water and the like. The amount of carrier in the treatment composition can range from about 5 to about 99 weight percent of the total weight of the treatment composition or therapeutic combination. Non-limiting examples of suitable pharmaceutically acceptable excipients and additives include non-toxic compatible fillers, binders such as starch, disintegrants, buffers, preservatives, anti-oxidants, lubricants, flavorings, thickeners, coloring agents, emulsifiers and the like. The amount of excipient or additive can range from about 0.1 to about 90 weight percent of the total weight of the treatment composition or therapeutic combination. One skilled in the art would understand that the amount of carrier(s), excipients and additives (if present) can vary.

The treatment compositions of the present invention can be administered in any conventional dosage form, preferably an oral dosage form such as a capsule, tablet, powder, cachet, suspension or solution. The formulations and pharmaceutical compositions can be prepared using conventional pharmaceutically acceptable and conventional techniques. Several examples of preparation of dosage formulations are provided below.

The following formulations exemplify some of the dosage forms of this invention. In each formulation, the term "Active Compound I" designates sterol absorption inhibitor(s) described herein above and the term "Active Compound II" designates blood modifier(s) described herein above.

**EXAMPLE**

| | Tablets | |
|---|---|---|
| No. | Ingredient | mg/tablet |
| 1 | Active Compound I | 10 |
| 2 | Lactose monohydrate NF | 55 |
| 3 | Microcrystalline cellulose NF | 20 |
| 4 | Povidone (K29-32) USP | 4 |
| 5 | Croscarmellose sodium NF | 8 |
| 6 | Sodium lauryl sulfate | 2 |
| 7 | Magnesium stearate NF | 1 |
| | Total | 100 |

In the present invention, the above-described tablet can be coadministered with a tablet, capsule, etc. comprising a dosage of Active Compound II, for example a blood modifier as described above.

### Method of Manufacture

Mix Item No. 4 with purified water in suitable mixer to form binder solution. Spray the binder solution and then water over Items 1, 2, 6 and a portion of Item 5 in a fluidized bed processor to granulate the ingredients. Continue fluidization to dry the damp granules. Screen the dried granules and blend with Item No. 3 and the remainder of Item 5. Add Item No. 7 and mix. Compress the mixture to appropriate size and weight on a suitable tablet machine.

For coadministration in separate tablets or capsules, representative formulations comprising a sterol absorption inhibitor such as are discussed above are well known in the art and representative formulations comprising a blood modifier such as are discussed above are well known in the art. It is contemplated that where the two active ingredients are administered as a single composition, the dosage forms disclosed above for sterol absorption inhibitor may readily be modified using the knowledge of one skilled in the art.

Since the present invention relates to treating vascular conditions or reducing the plasma sterol (especially cholesterol) concentrations or levels or controlling properties of the blood, such as viscosity, by treatment with a combination of active ingredients wherein the active ingredients may be administered separately, the invention also relates to combining separate pharmaceutical compositions in kit form. That is, a kit is contemplated wherein two separate units are combined: a pharmaceutical composition comprising at least one blood modifier and a separate pharmaceutical composition comprising at least one sterol absorption inhibitor as described above. The kit will preferably include directions for the administration of the separate components. The kit form is particularly advantageous when the separate components must be administered in different dosage forms (e.g., oral and parenteral) or are administered at different dosage intervals.

The treatment compositions and therapeutic combinations of the present invention can inhibit the intestinal absorption of cholesterol in mammals, and can be useful in the treatment and/or prevention of vascular conditions, such as vascular inflammation, atherosclerosis, hypercholesterolemia and sitosterolemia, stroke, vascular conditions and lowering of plasma levels of cholesterol in mammals, in particular in humans.

In another embodiment of the present invention, the compositions and therapeutic combinations of the present invention can inhibit sterol absorption or reduce plasma concentration of at least one sterol selected from the group consisting of phytosterols (such as sitosterol, campesterol, stigmasterol and avenosterol), 5α-stanols (such as cholestanol, 5α-campestanol, 5α-sitostanol), cholesterol and mixtures thereof. The plasma concentration can be reduced by administering to a mammal in need of such treatment an effective amount of at least one treatment composition or therapeutic combination comprising at least one blood modifier and at least one sterol absorption inhibitor described above. The reduction in plasma concentration of sterols can range from about 1 to about 70 percent, and preferably about 10 to about 50 percent. Methods of measuring serum total blood cholesterol and total LDL cholesterol are well known to those skilled in the art and for example include those disclosed in PCT WO 99/38498 at page 11, incorporated by reference herein. Methods of determining levels of other sterols in serum are disclosed in H. Gylling et al., "Serum Sterols During Stanol Ester Feeding in a Mildly Hypercholesterolemic Population", J. Lipid Res. 40: 593-600 (1999), incorporated by reference herein.

Illustrating the invention is the following example which, however, is not to be considered as limiting the invention to their details. Unless otherwise indicated, all parts and percentages in the following examples, as well as throughout the specification, are by weight.

### EXAMPLE

### PREPARATION OF COMPOUND OF FORMULA (II)

Step 1): To a solution of (S)-4-phenyl-2-oxazolidinone (41 g, 0.25 mol) in CH₂Cl₂ (200 ml), was added 4-dimethylaminopyridine (2.5 g, 0.02 mol) and triethylamine (84.7 ml, 0.61 mol) and the reaction mixture was cooled to 0°C. Methyl-4-(chloroformyl)butyrate (50 g, 0.3 mol) was added as a solution in CH₂Cl₂ (375 ml) dropwise over 1 h, and the reaction was allowed to warm to 22°C. After 17 h, water and H₂SO₄ (2N, 100 ml), was added the layers were separated, and the organic layer was washed sequentially with NaOH (10%), NaCl (sat'd) and water. The organic layer was dried over MgSO₄ and concentrated to obtain a semicrystalline product.

Step 2): To a solution of TiCl₄ (18.2 ml, 0.165 mol) in CH₂Cl₂ (600 ml) at 0°C, was added titanium isopropoxide (16.5 ml, 0.055 mol). After 15 min, the product of Step 1 (49.0 g, 0.17 mol) was added as a solution in CH₂Cl₂ (100 ml). After 5 min., diisopropylethylamine (DIPEA) (65.2 ml, 0.37 mol) was added and the reaction mixture was stirred at 0°C for 1 h, the reaction mixture was cooled to -20°C, and 4-benzyloxybenzylidine(4-fluoro)aniline (114.3 g, 0.37 mol) was added as a solid. The reaction mixture was stirred vigorously for 4 h at -20°C, then acetic acid was added as a solution in CH₂Cl₂ dropwise over 15 min, the reaction mixture was allowed to warm to 0°C, and H₂SO₄ (2N) was added. The reaction mixture was stirred an additional 1 h, the layers were separated, washed with water, separated and the organic layer was dried. The crude product was crystallized from ethanol/water to obtain the pure intermediate.

Step 3): To a solution of the product of Step 2 (8.9 g, 14.9 mmol) in toluene (100 ml) at 50°C, was added N,O-bis(trimethylsilyl)acetamide (BSA) (7.50 ml, 30.3 mmol). After 0.5 h, solid TBAF (0.39 g, 1.5 mmol) was added and the reaction mixture stirred at 50°C for an additional 3 h. The reaction mixture was cooled to 22°C, CH₃OH (10 ml), was added. The reaction mixture was washed with HCI (1 N), NaHCO₃ (1 N) and NaCl (sat'd.), and the organic layer was dried over MgSO₄.

Step 4): To a solution of the product of Step 3 (0.94 g, 2.2 mmol) in CH₃OH (3 ml), was added water (1 ml) and LiOH·H₂O (102 mg, 2.4 mmole). The reaction mixture was stirred at 22°C for 1 h and then additional LiOH·H₂O (54 mg, 1.3 mmole) was added. After a total of 2 h, HCl (1N) and EtOAc was added, the layers were separated, the organic layer was dried and concentrated in *vacuo.* To a solution of the resultant product (0.91 g, 2.2 mmol) in CH₂Cl₂ at 22°C, was added CICOCOCI (0.29 ml, 3.3 mmol) and the mixture stirred for 16 h. The solvent was removed in *vacuo.*

Step 5): To an efficiently stirred suspension of 4-fluorophenylzinc chloride (4.4 mmol) prepared from 4-fluorophenylmagnesium bromide (1 M in THF, 4.4 ml, 4.4 mmol) and ZnCl₂ (0.6 g, 4.4 mmol) at 4°C, was added tetrakis(triphenylphosphine)palladium (0.25 g, 0.21 mmol) followed by the product of Step 4 (0.94 g, 2.2 mmol) as a solution in THF (2 ml). The reaction was stirred for 1 h at 0°C and then for 0.5 h at 22°C. HCl (1N, 5 ml) was added and the mixture was extracted with EtOAc. The organic layer was concentrated to an oil and purified by silica gel chromatography to obtain 1-(4-fluorophenyl)-4(S)-(4-hydroxyphenyl)-3(R)-(3-oxo-3-phenylpropyl)-2-azetidinone:
HRMS calc'd for C₂₄H₁₉F₂NO₃ = 408.1429, found 408.1411.

Step 6): To the product of Step 5 (0.95 g, 1.91 mmol) in THF (3 ml), was added (R)-tetrahydro-1-methyl-3,3-diphenyl-1 H,3H-pyrrolo-[1,2-c][1,3,2] oxazaborole (120 mg, 0.43 mmol) and the mixture was cooled to -20°C. After 5 min, borohydride-dimethylsulfide complex (2M in THF, 0.85 ml, 1.7 mmol) was added dropwise over 0.5 h. After a total of 1.5 h , CH₃OH was added followed by HCl (1 N) and the reaction mixture was extracted with EtOAc to obtain 1-(4-fluorophenyl)-3(R)-[3(S)-(4-fluorophenyl)-3-hydroxypropyl)]-4(S)-[4-(phenylmethoxy)phenyl]-2-azetidinone (compound 6A-1) as an oil. ¹ H in CDCl₃ d H3 = 4.68. J = 2.3 Hz. Cl (M⁺H) 500.

Use of (S)-tetra-hydro-1-methyl-3,3-diphenyl-1 H,3H-pyrrolo-[1,2-c][1,3,2] oxazaborole gives the corresponding 3(R)-hydroxypropyl azetidinone (compound 6B-1). ¹ H in CDCl₃ d H3 = 4.69. J = 2.3 Hz. Cl (M⁺H) 500.

To a solution of compound 6A-1 (0.4 g, 0.8 mmol) in ethanol (2 ml), was added 10% Pd/C (0.03 g) and the reaction mixture was stirred under a pressure (60 psi) of H₂ gas for 16 h. The reaction mixture was filtered and the solvent was concentrated to obtain compound 6A. Mp 164-166°C; Cl (M⁺H) 410. [α]²⁵_{D} = -28.1° (c 3, CH₃OH). Elemental analysis calc'd for C₂₄H₂₁ F₂NO₃: C 70.41; H 5.17; N 3.42; found C 70.25; H 5.19; N 3.54.

Similarly treat compound 6B-1 to obtain compound 6B. Mp 129.5-132.5°C; Cl (M⁺H) 410. Elemental analysis calc'd for C₂₄H₂₁F₂NO₃: C 70.41; H 5.17; N 3.42; found C 70.30; H 5.14; N 3.52.

Step 6' (Alternative): To a solution of the product of Step 5 (0.14 g, 0.3 mmol) in ethanol (2 ml), was added 10% Pd/C (0.03 g) and the reaction was stirred under a pressure (60 psi) of H₂ gas for 16 h. The reaction mixture was filtered and the solvent was concentrated to afford a 1:1 mixture of compounds 6A and 6B.

It will be appreciated by those skilled in the art that changes could be made to the embodiments described above without departing from the broad inventive concept thereof. It is understood, therefore, that this invention is not limited to the particular embodiments disclosed, but it is intended to cover modifications which are within the spirit and scope of the invention, as defined by the appended claims.

## Claims

1. A composition comprising:
(a) at least one sterol absorption inhibitor or pharmaceutically acceptable salt or solvate thereof or prodrug of the at least one sterol absorption inhibitor or of the salt or solvate thereof; and
(b) at least one blood modifier for vascular conditions which is different from component (a) above.

2. The composition according to claim 1, wherein the at least one sterol absorption inhibitor is represented by Formula (I): 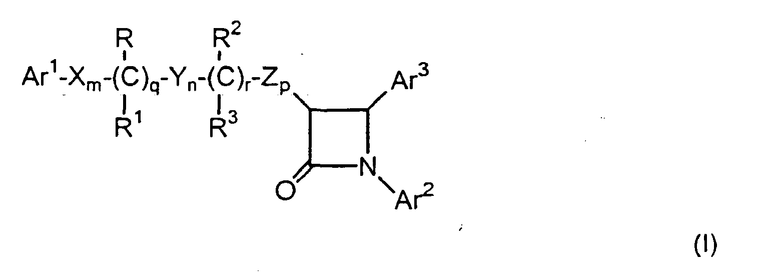 or isomers thereof, or pharmaceutically acceptable salts or solvates of the compounds of Formula (I) or of the isomers thereof, or prodrugs of the compounds of Formula (I) or of the isomers, salts or solvates thereof,
wherein:
Ar¹ and Ar² are independently selected from the group consisting of aryl and R⁴-substituted aryl;
Ar³ is aryl or R⁵-substituted aryl;
X, Y and Z are independently selected from the group consisting of -CH₂-, -CH(lower alkyl)- and -C(dilower alkyl)-;
R and R² are independently selected from the group consisting of -OR⁶, -O(CO)R⁶, -O(CO)OR⁹ and -O(CO)NR⁶R⁷;
R¹ and R³ are independently selected from the group consisting of hydrogen, lower alkyl and aryl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
R⁴ is 1-5 substituents independently selected from the group consisting of lower alkyl, -OR⁶, -O(CO)R⁶, -O(CO)OR⁹, -O(CH₂)₁₋₅OR⁶, -O(CO)NR⁶R⁷. -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶, -SO₂NR⁶R⁷, S(O)₀₋₂R⁹, -O(CH²)₁₋₁₀-COOR⁶, -O(CH₂)₁₋₁₀CONR⁶R⁷, -(lower alkylene)COOR⁶, -CH=CH-COOR⁶, -CF₃, -CN, -NO₂ and halogen;
R⁵ is 1-5 substituents independently selected from the group consisting of -OR⁶, -O(CO)R⁶, -O(CO)OR⁹, -O(CH₂)₁₋₅R⁶, -O(CO)NR⁶R⁷, -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶, -SO₂NR⁶R⁷, S(O)₀₋₂R⁹, -O(CH₂)₁₋₁₀-COOR⁶, -O(CH₂)₁₋₁₀CONR⁶R⁷, -(lower alkylene)COOR⁶ and -CH=CH-COOR⁶;
R⁶, R⁷ and R⁸ are independently selected from the group consisting of hydrogen, lower alkyl, aryl and aryl-substituted lower alkyl; and
R⁹ is lower alkyl, aryl or aryl-substituted lower alkyl.

3. The composition according to claim 2, wherein the sterol absorption inhibitor is represented by Formula (II) below: 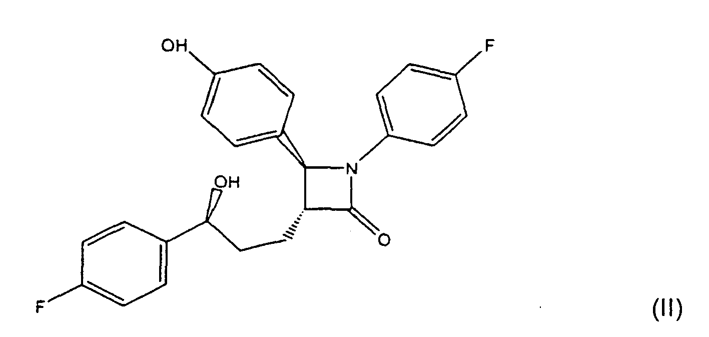 or pharmaceutically acceptable salts or solvates thereof, or prodrugs of the compound of Formula (II) or of the salts or solvates thereof.

4. The composition according to claim 1, wherein the at least one sterol absorption inhibitor is represented by Formula (III): 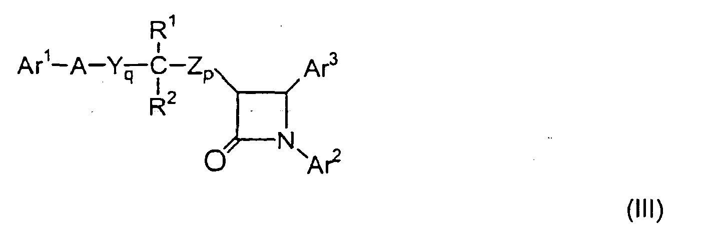 or isomers thereof, or pharmaceutically acceptable salts or solvates of the compounds of Formula (III) or of the isomers thereof, or prodrugs of the compounds of Formula (III) or of the isomers, salts or solvates thereof, wherein, in Formula (III) above:
Ar¹ is R³-substituted aryl;
Ar² is R⁴-substituted aryl;
Ar³ is R⁵-substituted aryl;
Y and Z are independently selected from the group consisting of -CH₂-, -CH(lower alkyl)- and -C(dilower alkyl)-;
A is selected from -O-, -S-, -S(O)- or -S(O)₂-;
R¹ is selected from the group consisting of -OR⁶, -O(CO)R ⁶, -O(CO)OR⁹ and -O(CO)NR⁶R⁷; R² is selected from the group consisting of hydrogen, lower alkyl and aryl; or R¹ and R² together are =O;
q is 1, 2 or 3;
pis 0, 1, 2, 3 or 4;
R⁵ is 1-3 substituents independently selected from the group consisting of -OR⁶, -O(CO)R⁶, -O(CO)OR⁹, -O(CH₂)₁₋₅OR⁹, -O(CO)NR⁶R⁷, -NR⁶R⁷, -NR⁶(CO)R⁷, -NR⁶(CO)OR⁹, -NR⁶(CO)NR⁷R⁸, -NR⁶SO₂-lower alkyl, -NR⁶SO₂-aryl, -CONR⁶R⁷,-COR ⁶ -SO₂NR⁶R⁷, S(O)₀₋₂-alkyl, S(O)₀₋₂-aryl, -O(CH₂)₁₋₁₀-COOR⁶, O(CH₂)₁₋₁₀CONR⁶R⁷, o-halogeno, m-halogeno, o-lower alkyl, m-lower alkyl, -(lower alkylene)-COOR ⁶ and
-CH=CH-COOR⁶;
R³ and R⁴ are independently 1-3 substituents independently selected from the group consisting of R⁵, hydrogen, p-lower alkyl, aryl, -NO₂, -CF₃ and p-halogeno;
R⁶, R⁷ and R⁸ are independently selected from the group consisting of hydrogen, lower alkyl, aryl and aryl-substituted lower alkyl; and
R⁹ is lower alkyl, aryl or aryl-substituted lower alkyl.

5. The composition according to claim 1, wherein the at least one sterol absorption inhibitor is represented by Formula (IV): 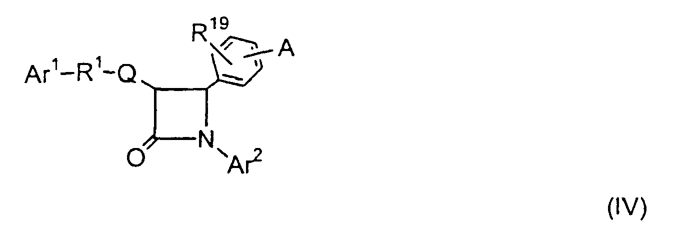 or isomers thereof, or pharmaceutically acceptable salts or solvates of the compounds of Formula (IV) or of the isomers thereof, or prodrugs of the compounds of Formula (IV) or of the isomers, salts or solvates thereof, wherein, in Formula (IV) above:
A is selected from the group consisting of R²-substituted heterocycloalkyl, R²-substituted heteroaryl, R²-substituted benzofused heterocycloalkyl, and R²-substituted benzofused heteroaryl;
Ar¹ is aryl or R³-substituted aryl;
Ar² is aryl or R⁴-substituted aryl;
Q is a bond or, with the 3-position ring carbon of the azetidinone, forms the spiro group 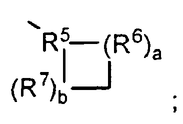 and
R¹ is selected from the group consisting of:
-(CH₂)q-, wherein q is 2-6, provided that when Q forms a spiro ring, q can also be zero or 1;
-(CH₂)ₑ-G-(CH₂)ᵣ-_{'} wherein G is -O-, -C(O)-, phenylene, -NR⁸- or -S(O)₀₋₂-, e is 0-5 and r is 0-5, provided that the sum of e and r is 1-6;
-(C₂-C₆ alkenylene)-; and
-(CH₂)_{f}-V-(CH₂)_{g}-, wherein V is C₃-C₆ cycloalkylene, f is 1-5 and g is 0-5, provided that the sum of f and g is 1-6;
R⁵ is selected from: 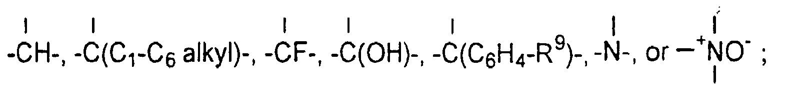
R⁶ and R⁷ are independently selected from the group consisting of -CH₂-, -CH(C₁-C₆ alkyl)-, -C(di-(C₁-C₆) alkyl), -CH=CH- and -C(C₁-C₆ alkyl)=CH-; or R⁵ together with an adjacent R⁶, or R⁵ together with an adjacent R⁷, form a -CH=CH- or a -CH=C(C₁-C₆ alkyl)- group;
a and b are independently 0, 1, 2 or 3, provided both are not zero; provided that when R⁶ is -CH=CH- or -C(C₁-C₆ alkyl)=CH-, a is 1; provided that when R⁷ is -CH=CH- or -C(C₁-C₆ alkyl)=CH-, b is 1; provided that when a is 2 or 3, the R⁶'s can be the same or different; and provided that when b is 2 or 3, the R⁷'s can be the same or different;
and when Q is a bond, R¹ also can be selected from: 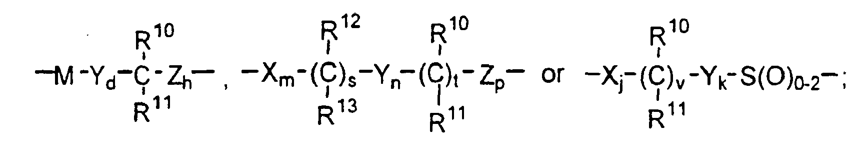 where M is -O-, -S-, -S(O)- or -S(O)₂-;
X, Y and Z are independently selected from the group consisting of -CH₂-, -CH(C₁-C₆ alkyl)- and -C(di-(C₁-C₆) alkyl);
R¹⁰ and R¹² are independently selected from the group consisting of -OR¹⁴, -O(CO)R¹⁴, -O(CO)OR¹⁶ and -O(CO)NR¹⁴R¹⁵;
R¹¹ and R¹³ are independently selected from the group consisting of hydrogen, (C₁-C₆)alkyl and aryl; or R¹⁰ and R¹¹ together are =O, or R¹² and R¹³ together are =O;
d is 1, 2 or 3;
h is 0, 1, 2, 3 or 4;
s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
R² is 1-3 substituents on the ring carbon atoms selected from the group consisting of hydrogen, (C₁-C₁₀)alkyl, (C₂-C₁₀)alkenyl, (C₂-C₁₀)alkynyl, (C₃-C₆)cycloalkyl, (C₃-C₆)cycloalkenyl, R¹⁷-substituted aryl, R¹⁷-substituted benzyl, R¹⁷-substituted benzyloxy, R¹⁷-substituted aryloxy, halogeno, -NR¹⁴R¹⁵, NR¹⁴ R¹⁵(C₁-C₆ alkylene)-, NR¹⁴R¹⁵C(O)(C₁-C₆alkylene)-, -NHC(O)R¹⁶, OH, C₁-C₆ alkoxy, -OC(O)R¹⁶, -COR¹⁴, hydroxy(C₁-C₆)alkyl, (C₁-C₆)alkoxy(C₁-C₆)alkyl, NO₂, -S(O)₀₋₂R¹⁶, -SO₂NR¹⁴R¹⁵ and -(C₁-C₆ alkylene)COOR¹⁴; when R² is a substituent on a heterocycloalkyl ring, R² is as defined, or is =O or 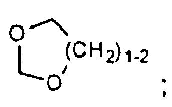 and, where R² is a substituent on a substitutable ring nitrogen, it is hydrogen, (C₁-C₆)alkyl, aryl, (C₁-C₆)alkoxy, aryloxy, (C-C₆)alkylcarbonyl, arylcarbonyl, hydroxy, -(CH₂)₁₋₆CONR¹⁸R¹⁸, 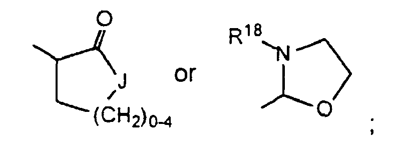 wherein J is -O-, -NH-, -NR¹⁸- or -CH₂-;
R³ and R⁴ are independently selected from the group consisting of 1-3 substituents independently selected from the group consisting of (C₁-C₆)alkyl, -OR¹⁴, -O(CO)R¹⁴, -O(CO)OR¹⁶, -O(CH₂)₁₋₅OR¹⁴, -O(CO)NR¹⁴R¹⁵, -NR¹⁴R¹⁵, -NR¹⁴(CO)R¹⁵, -NR¹⁴ (CO)OR¹⁶, -NR¹⁴(CO)NR¹⁵R¹⁹, -NR¹⁴SO₂R¹⁶, -COOR¹⁴, -CONR¹⁴R¹⁵, -COR¹⁴, -SO₂NR¹⁴R¹⁵, S(O)₀₋₂R¹⁶, -O(CH₂)₁₋₁₀-COOR¹⁴, -O(CH)₁₋₁₀CONR¹⁴R¹⁵, -(C₁-C₆ alkylene)-COOR¹⁴, -CH=CH-COOR¹⁴, -CF₃, -CN, - NO₂ and halogen;
R⁸ is hydrogen, (C₁-C₆)alkyl, aryl (C₁-C₆)alkyl, -C(O)R ¹⁴ or -COOR¹⁴;
R⁹ and R¹⁷ are independently 1-3 groups independently selected from the group consisting of hydrogen, (C₁-C₆)alkyl, (C₁-C₆)alkoxy, -COOH, NO₂, -NR¹⁴R¹⁵, OH and halogeno;
R¹⁴ and R¹⁵ are independently selected from the group consisting of hydrogen, (C₁-C₆)alkyl, aryl and aryl-substituted (C₁-C₆)alkyl;
R¹⁶ is (C₁-C₆)alkyl, aryl or R¹⁷ -substituted aryl;
R¹⁸ is hydrogen or (C₁-C₆)alkyl; and
R¹⁹ is hydrogen, hydroxy or (C₁-C₆)alkoxy.

6. The composition according to claim 1, wherein the at least one sterol absorption inhibitor is represented by Formula (V): 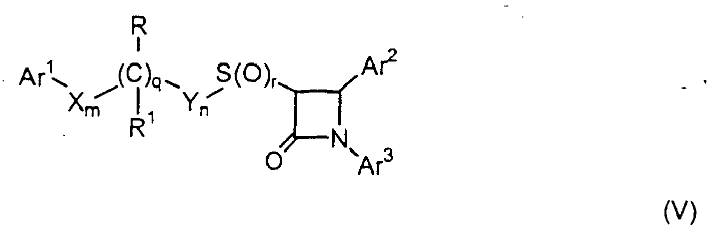 or isomers thereof, or pharmaceutically acceptable salts or solvates of the compounds of Formula (V) or of the isomers thereof, or prodrugs of the compounds of Formula (V) or of the isomers, salts or solvates thereof, wherein, in Formula (V) above:
Ar¹ is aryl, R¹⁰-substituted aryl or heteroaryl;
Ar² is aryl or R⁴-substituted aryl;
Ar³ is aryl or R⁵-substituted aryl;
X and Y are independently selected from the group consisting of -CH₂-, -CH(lower alkyl)- and -C(dilower alkyl)-;
R is -OR⁶, -O(CO)R⁶, -O(CO)OR⁹ or -O(CO)NR⁶R⁷; R¹ is hydrogen, lower alkyl or aryl; or R and R¹ together are =O;
q is 0 or 1;
r is 0, 1 or 2;
m and n are independently 0, 1, 2, 3, 4 or 5; provided that the sum of m, n and q is 1, 2, 3, 4 or 5;
R⁴ is 1-5 substituents independently selected from the group consisting of lower alkyl, -OR⁶, -O(CO)R⁶, -O(CO)OR⁹, -O(CH₂)₁₋₅OR⁶, -O(CO)NR⁶R⁷, -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶, -SO₂NR⁶R⁷, S(O)₀₋₂R⁹, -O(CH₂)₁₋₁₀-COOR⁶ , -O(CH₂)₁₋₁₀CONR⁶R⁷, -(lower alkylene)COOR⁶ and -CH=CH-COOR⁶;
R⁵ is 1-5 substituents independently selected from the group consisting of -OR⁶, -O(CO)R⁶, -O(CO)OR⁹, -O(CH₂)₁₋₅OR⁶, -O(CO)NR⁶R⁷, NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶,-SO₂NR⁶R⁷, S(O)₀₋₂R⁹, -O(CH₂)₁₋₁₀-COOR⁶, -O(CH₂)₁₋₁₀CONR⁶R⁷, -CF₃, -CN, -NO₂, halogen,
-(lower alkylene)COOR⁶ and -CH=CH-COOR⁶;
R⁶, R⁷ and R⁸ are independently selected from the group consisting of hydrogen, lower alkyl, aryl and aryl-substituted lower alkyl;
R⁹ is lower alkyl, aryl or aryl-substituted lower alkyl; and
R¹⁰ is 1-5 substituents independently selected from the group consisting of lower alkyl, -OR⁶, -O(CO)R⁶, -O(CO)OR⁹, -O(CH₂)₁₋₅OR⁶, -O(CO)NR⁶R⁷, -NR⁶R⁷, -NR⁶(CO)R⁷, -NR⁶(CO)OR⁹, -NR⁶(CO)NR⁷R⁸, -NR⁶SO₂R⁹, -COOR⁶, -CONR⁶R⁷, -COR⁶, -SO₂NR⁶R⁷, -S(O)₀₋₂R⁹, -O(CH₂)₁₋₁₀-COOR⁶,
-O(CH₂)₁₋₁₀CONR⁶R⁷, -CF₃, -CN, -NO₂ and halogen.

7. The composition according to claim 1, where the at least one sterol absorption inhibitor is represented by Formula (VI): 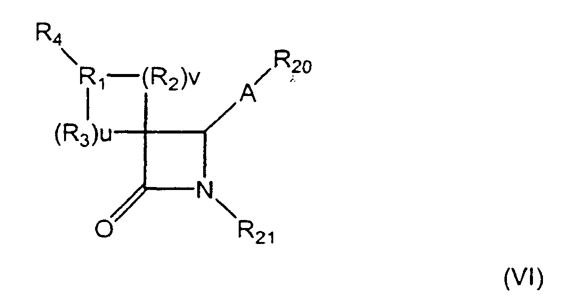 or isomers thereof, or pharmaceutically acceptable salts or solvates of the compounds of Formula (VI) or of the isomers thereof, or prodrugs of the compounds of Formula (VI) or of the isomers, salts or solvates thereof, wherein in Formula (VI) above:
R₁ is 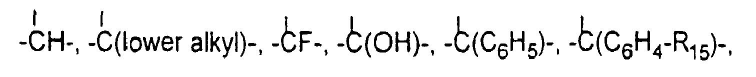 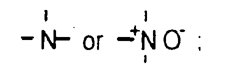
R₂ and R₃ are independently selected from the group consisting of: -CH₂-, -CH(lower alkyl)-, -C(di-lower alkyl)-, -CH=CH- and -C(lower alkyl)=CH-; or R₁ together with an adjacent R₂, or R₁ together with an adjacent R₃, form a -CH=CH- or a -CH=C(lower alkyl)- group;
u and v are independently 0, 1, 2 or 3, provided both are not zero; provided that when R₂ is -CH=CH- or -C(lower alkyl)=CH-, v is 1; provided that when R₃ is -CH=CH- or -C(lower alkyl)=CH-, u is 1; provided that when v is 2 or 3, the R₂'s can be the same or different; and provided that when u is 2 or 3, the R₃'s can be the same or different;
R₄ is selected from B-(CH₂)ₘC(O)-, wherein m is 0, 1, 2, 3, 4 or 5;
B-(CH₂)q-, wherein q is 0, 1, 2, 3, 4, 5 or 6;
B-(CH₂)ₑ-Z-(CH₂)ᵣ-, wherein Z is -O-, -C(O)-, phenylene, -N(R₈)- or -S(O)₀₋₂-, e is 0, 1, 2, 3, 4 or 5 and r is 0, 1, 2, 3, 4 or 5, provided that the sum of e and r is 0, 1, 2, 3, 4, 5 or 6;
B-(C₂-C₆ alkenylene)-;
B-(C₄-C₆ alkadienylene)-;
B-(CH₂)ₜ-Z-(C₂-C₆ alkenylene)-, wherein Z is as defined above, and wherein t is 0, 1, 2 or 3, provided that the sum of t and the number of carbon atoms in the alkenylene chain is 2, 3, 4, 5 or 6;
B-(CH₂)_{f}-V-(CH₂)_{g}-, wherein V is C₃-C₆ cycloalkylene, f is 1, 2, 3, 4 or 5 and g is 0, 1, 2, 3, 4 or 5, provided that the sum of f and g is 1, 2, 3, 4, 5 or 6;
B-(CH₂)ₜ-V-(C₂-C₆ alkenylene)- or
B-(C₂-C₆ alkenylene)-V-(CH₂)ₜ-, wherein V and t are as defined above, provided that the sum of t and the number of carbon atoms in the alkenylene chain is 2, 3, 4, 5 or 6; B-(CH₂)ₐ-Z-(CH₂)_{b}-V-(CH₂)_{d}-, wherein Z and V are as defined above and a, b and d are independently 0, 1, 2, 3, 4, 5 or 6, provided that the sum of a, b and d is 0, 1, 2, 3, 4, 5 or 6; or T-(CH₂)ₛ-, wherein T is cycloalkyl of 3-6 carbon atoms and s is 0, 1, 2, 3, 4, 5 or 6; or
R₁ and R₄ together form the group B-CH=C- ;
B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or 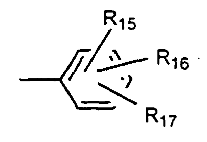
W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, -CF₃, -OCF₃, benzyl, R₇-benzyl, benzyloxy, R₇-benzyloxy, phenoxy, R₇-phenoxy, dioxolanyl, NO₂,-N(R₈)(R₉), N(R₈)(R₉)-lower alkylene-, N(R₈)(R₉)-lower alkylenyloxy-, OH, halogeno, -CN, -N₃, -NHC(O)OR₁₀, -NHC(O)R₁₀, R₁₁O₂SNH-, (R₁₁O₂S)₂N-, -S(O)2NH₂, -S(O)₀₋₂R⁸, tert-butyldimethyl-silyloxymethyl, -C(O)R₁₂, -COOR₁₉, -CON(R₈)(R₉), -CH=CHC(O)R₁₂, -lower alkylene-C(O)R₁₂, R₁₀C(O)(lower alkylenyloxy)-, N(R₈)(R₉)C(O)(lower alkylenyloxy)- and 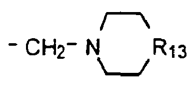 for substitution on ring carbon atoms, and the substituents on the substituted heteroaryl ring nitrogen atoms, when present, are selected from the group consisting of lower alkyl, lower alkoxy, -C(O)OR₁₀, -C(O)R₁₀, OH, N(R₈)(R₉)-lower alkylene-,N(R₈)(R₉)-lower alkylenyloxy-, -S(O)₂NH₂ and 2-(trimethylsilyl)-ethoxymethyl;
R₇ is 1-3 groups independently selected from the group consisting of lower alkyl, lower alkoxy, -COOH, NO₂, -N(R₈)(R₉), OH, and halogeno;
R₈ and R₉ are independently selected from H or lower alkyl;
R₁₀ is selected from lower alkyl, phenyl, R₇-phenyl, benzyl or R₇-benzyl;
R₁₁ is selected from OH, lower alkyl, phenyl, benzyl, R₇-phenyl or R₇-benzyl;
R₁₂ is selected from H, OH, alkoxy, phenoxy, benzyloxy, 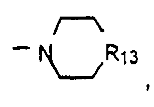 -N(R₈)(R₉), lower alkyl, phenyl or R₇-phenyl;
R₁₃ is selected from -O-, -CH₂-, -NH-, -N(lower alkyl)- or -NC(O)R₁₉;
R₁₅, R₁₆ and R₁₇ are independently selected from the group consisting of H and the groups defined for W; or R₁₅ is hydrogen and R₁₆ and R₁₇, together with adjacent carbon atoms to which they are attached, form a dioxolanyl ring;
R₁₉ is H, lower alkyl, phenyl or phenyl lower alkyl; and
R₂₀ and R₂₁ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above.

8. The composition according to claim 1, wherein the at least one sterol absorption inhibitor is represented by Formula (VIIA) or (VIIB): 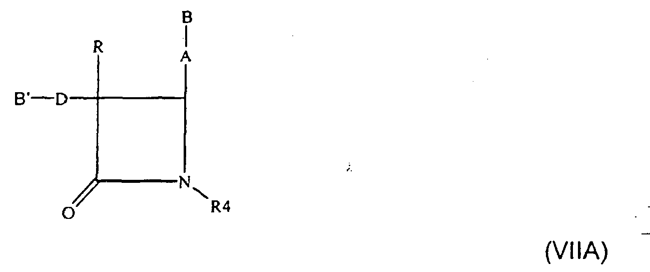 or 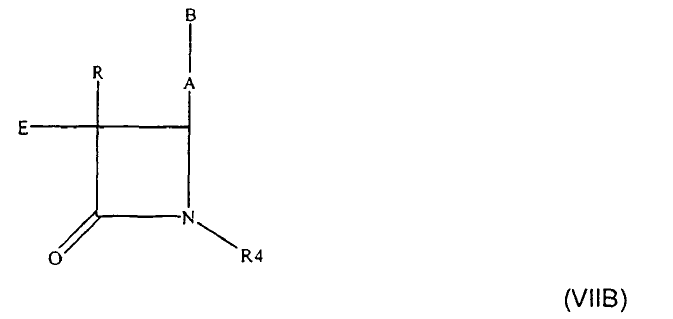 or isomers thereof, or pharmaceutically acceptable salts or solvates of the compounds of Formulae (VIIA) or (VIIB) or of the isomers thereof, or prodrugs of the compounds of Formulae (VIIA) or (VIIB) or of the isomers, salts or solvates thereof, wherein in Formulae (VIIA) and (VIIB) above:
A is -CH=CH-, -C≡C- or -(CH₂)p- wherein p is 0, 1 or 2;
B is 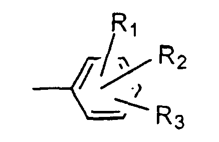
B' is 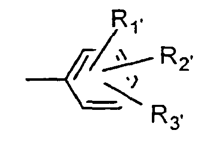
D is -(CH₂)ₘC(O)- or -(CH₂)q- wherein m is 1, 2, 3 or 4 and q is 2, 3 or 4;
E is C₁₀ to C₂₀ alkyl or -C(O)-(C₉ to C₁₉)-alkyl, wherein the alkyl is straight or branched, saturated or containing one or more double bonds;
R is hydrogen, C₁-C₁₅ alkyl, straight or branched, saturated or containing one or more double bonds, or B-(CH₂)ᵣ -, wherein r is 0, 1, 2, or 3;
R₁, R₂, R₃, R_{1'}, R_{2'}, and R_{3'} are independently selected from the group consisting of hydrogen, lower alkyl, lower alkoxy, carboxy, NO₂, NH₂, OH, halogeno, lower alkylamino, dilower alkylamino, -NHC(O)OR₅, R₆O₂SNH- and -S(O)₂NH₂;
R₄ is 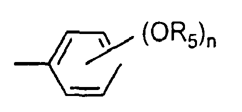 wherein n is 0, 1, 2 or 3;
R₅ is lower alkyl; and
R₆ is OH, lower alkyl, phenyl, benzyl or substituted phenyl wherein the substituents are 1-3 groups independently selected from the group consisting of lower alkyl, lower alkoxy, carboxy, NO₂, NH₂, OH, halogeno, lower alkylamino and dilower alkylamino.

9. The composition according to claim 1, wherein the at least one sterol absorption inhibitor is represented by Formula (VIII): 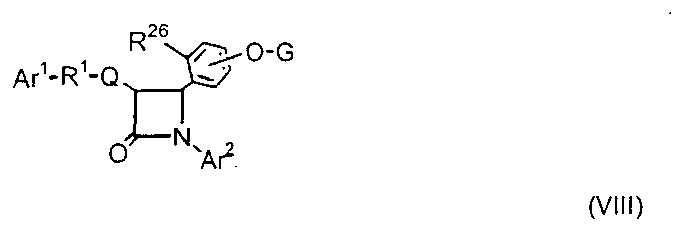 or isomers thereof, or pharmaceutically acceptable salts or solvates of the compounds of Formula (VIII) or of the isomers thereof, or prodrugs of the compounds of Formula (VIII) or of the isomers, salts or solvates thereof, wherein, in Formula (VIII) above,
R²⁶ is H or OG¹;
G and G¹ are independently selected from the group consisting of 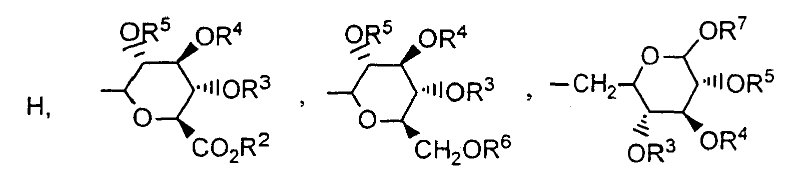 and 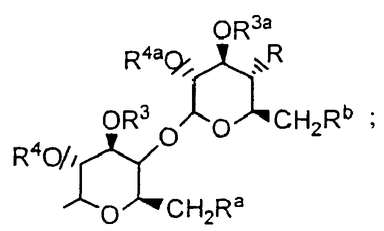 provided that when R²⁶ is H or OH, G is not H;
R, R^{a} and R^{b} are independently selected from the group consisting of H, -OH, halogeno, -NH₂, azido, (C₁-C₆)alkoxy(C₁-C₆)-alkoxy or -W-R³⁰;
W is independently selected from the group consisting of -NH-C(O)-, -O-C(O)-, -O-C(O)-N(R³¹)-, -NH-C(O)-N(R³¹)- and -O-C(S)-N(R³¹)-;
R² and R⁶ are independently selected from the group consisting of H, (C₁-C₆)alkyl, aryl and aryl(C₁-C₆)alkyl;
R³, R⁴, R⁵, R⁷, R^{3a} and R^{4a} are independently selected from the group consisting of H, (C₁-C₆)alkyl, aryl(C₁-C₆)alkyl, -C(O)(C₁-C₆)alkyl and -C(O)aryl;
R³⁰ is selected from the group consisting of R³²-substituted T, R³²-substituted-T-(C₁-C₆)alkyl, R³²-substituted-(C₂-C₄)alkenyl, R³²-substituted-(C₁-C₆)alkyl, R³²-substituted-(C₃-C₇)cycloalkyl and R³²-substituted-(C₃-C7)cycloalkyl(C₁-C₆)alkyl;
R³¹ is selected from the group consisting of H and (C₁-C₄)alkyl;
T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;
R32 is independently selected from 1-3 substituents independently selected from the group consisting of halogeno, (C₁-C₄)alkyl, -OH, phenoxy, -CF₃, -NO₂, (C₁-C₄)alkoxy, methylenedioxy, oxo, (C₁-C₄)alkylsulfanyl, (C₁-C₄)alkylsulfinyl, (C₁-C₄)alkylsulfonyl, -N(CH₃)₂, -C(O)-NH(C₁-C₄)alkyl, -C(O)-N((C₁-C₄)alkyl)₂, -C(O)-(C₁-C₄)alkyl, -C(O)-(C₁-C₄)alkoxy and pyrrolidinylcarbonyl; or R³² is a covalent bond and R³¹, the nitrogen to which it is attached and R³² form a pyrrolidinyl, piperidinyl, N-methyl-piperazinyl, indolinyl or morpholinyl group, or a (C₁-C₄)alkoxycarbonyl-substituted pyrrolidinyl, piperidinyl, N-methylpiperazinyl, indolinyl or morpholinyl group;
Ar¹ is aryl or R¹⁰-substituted aryl;
Ar² is aryl or R¹¹ -substituted aryl;
Q is a bond or, with the 3-position ring carbon of the azetidinone, forms the spiro group 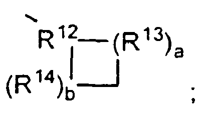 and
R¹ is selected from the group consisting of
-(CH₂)q-, wherein q is 2-6, provided that when Q forms a spiro ring, q can also be zero or 1;
-(CH₂)ₑ-E-(CH₂)ᵣ-, wherein E is -O-, -C(O)-, phenylene, -NR²²- or -S(O)₀₋₂-, e is 0-5 and r is 0-5, provided that the sum of e and r is 1-6;
-(C₂-C₆)alkenylene-; and
-(CH₂)_{f}-V-(CH₂)_{g}-, wherein V is C₃-C₆ cycloalkylene, f is 1-5 and g is 0-5, provided that the sum of f and g is 1-6;
R¹² is 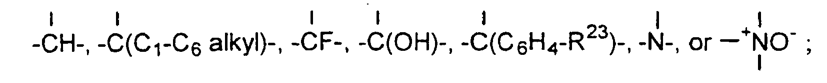
R¹³ and R¹⁴ are independently selected from the group consisting of -CH₂-, -CH(C₁-C₆ alkyl)-, -C(di-(C₁-C₆) alkyl), -CH=CH- and
-C(C₁-C₆ alkyl)=CH-; or R¹² together with an adjacent R¹³, or R¹² together with an adjacent R¹⁴, form a -CH=CH- or a -CH=C(C₁-C₆ alkyl)- group;
a and b are independently 0,1, 2 or 3, provided both are not zero;
provided that when R¹³ is -CH=CH- or -C(C₁-C₆ alkyl)=CH-, a is 1;
provided that when R¹⁴ is -CH=CH- or -C(C₁-C₆ alkyl)=CH-, b is 1;
provided that when a is 2 or 3, the R¹³'s can be the same or different; and
provided that when b is 2 or 3, the R¹⁴'s can be the same or different;
and when Q is a bond, R¹ also can be: 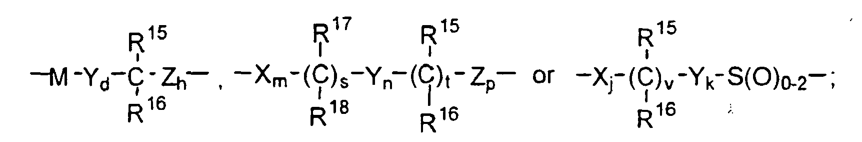
M is -O-, -S-, -S(O)- or -S(O)₂-;
X, Y and Z are independently selected from the group consisting of -CH₂-, -CH(C₁-C₆)alkyl- and -C(di-(C₁-C₆)alkyl);
R¹⁰ and R¹¹ are independently selected from the group consisting of 1-3 substituents independently selected from the group consisting of (C₁-C₆)alkyl, -OR¹⁹, -O(CO)R¹⁹, -O(CO)OR²¹, -O(CH₂)₁₋₅OR¹⁹, -O(CO)NR¹⁹R²⁰, -NR¹⁹R²⁰, -NR¹⁹(CO)R²⁰, -NR¹⁹(CO)OR²¹, -NR¹⁹(CO)NR²⁰R²⁵, -NR¹⁹SO₂R²¹, -COOR¹⁹, -CONR¹⁹R²⁰, -COR¹⁹, -SO₂NR¹⁹R²⁰, S(O)₀₋₂R²¹, -O(CH₂)₁₋₁₀-COOR¹⁹, -O(CH₂)₁₋₁₀CONR¹⁹R²⁰, -(C₁-C₆ alkylene)-COOR¹⁹, -CH=CH-COOR¹⁹, -CF₃, -CN, -NO₂ and halogen;
R¹⁵ and R¹⁷ are independently selected from the group consisting of -OR¹⁹, -O(CO)R¹⁹, -O(CO)OR²¹ and -O(CO)NR¹⁹R²⁰;
R¹⁶ and R¹⁸ are independently selected from the group consisting of H, (C₁-C₆)alkyl and aryl; or R¹⁵ and R¹⁶ together are =O, or R¹⁷ and R¹⁸ together are =O;
d is 1, 2 or 3;
h is 0, 1, 2, 3 or 4;
s is 0 or 1; t is 0 or 1; m, n and p are independently 0-4;
provided that at least one of s and t is 1, and the sum of m, n, p, s and t is 1-6;
provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5; and when Q is a bond and R¹ is 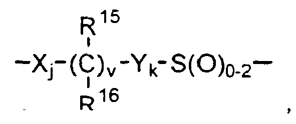 Ar¹ can also be pyridyl, isoxazolyl, furanyl, pyrrolyl, thienyl, imidazolyl, pyrazolyl, thiazolyl, pyrazinyl, pyrimidinyl or pyridazinyl;
R¹⁹ and R²⁰ are independently selected from the group consisting of H, (C₁-C₆)alkyl, aryl and aryl-substituted (C₁-C₆)alkyl;
R²¹ is (C₁-C₆)alkyl, aryl or R²⁴-substituted aryl;
R²² is H, (C₁-C₆)alkyl, aryl (C₁-C₆)alkyl, -C(O)R¹⁹ or -COOR¹⁹;
R²³ and R²⁴ are independently 1-3 groups independently selected from the group consisting of H, (C₁-C₆)alkyl, (C₁-C₆)alkoxy, -COOH, NO₂,
-NR¹⁹R²⁰, -OH and halogeno; and
R²⁵ is H, -OH or (C₁-C₆)alkoxy.

10. The composition according to claim 1, wherein the at least one sterol absorption inhibitor is represented by Formula (IX): 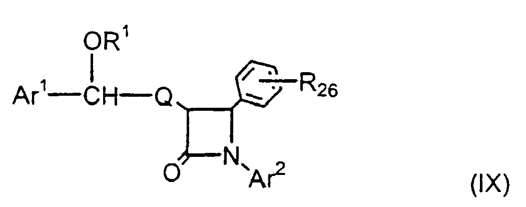 or isomers thereof, or pharmaceutically acceptable salts or solvates of the compounds of Formula (IX) or of the isomers thereof, or prodrugs of the compounds of Formula (IX) or of the isomers, salts or solvates thereof, wherein, in Formula (IX) above, R²⁶ is selected from the group consisting of:
a) OH;
b) OCH₃;
c) fluorine and
d) chlorine;
R¹ is selected from the group consisting of 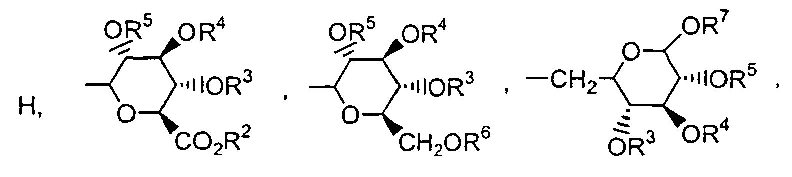 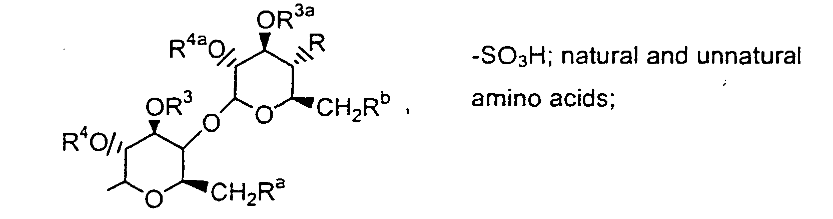 R, R^{a} and R^{b} are independently selected from the group consisting of H, -OH, halogeno, -NH₂, azido, (C₁-C₆)alkoxy(C₁-C₆)-alkoxy and -W-R30;
W is independently selected from the group consisting of -NH-C(O)-, -O-C(O)-, -O-C(O)-N(R³¹)-, -NH-C(O)-N(R³¹)- and -O-C(S)-N(R³¹)-;
R² and R⁶ are independently selected from the group consisting of H, (C₁-C₆)alkyl, aryl and aryl(C₁-C₆)alkyl;
R³, R⁴, R⁵, R⁷, R^{3a} and R^{4a} are independently selected from the group consisting of H, (C₁-C₆)alkyl, aryl(C₁-C₆)alkyl, -C(O)(C₁-C₆)alkyl and -C(O)aryl;
R³⁰ is independently selected form the group consisting of R32-substituted T, R³²-substituted-T-(C₁-C₆)alkyl, R³²-substituted-(C₂-C₄)alkenyl, R³²-substituted-(C₁-C₆)alkyl, R³²-substituted-(C₃-C7)cydoalkyl and R³²-substituted-(C₃-C₇)cycloalkyl(C₁-C₆)alkyl;
R³¹ is independently selected from the group consisting of H and (C₁-C₄)alkyl;
T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;
R³² is independently selected from 1-3 substituents independently selected from the group consisting of H, halogeno, (C₁-C₄)alkyl, -OH, phenoxy, -CF₃, -NO₂, (C₁-C₄)alkoxy, methylenedioxy, oxo, (C₁-C₄)alkylsulfanyl, (C₁-C₄)alkylsulfinyl, (C₁-C₄)alkylsulfonyl, -N(CH₃)₂, -C(O)-NH(C₁-C₄)alkyl, -C(O)-N((C₁-C₄)alkyl)₂, -C(O)-(C₁-C₄)alkyl, -C(O)-(C₁-C₄)alkoxy and pyrrolidinylcarbonyl; or R³² is a covalent bond and R³¹, the nitrogen to which it is attached and R³² form a pyrrolidinyl, piperidinyl, N-methyl-piperazinyl, indolinyl or morpholinyl group, or a (C₁-C₄)alkoxycarbonyl-substituted pyrrolidinyl, piperidinyl, N-methylpiperazinyl, indolinyl or morpholinyl group;
Ar¹ is aryl, R¹⁰-substituted aryl; pyridyl, isoxazolyl, furanyl, pyrrolyl, thienyl, imidazolyl, pyrazolyl, thiazolyl, pyrazinyl, pyrimidinyl or pyridazinyl;
Ar² is aryl or R¹¹-substituted aryl;
Q is -(CH₂)q-, wherein q is 2-6, or, with the 3-position ring carbon of the azetidinone,
forms the spiro group 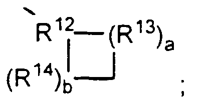
R¹² is 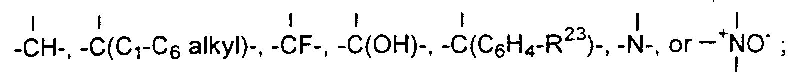
R¹³ and R¹⁴ are independently selected from the group consisting of -CH₂-, -CH(C₁-C₆ alkyl)-, -C(di-(C₁-C₆) alkyl), -CH=CH- and -C(C₁-C₆ alkyl)=CH-; or R¹² together with an adjacent R¹³, or R¹² together with an adjacent R¹⁴, form a -CH=CH- or a -CH=C(C₁-C₆ alkyl)- group;
a and b are independently 0, 1, 2 or 3, provided both are not zero; provided that when R¹³ is -CH=CH- or -C(C₁-C₆ alkyl)=CH-, a is 1; provided that when R¹⁴ is -CH=CH- or -C(C₁-C₆ alkyl)=CH-, b is 1; provided that when a is 2 or 3, the R¹³'s can be the same or different; and provided that when b is 2 or 3, the R¹⁴'s can be the same or different;
R¹⁰ and R¹¹ are independently selected from the group consisting of 1-3 substituents independently selected from the group consisting of (C₁-C₆)alkyl, -OR¹⁹, -O(CO)R¹⁹, -O(CO)OR²¹, -O(CH₂)₁₋₅OR¹⁹, -O(CO)NR¹⁹R²⁰, -NR¹⁹R²⁰, -NR¹⁹(CO)R²⁰, -NR¹⁹(CO)OR²¹, -NR¹⁹(CO)NR²⁰R²⁵, -NR¹⁹SO₂R²¹, -COOR¹⁹, -CONR¹⁹R²⁰, -COR¹⁹, -SO₂NR¹⁹R²⁰, -S(O)₀₋₂R²¹, -O(CH₂)₁₋₁₀-COOR¹⁹, -O(CH₂)₁₋₁₀CONR¹⁹R²⁰, -(C₁-C₆ alkylene)-COOR¹⁹, -CH=CH-COOR¹⁹, -CF₃, -CN, -NO₂ and halogen;
R19 and R²⁰ are independently selected from the group consisting of H, (C₁-C₆)alkyl, aryl and aryl-substituted (C₁-C₆)alkyl;
R²¹ is (C₁-C₆)alkyl, aryl or R²⁴-substituted aryl;
R²² is H, (C₁-C₆)alkyl, aryl (C₁-C₆)alkyl, -C(O)R¹⁹ or -COOR¹⁹;
R23 and R²⁴ are independently 1-3 groups independently selected from the group consisting of H, (C₁-C₆)alkyl, (C₁-C₆)alkoxy, -COOH, NO₂, -NR¹⁹R²⁰, -OH and halogeno; and
R²⁵ is H, -OH or (C₁-C₆)alkoxy.

11. The composition according to claim 1, wherein the at least one blood modifier is selected from the group consisting of anti-coagulants, antithrombotic agents, fibrinogen receptor antagonists, platelet inhibitors, platelet aggregation inhibitors, hemorrheologic agents, lipoprotein associated coagulation inhibitor, Factor VIIa inhibitors, Factor Xa inhibitors and combinations thereof.

12. The composition according to claim 11, wherein the at least one blood modifier is an anti-coagulant.

13. The composition according to claim 12, wherein the anti-coagulant is selected from the group consisting of argatroban, bivalirudin, dalteparin sodium, desirudin, dicumarol, lyapolate sodium, nafamostat mesylate, phenprocoumon, tinzaparin sodium, warfarin sodium and combinations thereof.

14. The composition according to claim 11, wherein the at least one blood modifier is an anti-thrombotic agent.

15. The composition according to claim 14, wherein the antithrombotic agent is selected from the group consisting of anagrelide hydrochloride, bivalirudin, cilostazol, dalteparin sodium, danaparoid sodium, dazoxiben hydrochloride, efegatran sulfate, enoxaparin sodium, fluretofen, ifetroban, ifetroban sodium, lamifiban, lotrafiban hydrochloride, napsagatran, orbofiban acetate, roxifiban acetate, sibrafiban, tinzaparin sodium, trifenagrel, abciximab, zolimomab aritox and combinations thereof.

16. The composition according to claim 11, wherein the at least one blood modifier is a fibrinogen receptor antagonist.

17. The composition according to claim 16, wherein the fibrinogen receptor antagonist is selected from the group consisting of roxifiban acetate, fradafiban, orbofiban, lotrafiban hydrochloride, tirofiban, xemilofiban, monoclonal antibody 7E3, sibrafiban and combinations thereof.

18. The composition according to claim 11, wherein the at least one blood modifier is a platelet inhibitor.

19. The composition according to claim 18, wherein the platelet inhibitor is selected from the group consisting of cilostazol, clopidogrel bisulfate, epoprostenol, epoprostenol sodium, ticlopidine hydrochloride, aspirin, ibuprofen, naproxen, sulindae, idomethacin, mefenamate, droxicam, diclofenac, sulfinpyrazone, piroxicam, dipyridamole and combinations thereof.

20. The composition according to claim 19, wherein the platelet inhibitor is aspirin.

21. The composition according to claim 11, wherein the at least one blood modifier is a platelet aggregation inhibitor.

22. The composition according to claim 21, wherein the platelet aggregation inhibitor is selected from the group consisting of acadesine, beraprost, beraprost sodium, ciprostene calcium, itazigrel, lifarizine, lotrafiban hydrochloride, orbofiban acetate, oxagrelate, fradafiban, orbofiban, tirofiban, xemilofiban and combinations thereof.

23. The composition according to claim 11, wherein the at least one blood modifier is a hemorrheologic agent.

24. The composition according to claim 23, wherein the hemorrheologic agent is pentoxifylline.

25. The composition according to claim 11, wherein the at least one blood modifier is a lipoprotein associated coagulation inhibitor.

26. The composition according to claim 11, wherein the at least one blood modifier is a Factor Xa inhibitor.

27. The composition according to claim 26, wherein the Factor Xa inhibitor is selected from the group consisting of disubstituted pyrazolines, disubstituted triazolines, substituted n-[(aminoiminomethyl)phenyl] propylamides, substituted n-[(aminomethyl)phenyl] propylamides, tissue factor pathway inhibitor (TFPI), low molecular weight heparins, heparinoids, benzimidazolines, benzoxazolinones, benzopiperazinones, indanones, dibasic (amidinoaryl) propanoic acid derivatives, amidinophenyl-pyrrolidines, amidinophenyl-pyrrolines, amidinophenyl-isoxazolidines, amidinoindoles, amidinoazoles, bis-arlysulfonylaminobenzamide derivatives, peptidic Factor Xa inhibitors and combinations thereof.

28. The composition according to claim 1, wherein the at least one blood modifier is a low molecular weight heparin.

29. The composition according to claim 28, wherein the low molecular weight heparin is selected from the group of enoxaparin, nardroparin, dalteparin, certroparin, parnaparin, reviparin, tinzaparin and combinations thereof.

30. The composition according to claim 1, wherein the at least one blood modifier is a heparinoid.

31. The composition according to claim 30, wherein the heparinoid is danaparoid.

32. The composition according to claim 11, wherein the at least one blood modifier is a Factor VIIa inhibitor.

33. The composition according to claim 32, wherein the Factor VIIa Inhibitor is selected from the group consisting of 4H-31-benzoxazin-4-ones, 4H-3,1-benzoxazin-4-thiones, quinazolin-4-ones, quinazolin-4-thiones, benzothiazin-4-ones, imidazolyl-boronic acid-derived peptide analogues TFPI-derived peptides and combinations thereof.

34. The composition according to claim 32, wherein the Factor VIIa Inhibitor is selected from the group consisting of naphthalene-2-sulfonic acid {1-[3-(aminoiminomethyl)-benzyl]-2-oxo-pyrrolidin-3-(S)-yl} amide trifluoroacetate, dibenzofuran-2-sulfonic acid {1-[3-(aminomethyl)-benzyl]-5-oxo-pyrrolidin-3-yl}-amide, tolulene-4-sulfonic acid {1-[3-(aminoiminomethyl)-benzyl]-2-oxo-pyrrolidin-3-(S)-yl}-amide trifluoroacetate, 3,4-dihydro-1 H-isoquinoline-2-sulfonic acid {1-[3-(aminoiminomethyl)-benzyl]-2-oxo-pyrrolin-3-(S)-yl}-amide trifluoroacetate and combinations thereof.

35. The composition according to claim 1, further comprising at least one cholesterol biosynthesis inhibitor.

36. The composition according to claim 35, wherein the at least one cholesterol biosynthesis inhibitor comprises at least one HMG CoA reductase inhibitor.

37. The composition according to claim 36, wherein the at least one HMG CoA reductase inhibitor is simvastatin.

38. The composition according to claim 1, further comprising at least one bile acid sequestrant.

39. The composition according to claim 1, further comprising at least one low-density lipoprotein receptor activator.

40. The composition according to claim 1, further comprising at least one Omega 3 fatty acid.

41. The composition according to claim 1, further comprising at least one natural water soluble fiber.

42. The composition according to claim 1, further comprising at least one antioxidant or vitamin.

43. The composition according to claim 1, wherein the at least one blood modifier is administered to a mammal in an amount ranging from about 1 to about 1000 milligrams of blood modifier per day.

44. The composition according to claim 1, wherein the at least one sterol absorption inhibitor is administered to a mammal in an amount ranging from about 0.1 to about 1000 milligrams of sterol absorption inhibitor per day.

45. A pharmaceutical composition for the treatment or prevention of vascular conditions, diabetes, obesity or lowering a concentration of a sterol in plasma of a mammal, comprising a therapeutically effective amount of the composition of claim 1 and a pharmaceutically acceptable carrier.

46. A method of treating or preventing vascular conditions, diabetes, obesity or lowering a concentration of a sterol in plasma of a mammal, comprising the step of administering to a mammal in need of such treatment:
(a) an effective amount of at least one sterol absorption inhibitor or pharmaceutically acceptable salt or solvate thereof or prodrug of the at least one sterol absorption inhibitor or of the salt or solvate thereof; and
(b) an effective amount of at least one blood modifier for vascular conditions which is different from the sterol absorption inhibitor.

47. A therapeutic combination comprising:
(a) a first amount of at least one sterol absorption inhibitor or pharmaceutically acceptable salt or solvate thereof or prodrug of the at least one sterol absorption inhibitor or of the salt or solvate thereof; and
(b) a second amount of at least one blood modifier different from the sterol absorption inhibitor,
wherein the first amount and the second amount together comprise a therapeutically effective amount for the treatment or prevention of vascular conditions, diabetes, obesity or lowering a concentration of a sterol in plasma of a mammal.

48. A method of treating or preventing vascular conditions, diabetes, obesity or lowering a concentration of a sterol in plasma of a mammal, comprising the step of administering to a mammal in need of such treatment an effective amount of the therapeutic combination of claim 47.
